(19) Europäisches Patentamt
European Patent Office
Office européen des brevets

(11) Publication number: **0 155 767**
**B1**

(12) **EUROPEAN PATENT SPECIFICATION**

(45) Date of publication of patent specification: **16.08.90**

(21) Application number: **85301119.5**

(22) Date of filing: **20.02.85**

(51) Int. Cl.⁵: **C 07 D 403/12,**
C 07 D 405/12,
C 07 D 409/12,
C 07 D 417/12,
C 07 D 491/048,
C 07 D 491/052,
C 07 D 251/46,
C 07 D 239/42,
C 07 D 239/46, A 01 N 47/36

(54) **Herbicidal sulfonamides.**

(30) Priority: **01.03.84 US 585170**
**26.12.84 US 686796**
**21.02.84 US 581817**
**11.12.84 US 679145**

(43) Date of publication of application:
**25.09.85 Bulletin 85/39**

(45) Publication of the grant of the patent:
**16.08.90 Bulletin 90/33**

(84) Designated Contracting States:
**BE CH DE FR GB IT LI LU NL SE**

(56) References cited:
**EP-A-51 465**
**EP-A-72 347**
**EP-A-79 683**
**EP-A-84 224**
**EP-A-94 790**
**EP-A-95 925**
**EP-A-99 339**
**US-A-4 435 206**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Pasteris, Robert James**
**305 Plymouth Road, Fairfax**
**Wilmington, DE 19803 (US)**
Inventor: **Thompson, Mark Ewell**
**34 Austin Circle**
**Wilmington, DE 19810 (US)**
Inventor: **Wexler, Barry Arthur**
**2205 Patwynn Road**
**Wilmington, DE 19810 (US)**

(74) Representative: **Hildyard, Edward Martin et al**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 155 767 B1**

**Description**

This invention relates to novel arylmethane- and arylsulfamoyl- sulfonylureas which are useful as pre- and post-emergence herbicides. The compounds of this invention and their agriculturally suitable salts, are useful as agricultural chemicals such as herbicides.

U.S. Patent Nos. 4,169,719 and 4,127,405 disclose herbicidal benzene and thiophene sulfonamides.

U.S. Patent No. 4,435,206 discloses herbicidal pyridine sulfonamides.

U.S. Patent No. 4,420,325 discloses benzyl sulfonylureas such as:

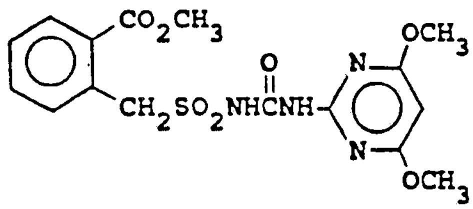

EP—A—87,780 (published September 7, 1983), filed by Nissan Chemical Industries, discloses pyrazole sulfonamides of the general formula shown below.

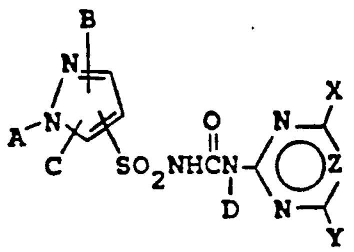

wherein

A is H, $C_1$—$C_8$ alkyl or optionally substituted phenyl;

B and C are independently H, halogen, $NO_2$, $C_1$—$C_8$ alkyl, $CO_2R$, etc.;

D is H or $C_1$—$C_8$ alkyl;

Z is CH or N; and

X and Y are methyl, methoxy, etc.

European Patent Application 71,441, published February 9, 1983, discloses compounds of the formula

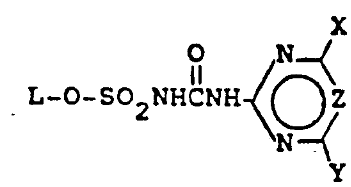

where Z is CH or N; X is $CH_3$ or $OCH_3$; Y is Cl, $CH_3$, $OCH_3$, $OC_2H_5$, $CH_2OCH_3$, $NH_2$, $NHCH_3$, $N(CH_3)_2$, $CH(OCH_3)_2$,

or 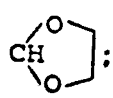;

and L is

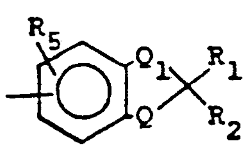 or 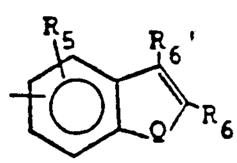

where

$R_1$ is H or $C_1$—$C_3$ alkyl; $R_2$ is H or $CH_3$, $R_5$ is H, $CH_3$, $OCH_3$, Cl, Br, $NO_2$, $CO_2R_7$, $SO_2R_8$, $OSO_2R_9$, $SO_2NR_{10}R_{11}$;

$R_6$ is H or $C_1$—$C_3$ alkyl;

$R_6'$ is H or $CH_3$;

Q is O, S, or $SO_2$; and

$Q_1$ is $CR_3R_4$ or O.

Utility is a pre- or post-emergent herbicide and as a plant growth regulant is disclosed.

European Patent Application 79,683, published May 25, 1983, discloses compounds, useful as herbicides or plant growth regulants, of the formula

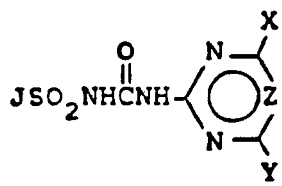

where

J is

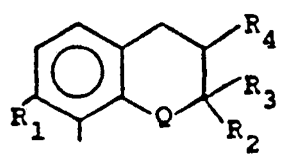 ,

J1

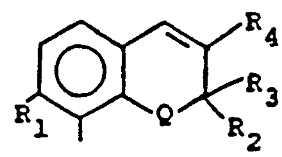 ,

J2

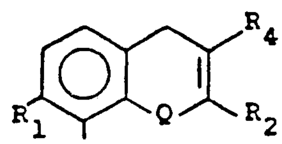 ,

J3

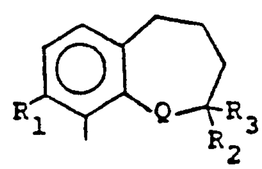

J4

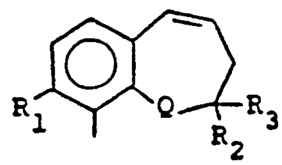 ,

J5

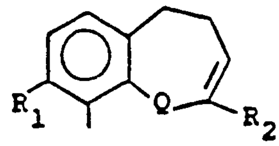

J6

or

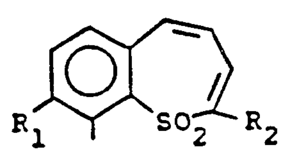 ;

J7

Q is O, S or $SO_2$;

R is H or $CH_3$;

$R_1$ is H, $CH_3$, $OCH_3$, Cl, Br, $CO_2R_5$, $SO_2R_6$, $OSO_2R_7$ or $SO_2NR_8R_9$;

$R_2$ and $R_3$ are independently H or $C_1$—$C_3$ alkyl;

$R_4$ is H or $CH_3$;

$R_5$ is $C_1$—$C_3$ alkyl, $CH_2CH{=}CH_2$, $CH_2CH_2OCH_3$ or $CH_2CH_2Cl$;

$R_6$ is $C_1$—$C_3$ alkyl;

$R_7$ is $C_1$—$C_3$ alkyl or $CF_3$; and

$R_8$ and $R_9$ are independently $C_1$—$C_2$ alkyl.

Further herbicidal sulfonylureas are disclosed in EP—A—95,925; EP—A—84,224; EP—A—99,339 and EP—A—51,465.

The presence of undesired vegetation causes substantial damage to useful crops, especially agricultural products that satisfy mans' basic food and fiber needs, such as cotton, rice, corn, wheat and the like. The current population explosion and concomitant world food and fiber shortage demand improvements in the efficiency of producing these crops. Preventing or minimizing loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides.

## Summary of the Invention

This invention relates to novel compounds of Formula I, agriculturally suitable compositions containing them and their method-of-use as preemergent and/or postemergent herbicides or as plant growth regulants.

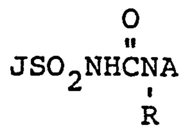

I

wherein

J is

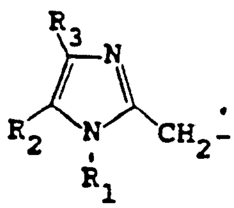

,

J-1

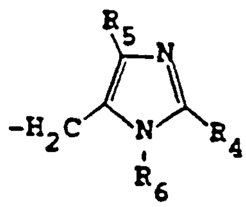

,

J-2

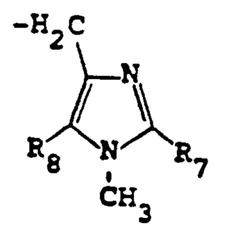

,

J-3

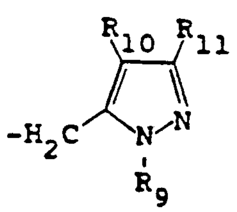

,

J-4

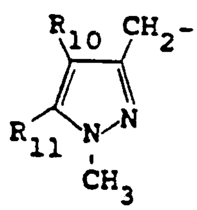

,

J-5

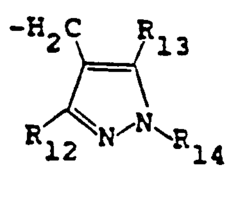

,

J-6

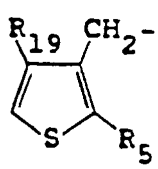

,

J-7

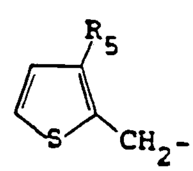

,

J-8

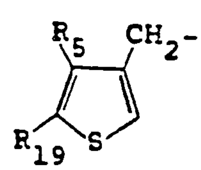

,

J-9

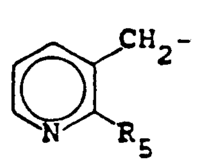

,

J-10

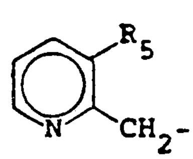

,

J-11

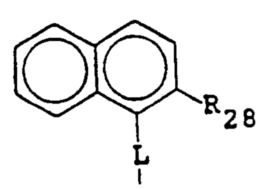

$J_{32}$

L is $CH_2$ or O;
R is H or $CH_3$;
$R_1$ is H, $C_1$—$C_3$ alkyl, $CO_2R_{15}$, $SO_2NR_{16}R_{17}$ or $SO_2R_{18}$;
$R_2$ is H or $CH_3$;
$R_3$ is H or $CH_3$;
$R_4$ is H or $CH_3$;
$R_5$ is H, $CH_3$, Cl, Br, $CO_2R_{15}$, $SO_2NR_{16}R_{17}$, $SO_2N(OCH_3)CH_3$, $SO_2R_{18}$ or $NO_2$;
$R_6$ is H $C_1$—$C_3$ alkyl, $CO_2R_{15}$, $SO_2NR_{16}R_{17}$ or $SO_2R_{18}$;
$R_7$ is H or $CH_3$;
$R_8$ is $CO_2R_{15}$, $SO_2NR_{16}R_{17}$, $SO_2N(OCH_3)CH_3$, $SO_2R_{18}$ or $NO_2$;
$R_9$ is H, $C_1$—$C_3$ alkyl or phenyl;
$R_{10}$ is $CO_2R_{15}$, $SO_2NR_{16}R_{17}$, $SO_2N(OCH_3)CH_3$, $SO_2R_{18}$ or $NO_2$;
$R_{11}$ is H or $CH_3$;
$R_{12}$ is H, $CO_2R_{15}$, $SO_2NR_{16}R_{17}$, $SO_2N(OCH_3)CH_3$, $SO_2R_{18}$ or $NO_2$;
$R_{13}$ is H, $CO_2R_{15}$, $SO_2NR_{16}R_{17}$, $SO_2N(OCH_3)CH_3$, $SO_2R_{18}$ or $NO_2$;
$R_{14}$ is H or $CH_3$;
$R_{15}$ is $C_1$—$C_2$ alkyl;
$R_{16}$ is $C_1$—$C_2$ alkyl;
$R_{17}$ is $C_1$—$C_2$ alkyl;
$R_{18}$ is $C_1$—$C_2$ alkyl;
$R_{19}$ is $CH_3$, Cl, Br, $NO_2$, $C_1$—$C_2$ alkylthio or $C_1$—$C_2$ alkylsulfonyl;
$R_{28}$ is F, Cl, Br, $OCH_3$, $SCH_3$, $SO_2CH_3$, $CO_2CH_3$, $SO_2N(CH_3)_2$ or $OSO_2CH_3$;

A is 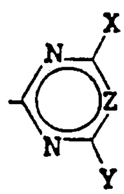 , 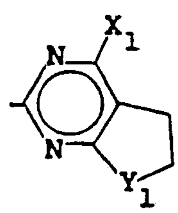 , 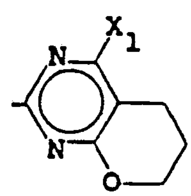 , 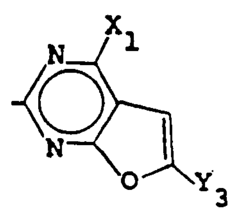

A-1 A-2 A-3 A-4

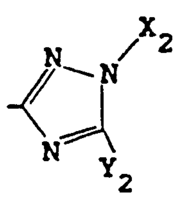 , 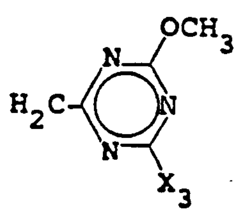 ;

A-5 A-6

X is $CH_3$, $OCH_3$, Cl, Br, $OCH_2CF_3$ or $OCHF_2$;
Y is $C_1$—$C_3$ alkyl, $CH_2F$, cyclopropyl, C≡CH, $OCH_3$, $OC_2H_5$, $NH_2$, $NHCH_3$, $N(CH_3)_2$, $OCH_2CH_2F$, $CF_3$, $SCH_3$, $OCH_2CH{=}CH_2$, $OCH_2C{\equiv}CH$, $OCH_2CH_2OCH_3$ or $OCF_2H$;
Z is CH or N;
$X_1$ is $CH_3$, $OCH_3$, $OC_2H_5$ or $OCF_2H$;
$Y_1$ is O or $CH_2$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$;
$Y_3$ is H or $CH_3$; and agriculturally suitable salts thereof; provided that
1) when X is Cl or Br, then Z is CH and Y is $OCH_3$, $OC_2H_5$, $NHCH_3$, $N(CH_3)_2$ or $OCF_2H$;
2) when X or Y is $OCF_2H$, then Z is CH;
3) $R_5$ and $R_6$ are not simultaneously H;
4) when $R_6$ is other than H or $C_1$—$C_3$ alkyl, then $R_5$ must be H;
5) $R_{12}$ and $R_{13}$ are not simultaneously H;
6) when $R_{12}$ is other than H, then $R_{13}$ must be H;
7) $R_5$ and $R_{19}$ are not simultaneously H; and
8) when J is J-8 or J-11 then $R_5$ is other than H.

Preferred Compounds

Preferred for reasons of their higher herbicidal activity, greater plant growth regulant activity or more favorable ease of synthesis are:

1) Compounds of Formula I where R is H and A is A-1.

2) Compounds of Preferred 1 where Y is $CH_3$, $C_2H_5$, $OCH_3$, $OC_2H_5$, $CH_2F$ or $CF_3$; and L is $CH_2$.

3) Compounds of Preferred 1 where J is J-1, J-4, J-5 or J-6; X is $CH_3$ or $OCH_3$; and Y is $C_1$—$C_2$ alkoxy or $C_1$—$C_2$ alkyl.

Specifically Preferred for reasons of their highest herbicidal activity, greatest plant growth regulant activity and/or most favorable ease of synthesis are:

3-[[(4,6-dimethoxypyrimidin-2-yl)aminocarbonyl]-aminosulfonylmethyl]-1,5-dimethyl-1H-pyrazole-4-carboxylic acid, ethyl ester, m.p. 180—183°C; and

5-[[(4,6-dimethoxypyrimidin-2-yl)aminocarbonyl]-aminosulfonylmethyl]-1,3-dimethyl-1H-pyrazole-4-carboxylic acid, ethyl ester, m.p. 190°C.

## Detailed Description of the Invention

Synthesis

The compounds of Formula I can be prepared by one or more of the methods described below in Equations 1, 2, 3 and 4. Preferred conditions and reagents are given below for each reaction, but these may be varied within the scope conventional in the art.

As shown in Equation 1, compounds of Formula I can be prepared by reacting a sulfonyl isocyanate of Formula II with an appropriate heterocyclic amine of Formula III. J, R and A are as previously defined.

Equation 1

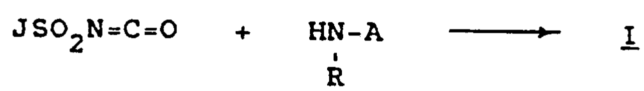

II III

The reaction is preferably carried out at 25° to 100°C in an inert, aprotic solvent such as methylene chloride or xylene for 0.5 to 24 hours as taught in U.S. Patent 4,127,405.

Compounds of Formula I, where L is —$CH_2$—, and where $R_1$, $R_5$, $R_6$, $R_8$, $R_{10}$, $R_{12}$ and $R_{13}$ of J are other than $CO_2R_{15}$, can be prepared by reacting the sulfonamides of Formula IV with an appropriate methyl carbamate of Formula V in the presence of an equimolar or greater amount of trimethylaluminum, as shown in Equation 2.

Equation 2

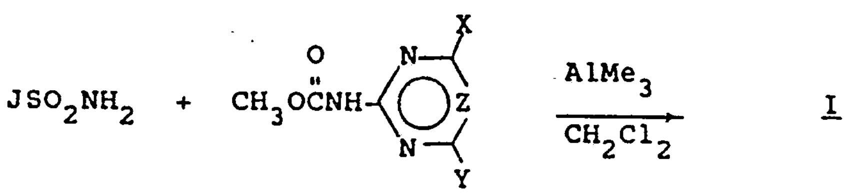


IV V

The reaction Equation 2 is preferably carried out at 25° to 40°C in a solvent such as methylene chloride under an inert atmosphere, as taught in our EP—A—84,224 (published July 27, 1983). The required carbamates V are prepared by reacting the corresponding amines III with dimethylcarbonate or methyl chloroformate in the presence of a base.

Alternatively, compounds of Formula I, where L is —$CH_2$—, can be prepared by reacting a sulfonyl carbamate of Formula VI with an appropriate amine of Formula III, as shown in Equation 3.

Equation 3

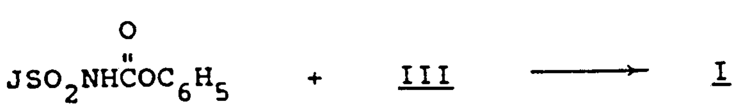

VI

The reaction is preferably carried out at 50° to 100°C in a solvent such as dioxane for 0.5 to 24 hours as taught for example in our EP—A—44,807. The required carbamates VI are prepared by reacting the corresponding sulfonamides IV with diphenylcarbonate in the presence of a strong base.

Additionally, compounds of Formula I, L is —$CH_2$—, can be prepared by reacting a sulfonamide of Formula IV with an appropriate heterocyclic phenyl carbamate of Formula VII, in the presence of a nonnucleophilic base, such as 1,8-diazabicyclo-[5.4.0]undec-7-ene (DBU) as shown below in Equation 4.

Equation 4

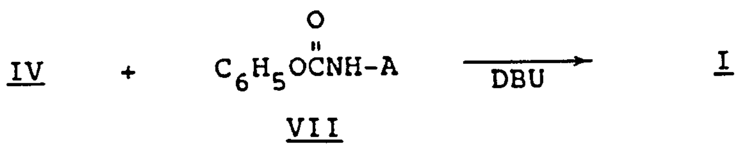


IV + VII → I

The reaction is preferably carried out at 20° to 100°C in an inert solvent, such as dioxane, for 0.5 to 24 hours by the methods taught in EP—A—44,807. The required carbamates, VII, are prepared by reacting the corresponding heterocyclic amines, III, with phenylchloroformate in the presence of a base.

Compounds of Formula I, where J is $J_{32}$ and where L is 0, can be prepared as shown below in Equation 5. The intermediate chlorosulfonylurea of Formula VIII is formed by treatment of the corresponding heterocyclic amine, III, with commercially available chlorosulfonyl isocyanate and is then allowed to react with an appropriate phenol of Formula IX.

Equation 5

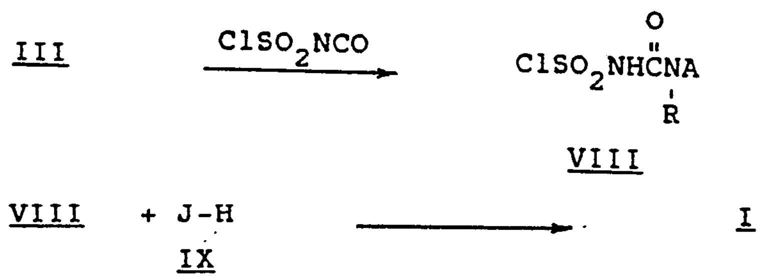


III

VIII

VIII + IX

I

wherein

J and A are as previously defined.

The reaction shown in Equation 5 is best carried out according to the methods taught in United States Patent 4,391,976.

The intermediate sulfonyl isocyanates of Formula II in Equation 1 can be prepared as shown in Equations 6 and 7.

As shown in Equation 6, sulfonyl isocyanates of Formula II where J is other than $J_1$, $J_2$ or $J_3$ and where L is —$CH_2$—, can be prepared by the reaction of sulfonamides of Formula IV with phosgene, in the presence of *n*-butyl isocyanate and a tertiary amine catalyst, such as diazabicyclo-[2.2.2]octane (DABCO®), at reflux in a solvent such as xylene by the method of U.S. Patent 4,238,621.

Equation 6

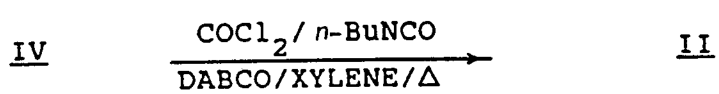


IV

II

The sulfonyl isocyanates can also be prepared from the sulfonamides by a two step procedure involving (a) reacting the sulfonamides with *n*-butyl isocyanate in the presence of a base such as $K_2CO_3$ at reflux in an inert solvent such as 2-butanone forming a *n*-butyl sulfonylurea; and (b) reacting this compound with phosgene and a tertiary amine catalyst at reflux in xylene solvent. The method is similar to a procedure taught by Ulrich and Sayigh, *Newer Methods of Preparative Organic Chemistry,* Vol. VI, p. 223—241, Academic Press, New York and London, W. Foerst Ed.

Alternatively, as shown in Equation 7, the sulfonyl isocyanates of Formula II can be prepared by reacting the corresponding sulfonyl chlorides X with cyanic acid salts.

Equation 7

$$\underset{\underline{X}}{JSO_2Cl} \xrightarrow{M^{\oplus}OCN^{\ominus}} \underline{II}$$

The reaction is carried out at 25° to 100°C in an inert aprotic solvent such as acetonitrile for 0.5—24 hours in the presence of phosphorus pentoxide and an alkali metal salt such as lithium iodide according to the teachings of Japanese Patent No. 76/26,816 (*Chem. Abst.*, 85:77892e (1976)).

Another method for the preparation of sulfonyl isocyanates II is shown in Equation 8. Treatment of sulfonamides of Formula IV with thionyl chloride gives intermediate N-sulfinylsulfonamides, XI, which afford sulfonyl isocyanates of Formula II upon exposure to phosgene in the presence of a catalytic amount of pyridine.

Equation 8

$$\underset{\underline{IV}}{JSO_2NH_2} \xrightarrow[\Delta]{SOCl_2} \underset{\underline{XI}}{JSO_2NSO}$$

$$\underline{XI} \xrightarrow[\text{cat. pyridine toluene}]{COCl_2} \underset{\underline{II}}{JSO_2NCO}$$

wherein

J is as previously defined.

The reaction of Equation 8 can best be performed according to the procedure of H. Ulrich, B. Tucker, and A. Sayigh, *J. Org. Chem.*, *34*, 3200 (1969).

Sulfonyl isocyanates of Formula II, where J is $J_{32}$ and L is 0, can be most easily prepared as shown below in Equation 9 by the reaction of appropriately substituted phenols of Formula IX with chlorosulfonyl isocyanate. This procedure is taught in United States Patents 4,191,553 and 4,394,153, and by Lohaus in *Chem. Ber.*, *105*, 2791 (1972).

Equation 9

$$\underset{\underline{IX}}{J\text{-}H} \xrightarrow[\text{toluene or xylenes, } \Delta]{\text{chlorosulfonyl isocyanate,}} \underset{\underline{II}}{JSO_2NCO}$$

wherein

J is as previously defined.

As shown in Equation 10, sulfonamides of Formula IV can be prepared from the corresponding sulfonyl chlorides of Formula X by contacting with either anhydrous or aqueous ammonia.

Equation 10

$$\underline{X} \xrightarrow[\text{or } NH_3]{NH_4OH} \underline{IV}$$

The preparation of sulfonamides from sulfonyl chlorides is widely reported in the literature, for reviews see: F. Hawking and J. S. Lawrence, "The Sulfonamides," H. K. Lewis and Co., London, 1950 and E. H. Northey, "The Sulfonamides and Allied Compounds", Reinhold Publishing Corp., New York, 1948.

The requisite methylsulfonyl chlorides of Formula X may be synthesized from appropriately substituted benzylic type chlorides or benzylic type bromides, XII, by the two-step sequence of reactions outlined below in Equation 11 and Equation 12.

Equation 11

(a)

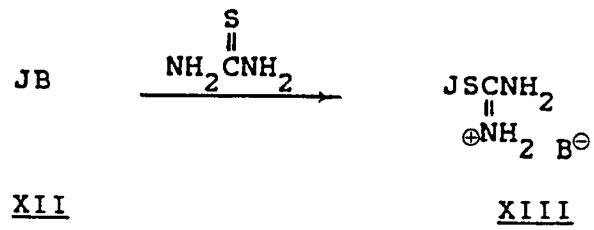


(b)

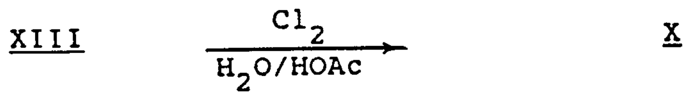


wherein

B is Cl or Br; and

J is $J_1$—$J_{11}$ or $J_{32}$;

L is $—CH_2—$,

Equation 12

(a)

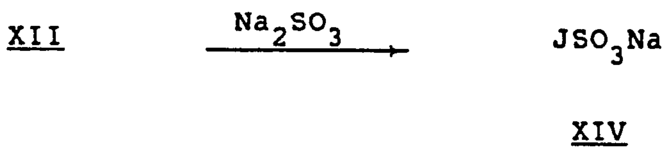


(b)

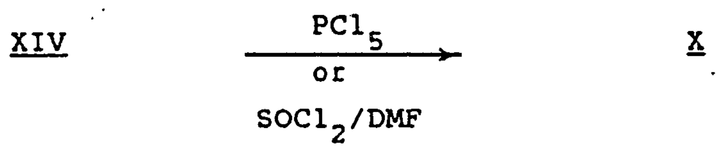


B is Cl or Br;

J is $J_1$—$J_{11}$ or $J_{32}$ and

L is $—CH_2—$.

Equation 11(a)

The conversion of alkyl halides to isothiouronium salts is well precedented in the chemical literature. For relevant examples, see T. B. Johnson and J. M. Sprague, *J. Am. Chem. Soc., 58,* 1348 (1936); *ibid, 59,* 1837 and 2439 (1937); *ibid, 61,* 176 (1939). In a typical procedure, a benzylic type halide of Formula XII is treated with thiourea in protic solvents such as methanol or ethanol, or aprotic solvents such as methylene chloride or benzene. Temperatures of 40—80° over one-half to 24 hours are typically required to complete the reaction. The product salts, XIII, are isolated by cooling and filtration or by concentration to remove the solvent. The salts, XIII, are generally sufficiently pure to be carried on directly to step (11b) without further purification.

Equation 11(b)

The oxidative chlorination of isothiouronium salts to afford sulfonyl chlorides is most efficiently carried out according to the procedure of Johnson as described in *J. Am. Chem. Soc., 61,* 2548 (1939). Thus, the salts of Formula XIII (B = chlorine or bromine) are dissolved or suspended in water or aqueous acetic acid and treated with at least three equivalents of chlorine at temperatures between 5—20°C. When the hydrobromide salts XIII (B = bromine) are used, it is sometimes advantageous to exchange the bromide ion for nitrate ion before chlorination by treatment with an aqueous solution of one equivalent of silver nitrate; the precipitated silver bromide is removed by filtration and the filtrate treated as described above. The product sulfonyl chlorides are isolated either by simple filtration or extraction into a suitable solvent such as methylene chloride or *n*-butyl chloride followed by drying and evaporation of the combined organic extracts. No further purification of the sulfonyl chlorides, X, is necessary.

The conversion of a benzylic type chloride or bromide of Formula XII to a sulfonic acid salt of Formula XIV as shown in Equation 12(a) is well known in the art. For a review, see Gilbert "Sulfonation and Related

Reactions" Interscience Publishers, New York, 1965, pp. 136—148 and 161—163. The methods of conversion of a sulfonic acid salt of Formula XIV to a sulfonyl chloride of Formula X is well known to those skilled in the art.

Benzylic type bromides of Formula XII (B = bromine) may be prepared as shown below in Equation 13 by treating the appropriate methyl aryl compound IX, with N-bromosuccinimide (NBS).

Equation 13

$$\underset{\underline{IX}}{J\text{-}H} \xrightarrow[\text{Catalyst, } CCl_4,\ \Delta]{NBS} \underset{\underline{XII}\ (B\ \text{is}\ Br)}{J\text{-}Br}$$

wherein

J is $J_1$—$J_{11}$ or $J_{32}$,

L is —$CH_2$—.

The reaction represented in Equation 13 can be most conveniently effected by heating a solution of the aryl methane derivatives, IX, and N-bromosuccinimide in a suitable solvent such as carbon tetrachloride at reflux temperature. A free radical catalyst such as azoisobutyronitrile (AIBN) or benzoyl peroxide is usually employed to initiate this reaction. When bromination is complete, the cooled reaction mixture is filtered to remove the by-product succinimide and the filtrate is concentrated *in vacuo*. The benzylic type bromides of Formula XII are often obtained in a sufficiently pure condition for further transformations. If not, they can be purified by vacuum distillation or column chromatography.

Benzylic type chlorides of Formula XII (L = chlorine) may be most efficiently synthesized by one of two methods, both of which are well known in the chemical literature. First, benzylic type chlorides, XII, can be prepared from the appropriate aryl methane derivatives of Formula IX by reaction with N-chlorosuccinimide (NCS). This reaction is depicted below in Equation 14.

Equation 14

$$\underline{IX} \xrightarrow[\text{Catalyst, } CCl_4,\ \Delta]{NCS} \underline{XII}\ (B\ \text{is}\ Cl)$$

The reaction of Equation 14 can be carried out in a manner analogous to that described for the NBS bromination in Equation 13.

In compounds of Formula IX where J is J-1, J-2 or J-3, ring halogenation may occur together with benzylic-type halogenation in Equations 13 and 14. Subsequent removal of the ring halogen after conversion to the desired sulfonamide IV may be carried out by methods known in the art.

Alternatively, benzylic type chlorides of Formula XII may be prepared from the appropriate benzylic type alcohols of Formula XV as shown below in Equation 15.

Equation 15

$$\underset{\underline{XV}}{JOH} \xrightarrow{SOCl_2} \underline{XII}\ (B\ \text{is}\ Cl)$$

There exists a variety of well known methods for converting alcohols to the corresponding chlorides. One of the most common procedures involves reaction of the alcohol with thionyl chloride either alone or in the presence of a trace of a suitable base such as pyridine. For relevant examples, see the following references: H. Gilman and J. E. Kirby, *J. Am. Chem. Soc., 51,* 3475 (1929); H. Gilman and A. P. Hewlett, *Rec. Trav. Chim., 51,* 93 (1932); and M. S. Newman, *J. Am. Chem. Soc., 62,* 2295 (1940).

The requisite aryl methanols of Formula XV shown below in Equation 16 may be prepared by reduction of an appropriately substituted aryl carboxylic acid of Formula XVI with diborane in a solvent such as tetrahydrofuran.

Equation 16

$$\underset{\underline{XVI}}{J^1\text{-}CO_2H} \xrightarrow[THF]{BH_3} \underset{\underline{XV}}{J^1\text{-}CH_2OH}$$

wherein

$J^1$ is $J_1$—$J_{11}$ or $J_{32}$ wherein —$CH_2$— or —L— is absent.

For a description of the use of diborane for reduction of benzoic acid derivatives, see H. C. Brown, *J. Org. Chem., 38,* 2786 (1973).

Alternatively, benzyl alcohols of Formula XV shown below in Equation 17 can be synthesized by reduction of the appropriately substituted benzaldehyde derivatives of Formula XVII with sodium borohydride in a solvent such as ethanol.

Equation 17

$$\underset{\underline{XVII}}{J^1\text{-}CHO} \xrightarrow[EtOH]{NaBH_4} \underline{XV}$$

wherein

$J^1$ is $J_1$—$J_{11}$ or $J_{32}$ wherein —$CH_2$— or —L— is absent.

For a description of the use of sodium borohydride for the selective reduction of aldehydes, see S. Chaikin and W. Brown, *J. Am. Chem., Soc., 71,* 122 (1949).

The aldehydes of Formula XVII can be prepared by the procedure shown in Equation 18 starting with the appropriate nitriles of Formula XVIII.

Equation 18

$$\underset{\underline{XVIII}}{J^1\text{-}CN} \xrightarrow[\text{or } [(CH_3)_2CHCH_2]_2AlH]{LiAlH(OC_2H_5)_3} \xrightarrow{H_3O^+} \underline{XVII}$$

wherein $J^1$ is $J_1$—$J_{11}$ or $J_{32}$ wherein —$CH_2$— or —L— is absent.

The reaction shown in Equation 18 can be carried out according to the procedures of H. C. Brown, C. J. Shoaf, and C. P. Garg, *Tetrahedron Lett.,* 9 (1959), and J. A. Marshall, N. H. Andersen, and J. W. Schlicher, *J. Org. Chem., 35,* 858 (1970).

Benzoic acid derivatives of Formula XVI shown below in Equation 19 can be prepared by hydrolysis of the appropriate nitriles of Formula XVIII which are, in turn, prepared by a Sandmeyer reaction on the corresponding aniline derivatives XIX.

Equation 19

(a)

$$\underset{\underline{XIX}}{J^1\text{-}NH_2} \xrightarrow[\text{2) } CuCN]{\text{1) } HNO_2,\ HCl} \underset{\underline{XVIII}}{J^L CN}$$

(b)

$$\underline{XVIII} \xrightarrow{H_3O^+} \underline{XVI}$$

wherein $J^1$ is $J_1$—$J_{11}$ or $J_{32}$ wherein —$CH_2$— or —L— is absent.

Equation 19(a)

The diazotization and cuprous cyanide coupling reaction of Equation 19(a) is best carried out according to the procedure described in *Organic Syntheses,* Coll. Vol. I, p. 514.

Equation 19(b)

Nitriles of Formula XVIII are most easily hydrolyzed to the desired carboxylic acids XVI by heating at reflux temperature in concentrated solutions of sulfuric acid or by treatment with 100% phosphoric acid and subsequent exposure to nitrous acid. The products are typically isolated by extraction into aqueous base solution followed by washing with a suitable organic solvent such as ether and acidification of the water layer. For a compilation of references dealing with this process, see R. Wagner and H. Zook, "Synthetic Organic Chemistry", John Wiley & Sons, Inc., New York, 1953, p. 412.

A method similar to that described in Equation 19 above can be applied to the synthesis of phenols of Formula IX as shown below in Equation 20.

Equation 20

$$\underline{XIX} \xrightarrow[\text{2) } H_2O,\ \Delta]{\text{1) } HNO_2,\ H_2SO_4} \underline{IX}$$

wherein $J^1$ is $J_1$—$J_{11}$ or $J_{32}$ wherein —$CH_2$— or L is absent.

The diazotization and hydrolysis reactions shown in Equation 20 can be carried out according to the following procedures: Stevens and Beutel, *J. Am. Chem. Soc., 63,* 311 (1941); Grillot and Gormley, *J. Am. Chem. Soc., 67,* 1968 (1945); and Mosettig and Stuart, *J. Am. Chem. Soc., 61,* 1 (1939).

The requisite aryl amine derivatives of Formula XIX can be prepared in a straightforward manner by reduction of the corresponding nitro compounds of Formula XX as shown in Equation 21.

Equation 21

$$\underset{\underline{XX}}{J^1\text{-}NO_2} \xrightarrow{[H]} \underline{XIX}$$

wherein $J^1$ is $J_1$—$J_{11}$ or $J_{32}$ wherein —$CH_2$— or —L— is absent.

There exists a wide variety of methods for effecting the reduction of aromatic nitro groups to the corresponding aryl amine derivatives. One of the more common procedures involves treating the nitro compounds of Formula XX with a slight excess of stannous chloride dihydrate in concentrated hydrochloric acid solution at temperatures between 25° and 80°C. Alternatively, reduction may be accomplished with iron powder in glacial acetic acid solution as described by Hazlet and Dornfeld, *J. Am. Chem. Soc., 66,* 1781 (1944), and by West in *J. Chem. Soc., 127,* 494 (1925). For a general review, see Groggins, "Unit Processes In Organic Synthesis", McGraw-Hill Book Co., New York, 1947, pp. 73—128.

Many of the substituted aromatic nitro compounds of Formula XX (where it is understood that all ring systems defined in the Summary of Invention are represented) can be synthesized by any of several possible synthetic routes known to one skilled in the art.

The intermediate functionalized aryl methane derivatives of Formula IX (L = —$CH_2$—) and aryl methanols of Formula XV. (L = —$CH_2$—) may also be prepared by straightforward synthetic routes known to one skilled in the art.

The heterocyclic amines of Formula III can be prepared by methods known in the literature or simple modifications thereof, by those skilled in the art. For instance, EP—A—84,224 (published July 27, 1983) and W. Braker et al., *J. Am. Chem. Soc.,* 1947, *69,* 3072 describe methods for preparing aminopyrimidines and triazines substituted by acetal groups. Also, South African Patent Application Nos. 825,045 and 825,671 describe methods for preparing aminopyrimidines and triazines substituted by haloalkyl or haloalkylthio groups such as $OCH_2CH_2F$, $OCH_2CF_3$, $SCF_2H$, and $OCF_2H$ among other groups. South African Patent Application 837,434 (published October 5, 1983) describes methods for the synthesis of cyclopropylpyrimidines and triazines substituted by such groups as alkyl, haloalkyl, alkoxy, haloalkoxy, alkylamino, dialkylamino, and alkoxyalkyl.

The 5,6-dihydrofuro[2.3-d]pyrimidin-2-amines, and the cyclopenta[d]pyrimidin-2-amines, of formula III, where A is A-2, and the 6,7-dihydro-5H-pyrano[2.3-d]pyrimidin-2-amines, of formula III, where A is A-3, can be prepared as described in EP—A No. 15,863. The furo[2.3-d]-pyrimidin-2-amines, of formula III, where A is A-4, are described in EP—A No. 46,677. Heterocycles of formula III, where A is A-5, may be prepared as described in EP—A No. 73,562. Heterocycles of Formula III, where A is A-6, may be prepared by methods taught in EP—A—94,260.

In addition, general methods for preparing aminopyrimidines and triazines have been reviewed in the following publications.

"The Chemistry of Heterocyclic Compounds", a series published by Interscience Publishers, Inc., New York and London;

"Pyrimidines", Vol. 16 of the same series by D. J. Brown;

"*s*-Triazines and Derivatives", Vol. 13 of the same series by E. M. Smolin and L. Rappaport; and

F. C. Schaefer, U.S. Patent 3,154,547 and K. R. Huffman and F. C. Schaefer, *J. Org. Chem., 28,* 1812 (1963), which describe the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., *p*-toluenesulfonic acid, trichloroacetic acid or the like.

The preparation of the compounds of this invention is illustrated by the following examples.

Example 1

Ethyl 5-(chloromethyl)-1,3-dimethyl-1H-pyrazole-4-carboxylate

To 5.3 g (26.8 mmol) of 4-ethoxycarbonyl-5-(1-hydroxymethyl)-1,3-dimethylpyrazole prepared by the method of S. Gelin, et al., *J. Heterocyclic Chem.*, *16*, (1979), 1117—20, was added 1.95 ml (26.8 mmol) of thionyl chloride, dropwise. Gas evolution and an exotherm occurred. The solution was cooled with an ice bath. After ten minutes the solution gave a solid, m.p. 81—83°C, $^1H$ NMR (90 MHz, $CDCl_3$): δ 1.4 (t, $OCCH_3$, 3H); 2.65 (s, $CH_3$, 3H); 4.2 (s, $NCH_3$, 3H); 4.4 (q, $OCH_2C$, 2H); 5.2 (s, $CH_2Cl$, 2H).

This material was combined with the product of a second reaction (5.0 g of 4-ethoxycarbonyl-5-(1-hydroxymethyl)-1,3-dimethylpyrazole and 1.93 ml thionyl chloride), dissolved in dichloromethane, washed with brine, dried ($MgSO_4$) and concentrated to give 10.7 g of a solid, m.p. 81—83°C.

Example 2

Ethyl 5-[[(amino)(imino)methyl]thiomethyl]-1,3-dimethyl-1H-pyrazole-4-carboxylate

A solution under nitrogen was prepared from 10.7 g of the compound produced in Example 1 and 3.95 g of thiourea in 200 ml of ethanol. After refluxing the solution four hours, it was allowed to stir at room temperature overnight. The solvent was removed under reduced pressure. Then, the residue was triturated with hexane to give 16 g of solid (wet weight), m.p. 175—180°C.

Example 3

Ethyl 5-methanesulfonamide-1,3-dimethyl-1H-pyrazole-4-carboxylate

To 14 g of the compound produced in Example 2, dissolved in 90 ml of water at 5°C was added 10 ml of chlorine dropwise. The rate of addition was monitored so that the temperature did not exceed 20°C. A precipitate formed. The aqueous portion was decanted off and the residue was dissolved in dichloromethane, washed with saturated sodium bicarbonate solution, dried ($MgSO_4$) and concentrated. The residue was dissolved in 100 ml of THF. To a 75 ml portion of the THF solution, cooled to 5°C, was added 1.5 ml of concentrated ammonium hydroxide solution in 10 ml of THF. A suspension formed, which was stirred for six hours. The reaction mixture was filtered and the mother liquor was concentrated under reduced pressure. The residue, triturated with ethyl acetate-diethyl ether (40:60) gave 3.0 g of a light yellow solid, m.p. 135—138°C; $^1H$ NMR (90 MHz, $d_6$-DMSO): δ 1.2 (t, $CH_3$, 3H); 2.3 (s, $CH_3$, 3H); 3.8 (s, $NCH_3$, 3H) 4.2 (q, $OCH_2$, 2H); 4.8 (s, $CH_2SO_2$, 2H); 7.1 (bs, $NH_2$, 2H).

Example 4

Ethyl 5-[[[[4,6-dimethoxypyrimidin-2-yl]aminocarbonyl]aminosulfonyl]methyl]-1,3-dimethyl-1H-pyrazole-4-carboxylate

In a dry flask under nitrogen was stirred 0.33 g of the sulfonamide from Example 3 and 0.35 g of phenyl (4,6-dimethoxypyrimidin-2-yl)carbamate and 30 ml of dry acetonitrile. Then 0.19 ml of 1,8-diazabicyclo[5.4.0]undec-7-ene was added via syringe and the solution stirred overnight. Then 50 ml of water was added, followed by a dropwise addition of 1 NHCl until a precipitate formed. The suspension was filtered. The filtrant, after being triturated with diethyl ether, gave 0.4 g of solid, m.p. 190°C; IR (Nujol) 1711 and 1680 (C=0) $cm^{-1}$; $^1H$ NMR (200 MHz, $CDCl_3$): δ 1.25 (t, $CH_3$, 3H); 2.38 (s, $CH_3$, 3H); 3.80 (s, $OCH_3$, 6H); 3.94 (s, $NCH_3$, 3H); 4.15 (q, $OCH_2$, 2H); 5.23 (s, $CH_2SO_2$, 2H); 5.71 (s, bs, CH, 1H); 7.35 (bs, NH, 1H) and 12.4 (bs, NH, 1H).

The invention is further exemplified, but not limited to, the compounds in Tables I—XXIX. The compounds depicted in these tables may be prepared by methods described in Examples 1—4, or by modifications thereof apparent to those skilled in the art.

General Formulas for Tables

General Formula 1

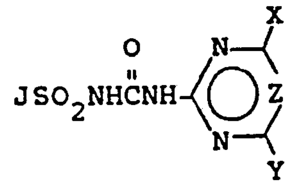

General Formula 2a

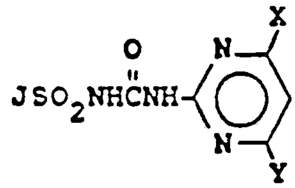

General Formula 3a

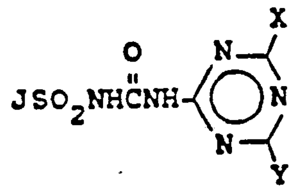

General Formula 4

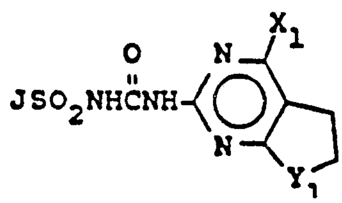

General Formula 5

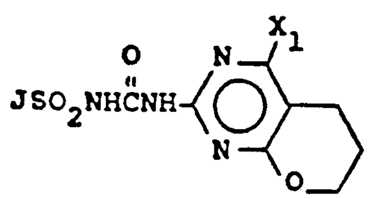

General Formula 6

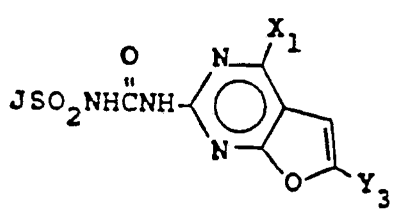

General Formula 7

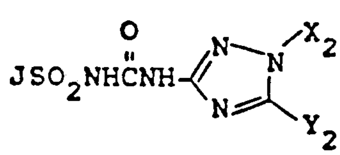

General Formula 8

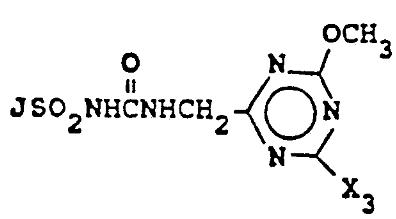

Table I

General Formula 1

($J=J_1$)

| $R_1$ | $R_2$ | $R_3$ | X | Y | Z | m.p.(°C) |
|---|---|---|---|---|---|---|
| H | H | H | $CH_3$ | $CH_3$ | CH | |
| H | H | H | $CH_3$ | $OCH_3$ | CH | |
| $CH_3$ | H | H | $OCH_3$ | $OCH_3$ | CH | |
| $CH_3$ | H | H | $CH_3$ | $CH_3$ | N | |
| $CH_3$ | H | H | $CH_3$ | $OCH_3$ | N | |
| $CH_2CH_3$ | H | H | $OCH_3$ | $OCH_3$ | N | |
| $CH_2CH_2CH_3$ | H | H | $CH_3$ | $OCH_2CH_3$ | CH | |
| $CH(CH_3)_2$ | H | H | $CH_3$ | $OCH_3$ | N | |
| $CO_2CH_3$ | H | H | $OCH_3$ | $OCH_3$ | N | |
| $CO_2CH_3$ | H | H | Br | $OCH_3$ | CH | |
| $CO_2CH_3$ | H | H | $CH_3$ | $OCH_3$ | CH | |
| $CO_2CH_3$ | H | H | $OCH_3$ | $OCH_3$ | CH | |
| $CO_2CH_3$ | H | H | $CH_3$ | $CH_3$ | N | |
| $CO_2CH_3$ | H | H | $CH_3$ | $OCH_3$ | N | |
| $CO_2CH_2CH_3$ | H | H | $OCH_3$ | $OCH_3$ | N | |
| $CO_2CH_2CH_3$ | H | H | $CH_3$ | $OCH_3$ | CH | |
| $SO_2N(CH_3)_2$ | H | H | $OCH_3$ | $OCH_3$ | CH | |
| $SO_2N(CH_3)CH_2CH_3$ | H | H | $CH_3$ | $CH_3$ | N | |
| $SO_2N(CH_2CH_3)_2$ | H | H | $CH_3$ | $OCH_3$ | N | |
| $SO_2CH_3$ | H | H | $OCH_3$ | $OCH_3$ | N | |
| $SO_2CH_3$ | H | H | $CH_3$ | $CH_2F$ | CH | |
| $SO_2CH_3$ | H | H | $OCH_3$ | $OCH_3$ | CH | |
| $SO_2CH_3$ | H | H | Cl | $OCH_3$ | CH | |
| $SO_2CH_2CH_3$ | H | H | $OCH_3$ | $OCH_3$ | CH | |
| H | $CH_3$ | H | $CH_3$ | $CH_3$ | CH | |
| H | $CH_3$ | H | $CH_3$ | $OCH_3$ | CH | |
| $CH_3$ | $CH_3$ | H | $OCH_3$ | $OCH_3$ | CH | |
| $CH_3$ | $CH_3$ | H | $CH_3$ | $CH_3$ | N | |
| $CH_3$ | $CH_3$ | H | $CH_3$ | $OCH_3$ | N | |
| $CH_2CH_3$ | $CH_3$ | H | $OCH_3$ | $OCH_3$ | N | |
| $CH_2CH_2CH_3$ | $CH_3$ | H | $CH_3$ | $OCH_2CH_3$ | CH | |
| $CH_2CH_2CH_3$ | $CH_3$ | H | $CH_3$ | $CH_3$ | N | |
| $CH(CH_3)_2$ | $CH_3$ | H | $CH_3$ | $OCH_3$ | N | |
| $CO_2CH_3$ | $CH_3$ | H | $OCH_3$ | $OCH_3$ | N | |
| $CO_2CH_3$ | $CH_3$ | H | $OCH_2CF_3$ | $CH_3$ | CH | |
| $CO_2CH_3$ | $CH_3$ | H | $CH_3$ | $OCH_3$ | CH | |

Table I (continued)

| $R_1$ | $R_2$ | $R_3$ | X | Y | Z | m.p.(°C) |
|---|---|---|---|---|---|---|
| $CO_2CH_3$ | $CH_3$ | H | $OCH_3$ | $OCH_3$ | CH | |
| $CO_2CH_3$ | $CH_3$ | H | $CH_3$ | $OCH_3$ | N | |
| $CO_2CH_2CH_3$ | $CH_3$ | H | $OCH_3$ | $OCH_3$ | N | |
| $CO_2CH_2CH_3$ | $CH_3$ | H | $CH_3$ | $OCH_3$ | CH | |
| $SO_2N(CH_3)_2$ | $CH_3$ | H | $OCH_3$ | $OCH_3$ | CH | |
| $SO_2N(CH_3)CH_2CH_3$ | $CH_3$ | H | $CH_3$ | $CH_3$ | N | |
| $SO_2N(CH_2CH_3)_2$ | $CH_3$ | H | $CH_3$ | $OCH_3$ | N | |
| $SO_2CH_3$ | $CH_3$ | H | $OCH_3$ | $OCH_3$ | N | |
| $SO_2CH_3$ | $CH_3$ | H | Cl | $OCH_3$ | CH | |
| $SO_2CH_2CH_3$ | $CH_3$ | H | $OCH_3$ | $OCH_3$ | CH | |
| H | H | $CH_3$ | $CH_3$ | $CH_3$ | CH | |
| H | H | $CH_3$ | $CH_3$ | $OCH_3$ | CH | |
| $CH_3$ | H | $CH_3$ | $OCH_3$ | $OCH_3$ | CH | |
| $CH_3$ | H | $CH_3$ | $CH_3$ | $CH_3$ | N | |
| $CH_3$ | H | $CH_3$ | $CH_3$ | $OCH_3$ | N | |
| $CH_2CH_3$ | H | $CH_3$ | $OCH_3$ | Δ | N | |
| $CH_2CH_2CH_3$ | H | $CH_3$ | $OCH_3$ | $OCH_3$ | CH | |
| $CH_2CH_2CH_3$ | H | $CH_3$ | $CH_3$ | $CH_3$ | N | |
| $CH(CH_3)_2$ | H | $CH_3$ | $CH_3$ | $OCH_3$ | N | |
| $CO_2CH_3$ | H | $CH_3$ | $CH_3$ | $CH_3$ | CH | |
| $CO_2CH_3$ | H | $CH_3$ | $CH_3$ | $OCH_3$ | CH | |
| $CO_2CH_3$ | H | $CH_3$ | $OCH_3$ | $OCH_3$ | CH | |
| $CO_2CH_3$ | H | $CH_3$ | $CH_3$ | $CH_3$ | N | |
| $CO_2CH_3$ | H | $CH_3$ | $CH_3$ | $OCH_3$ | N | |
| $CO_2CH_2CH_3$ | H | $CH_3$ | $OCH_3$ | $OCH_3$ | N | |
| $CO_2CH_2CH_3$ | H | $CH_3$ | $CH_3$ | $OCH_3$ | CH | |
| $SO_2N(CH_3)_2$ | H | $CH_3$ | $OCH_3$ | $OCH_3$ | CH | |
| $SO_2N(CH_3)CH_2CH_3$ | H | $CH_3$ | $CH_3$ | $CH_3$ | N | |
| $SO_2N(CH_2CH_3)_2$ | H | $CH_3$ | $CH_3$ | $OCH_2CH_2F$ | N | |
| $SO_2CH_3$ | H | $CH_3$ | $OCH_3$ | $OCH_3$ | N | |
| $SO_2CH_3$ | H | $CH_3$ | $OCH_3$ | $OCH_3$ | CH | |
| $SO_2CH_3$ | H | $CH_3$ | Cl | $OCH_3$ | CH | |
| $SO_2CH_2CH_3$ | H | $CH_3$ | $OCH_3$ | $OCH_3$ | CH | |
| H | $CH_3$ | $CH_3$ | $CH_3$ | $CF_3$ | CH | |

Table I (continued)

| $R_1$ | $R_2$ | $R_3$ | X | Y | Z | m.p.(°C) |
|---|---|---|---|---|---|---|
| H | $CH_3$ | $CH_3$ | $CH_3$ | $OCH_3$ | CH | |
| $CH_3$ | $CH_3$ | $CH_3$ | $OCH_3$ | $OCH_3$ | CH | |
| $CH_3$ | $CH_3$ | $CH_3$ | $CH_3$ | $SCH_3$ | N | |
| $CH_3$ | $CH_3$ | $CH_3$ | $CH_3$ | $OCH_3$ | N | |
| $CH_2CH_3$ | $CH_3$ | $CH_3$ | $OCH_3$ | $OCH_3$ | N | |
| $CH_2CH_2CH_3$ | $CH_3$ | $CH_3$ | $CH_3$ | $OCH_2CH_3$ | CH | |
| $CH_2CH_2CH_3$ | $CH_3$ | $CH_3$ | $CH_3$ | $OCH_3$ | CH | |
| $CH_2CH_2CH_3$ | $CH_3$ | $CH_3$ | $OCH_3$ | $OCH_2C{\equiv}CH$ | CH | |
| $CH_2CH_2CH_3$ | $CH_3$ | $CH_3$ | $CH_3$ | $CH_3$ | N | |
| $CH(CH_3)_2$ | $CH_3$ | $CH_3$ | $CH_3$ | $OCH_3$ | N | |
| $CO_2CH_3$ | $CH_3$ | $CH_3$ | $OCH_3$ | $OCH_3$ | N | |
| $CO_2CH_3$ | $CH_3$ | $CH_3$ | $CH_3$ | $OCH_2CH_3OCH_3$ | CH | |
| $CO_2CH_3$ | $CH_3$ | $CH_3$ | $CH_3$ | $OCH_3$ | CH | |
| $CO_2CH_3$ | $CH_3$ | $CH_3$ | $OCH_3$ | $OCH_3$ | CH | |
| $CO_2CH_3$ | $CH_3$ | $CH_3$ | $CH_3$ | $CH_3$ | N | |
| $CO_2CH_3$ | $CH_3$ | $CH_3$ | $CH_3$ | $OCH_3$ | N | |
| $CO_2CH_2CH_3$ | $CH_3$ | $CH_3$ | $CH_3$ | $OCH_3$ | CH | |
| $SO_2N(CH_3)CH_2CH_3$ | $CH_3$ | $CH_3$ | $CH_3$ | $CH_3$ | N | |
| $SO_2N(CH_2CH_3)_2$ | $CH_3$ | $CH_3$ | $CH_3$ | $OCH_3$ | N | |
| $SO_2CH_3$ | $CH_3$ | $CH_3$ | $OCH_3$ | $OCH_3$ | N | |
| $SO_2CH_3$ | $CH_3$ | $CH_3$ | $CH_3$ | $CH_3$ | CH | |
| $SO_2CH_3$ | $CH_3$ | $CH_3$ | $CH_3$ | $OCH_3$ | CH | |
| $SO_2CH_3$ | $CH_3$ | $CH_3$ | $OCH_3$ | $OCH_3$ | CH | |
| $SO_2CH_3$ | $CH_3$ | $CH_3$ | Cl | $OCH_3$ | CH | |

Table II

General Formula 1

($J=J_2$)

| $R_4$ | $R_5$ | $R_6$ | X | Y | Z | m.p.(°C) |
|---|---|---|---|---|---|---|
| H | $CH_3$ | H | $CH_3$ | $OCH_3$ | CH | |
| H | $CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_3$ | H | $OCH_3$ | $OCH_3$ | N | |
| H | $CO_2CH_3$ | H | $CH_3$ | $OCF_2H$ | CH | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_2CH_3$ | H | $CH_3$ | $CH_3$ | N | |
| H | $CO_2CH_2CH_3$ | H | $CH_3$ | $OCH_2CH_3$ | N | |
| H | $SO_2N(CH_3)_2$ | H | $OCH_3$ | $NHCH_3$ | N | |
| H | $SO_2N(CH_3)_2$ | H | $CH_3$ | $CH_3$ | CH | |
| H | $SO_2N(CH_3)_2$ | H | $CH_3$ | C≡CH | CH | |
| H | $SO_2N(CH_3)_2$ | H | $OCH_3$ | $OCH_3$ | CH | |
| H | $SO_2N(CH_3)_2$ | H | $CH_3$ | $CH_3$ | N | |
| H | $SO_2N(CH_3)_2$ | H | $CH_3$ | $OCH_3$ | N | |
| H | $SO_2N(CH_3)CH_2CH_3$ | H | $OCH_3$ | $OCH_3$ | N | |
| H | $SO_2N(OCH_3)CH_3$ | H | $CH_3$ | $CH_3$ | CH | |
| H | $SO_2N(CH_2CH_3)_2$ | H | $CH_3$ | $OCH_3$ | CH | |
| H | $SO_2N(CH_2CH_3)_2$ | H | Br | $OCH_3$ | CH | |
| HH | Cl | H | $OCH_3$ | $OCH_3$ | N | |
| H | $SO_2CH_3$ | H | $CH_3$ | $CH_2CH_3$ | CH | |
| H | $SO_2CH_3$ | H | $CH_3$ | $OCH_3$ | CH | |

Table II (continued)

| $R_4$ | $R_5$ | $R_6$ | X | Y | Z | m.p. (°C) |
|---|---|---|---|---|---|---|
| H | $SO_2CH_2CH_3$ | H | Cl | $OCH_3$ | CH | |
| H | $SO_2CH_2CH_3$ | H | $OCH_3$ | $OCH_3$ | N | |
| H | H | $CH_3$ | $CH_3$ | $CH_3$ | CH | |
| H | $CH_3$ | $CH_3$ | $CH_3$ | $OCH_3$ | CH | |
| H | $CH_3$ | $CH_3$ | $OCH_3$ | $OCH_3$ | CH | |
| H | $CO_2CH_3$ | $CH_3$ | $CH_3$ | $CH_3$ | N | |
| H | $CO_2CH_3$ | $CH_3$ | $CH_3$ | $OCH_3$ | N | |
| H | $CO_2CH_3$ | $CH_3$ | $OCH_3$ | $OCH_3$ | N | |
| H | $CO_2CH_3$ | $CH_3$ | $CH_3$ | $CH_3$ | CH | |
| H | $CO_2CH_3$ | $CH_3$ | $CH_3$ | $OCH_3$ | CH | |
| H | $CO_2CH_3$ | $CH_3$ | $OCH_3$ | $OCH_3$ | CH | |
| H | $CO_2CH_2CH_3$ | $CH_3$ | $CH_3$ | $CH_3$ | N | |
| H | $CO_2CH_2CH_3$ | $CH_3$ | $CH_3$ | $OCH_2CH_3$ | N | |
| H | $SO_2N(CH_3)_2$ | $CH_3$ | $OCH_3$ | $OCH_3$ | N | |
| H | $SO_2N(CH_3)_2$ | $CH_3$ | $CH_3$ | $CH_3$ | CH | |
| H | $SO_2N(CH_3)_2$ | $CH_3$ | $CH_3$ | $OCH_3$ | N | |
| H | $SO_2N(CH_3)CH_2CH_3$ | $CH_3$ | $OCH_3$ | $OCH_3$ | N | |
| H | $SO_2N(OCH_3)CH_3$ | $CH_3$ | $CH_3$ | $CH_3$ | CH | |
| H | $SO_2N(CH_2CH_3)_2$ | $CH_3$ | $CH_3$ | $OCH_2CH=CH_2$ | CH | |
| H | $SO_2N(CH_2CH_3)_2$ | $CH_3$ | $OCH_3$ | $OCH_3$ | CH | |
| H | $SO_2CH_3$ | $CH_3$ | $CH_3$ | $CH_3$ | N | |
| H | $SO_2CH_3$ | $CH_3$ | $CH_3$ | $OCH_3$ | N | |
| H | $SO_2CH_3$ | $CH_3$ | $OCH_3$ | $OCH_3$ | N | |
| H | $SO_2CH_3$ | $CH_3$ | $CH_3$ | $CH_2CH_3$ | CH | |
| H | Br | $CH_3$ | $CH_3$ | $OCH_3$ | CH | |
| H | $SO_2CH_3$ | $CH_3$ | $OCH_3$ | $OCH_3$ | CH | |
| H | $SO_2CH_2CH_3$ | $CH_3$ | Cl | $OCH_3$ | CH | |
| H | $SO_2CH_2CH_3$ | $CH_3$ | $OCH_3$ | $OCH_3$ | N | |

Table II (continued)

| $R_4$ | $R_5$ | $R_6$ | X | Y | Z | m.p. (°C) |
|---|---|---|---|---|---|---|
| H | H | $CH_2CH_2CH_3$ | $CH_3$ | $CH_3$ | CH | |
| H | $CH_3$ | $CH_2CH_2CH_3$ | $CH_3$ | $OCH_3$ | CH | |
| H | $CH_3$ | $CH_2CH_2CH_3$ | $OCH_3$ | $OCH_3$ | CH | |
| H | $CO_2CH_3$ | $CH_2CH_2CH_3$ | $CH_3$ | $CH_3$ | N | |
| H | $CO_2CH_3$ | $CH_2CH_2CH_3$ | $CH_3$ | $OCH_3$ | N | |
| H | $CO_2CH_3$ | $CH_2CH_2CH_3$ | $OCH_3$ | $OCH_3$ | N | |
| H | $CO_2CH_3$ | $CH_2CH_2CH_3$ | $CH_3$ | $CH_3$ | CH | |
| H | $CO_2CH_3$ | $CH_2CH_2CH_3$ | $CH_3$ | $OCH_3$ | CH | |
| H | $CO_2CH_3$ | $CH_2CH_2CH_3$ | $OCH_3$ | $SCH_3$ | CH | |
| H | $CO_2CH_2CH_3$ | $CH_2CH_2CH_3$ | $CH_3$ | $CH_3$ | N | |
| H | $CO_2CH_2CH_3$ | $CH_2CH_2CH_3$ | $CH_3$ | $OCH_2CH_3$ | N | |
| H | $SO_2N(CH_3)_2$ | $CH_2CH_2CH_3$ | $OCH_2CF_3$ | $OCH_3$ | N | |
| H | $SO_2N(CH_3)_2$ | $CH_2CH_2CH_3$ | $CH_3$ | $OCH_3$ | CH | |
| H | $SO_2N(CH_3)_2$ | $CH_2CH_2CH_3$ | $OCH_3$ | $OCH_3$ | CH | |
| H | $SO_2N(CH_3)_2$ | $CH_2CH_2CH_3$ | $CH_3$ | $OCH_3$ | N | |
| H | $SO_2N(CH_3)CH_2CH_3$ | $CH_2CH_2CH_3$ | $OCH_3$ | $OCH_3$ | N | |
| H | $SO_2N(OCH_3)CH_3$ | $CH_2CH_2CH_3$ | $CH_3$ | $CH_3$ | CH | |
| H | $SO_2N(CH_2CH_3)_2$ | $CH_2CH_2CH_3$ | $CH_3$ | $OCH_3$ | CH | |
| H | $SO_2N(CH_2CH_3)_2$ | $CH_2CH_2CH_3$ | $OCH_3$ | $OCH_3$ | CH | |
| H | $SO_2CH_3$ | $CH_2CH_2CH_3$ | $CH_3$ | $OCH_3$ | N | |
| H | $SO_2CH_3$ | $CH_2CH_2CH_3$ | $OCH_3$ | $OCH_3$ | N | |
| H | $SO_2CH_3$ | $CH_2CH_2CH_3$ | $CH_3$ | $CH_2CH_3$ | CH | |
| H | $SO_2CH_3$ | $CH_2CH_2CH_3$ | $CH_3$ | $OCH_3$ | CH | |
| H | $SO_2CH_3$ | $CH_2CH_2CH_3$ | $OCH_3$ | $OCH_3$ | CH | |
| H | $SO_2CH_2CH_3$ | $CH_2CH_2CH_3$ | Cl | $CH_2F$ | CH | |
| H | $SO_2CH_2CH_3$ | $CH_2CH_2CH_3$ | $OCH_3$ | $OCH_3$ | N | |
| $CH_3$ | H | $CH_3$ | $CH_3$ | $CH_3$ | CH | |
| $CH_3$ | $CH_3$ | $CH_3$ | $CH_3$ | $OCH_3$ | CH | |

Table II (continued)

| $R_4$ | $R_5$ | $R_6$ | X | Y | Z | m.p. (°C) |
|---|---|---|---|---|---|---|
| $CH_3$ | $CH_3$ | $CH_3$ | $OCH_3$ | $OCH_3$ | CH | |
| $CH_3$ | $CO_2CH_3$ | $CH_3$ | $CH_3$ | $CH_3$ | N | |
| $CH_3$ | $CO_2CH_3$ | $CH_3$ | $CH_3$ | $OCH_3$ | N | |
| $CH_3$ | $CO_2CH_3$ | $CH_3$ | $OCH_3$ | $OCH_3$ | N | |
| $CH_3$ | $CO_2CH_3$ | $CH_3$ | $CH_3$ | $CH_3$ | CH | |
| $CH_3$ | $CO_2CH_3$ | $CH_3$ | $CH_3$ | $OCH_3$ | CH | |
| $CH_3$ | $CO_2CH_3$ | $CH_3$ | $OCH_3$ | $NH_2$ | CH | |
| $CH_3$ | $CO_2CH_2CH_3$ | $CH_3$ | $CH_3$ | $CH_3$ | N | |
| $CH_3$ | $CO_2CH_2CH_3$ | $CH_3$ | $CH_3$ | $OCH_2CH_3$ | N | |
| $CH_3$ | $SO_2N(CH_3)_2$ | $CH_3$ | $OCH_3$ | $OCH_3$ | N | |
| $CH_3$ | $SO_2N(CH_3)_2$ | $CH_3$ | $CH_3$ | $OCH_3$ | CH | |
| $CH_3$ | $SO_2N(CH_3)_2$ | $CH_3$ | $OCH_3$ | $OCH_3$ | CH | |
| $CH_3$ | Br | $CH_3$ | $CH_3$ | $OCH_3$ | N | |
| $CH_3$ | $SO_2N(CH_3)CH_2CH_3$ | $CH_3$ | $OCH_3$ | $OCH_3$ | N | |
| $CH_3$ | $SO_2N(OCH_3)CH_3$ | $CH_3$ | $CH_3$ | $CH_3$ | CH | |
| $CH_3$ | $SO_2N(CH_2CH_3)_2$ | $CH_3$ | $CH_3$ | $OCH_3$ | CH | |
| $CH_3$ | $SO_2N(CH_2CH_3)_2$ | $CH_3$ | $OCH_3$ | $OCH_3$ | CH | |
| $CH_3$ | $SO_2CH_3$ | $CH_3$ | $OCH_3$ | $OCH_3$ | N | |
| $CH_3$ | $SO_2CH_3$ | $CH_3$ | $CH_3$ | $CH_2CH_3$ | CH | |
| $CH_3$ | $SO_2CH_3$ | $CH_3$ | $CH_3$ | $OCH_3$ | CH | |
| $CH_3$ | $SO_2CH_3$ | $CH_3$ | $OCF_2H$ | $CH_3$ | CH | |
| $CH_3$ | $SO_2CH_2CH_3$ | $CH_3$ | Cl | $OCH_3$ | CH | |
| $CH_3$ | $SO_2CH_2CH_3$ | $CH_3$ | $OCH_3$ | $OCH_3$ | N | |
| $CH_3$ | H | $CH_2CH_2CH_3$ | $CH_3$ | $CH_3$ | CH | |
| $CH_3$ | $CH_3$ | $CH_2CH_2CH_3$ | $OCH_3$ | $OCH_3$ | CH | |
| $CH_3$ | $CO_2CH_3$ | $CH_2CH_2CH_3$ | $CH_3$ | $CH_3$ | N | |

Table II (continued)

| $R_4$ | $R_5$ | $R_6$ | X | Y | Z | m.p. (°C) |
|---|---|---|---|---|---|---|
| $CH_3$ | $CO_2CH_3$ | $CH_2CH_2CH_3$ | $CH_3$ | $OCH_3$ | N | |
| $CH_3$ | $CO_2CH_3$ | $CH_2CH_2CH_3$ | $OCH_3$ | $OCH_3$ | N | |
| $CH_3$ | $CO_2CH_3$ | $CH_2CH_2CH_3$ | $CH_3$ | $CH_3$ | CH | |
| $CH_3$ | $CO_2CH_3$ | $CH_2CH_2CH_3$ | $CH_3$ | $OCH_3$ | CH | |
| $CH_3$ | $CO_2CH_3$ | $CH_2CH_2CH_3$ | $OCH_3$ | $OCH_3$ | CH | |
| $CH_3$ | $CO_2CH_2CH_3$ | $CH_2CH_2CH_3$ | $CH_3$ | $CH_3$ | N | |
| $CH_3$ | $CO_2CH_2CH_3$ | $CH_2CH_2CH_3$ | $CH_3$ | $OCH_2CH_3$ | N | |
| $CH_3$ | $SO_2N(CH_3)_2$ | $CH(CH_3)_2$ | $OCH_3$ | $OCH_3$ | N | |
| $CH_3$ | $SO_2N(CH_3)_2$ | $CH_2CH_2CH_3$ | $CH_3$ | $CH_3$ | CH | |
| $CH_3$ | $SO_2N(CH_3)_2$ | $CH_2CH_2CH_3$ | $CH_3$ | $OCH_3$ | CH | |
| $CH_3$ | $SO_2N(CH_3)_2$ | $CH_2CH_2CH_3$ | $OCH_3$ | $OCH_3$ | CH | |
| $CH_3$ | $SO_2N(CH_3)_2$ | $CH_2CH_2CH_3$ | $CH_3$ | $CH_3$ | N | |
| $CH_3$ | $SO_2N(CH_3)_2$ | $CH_2CH_2CH_3$ | $CH_3$ | $OCH_3$ | N | |
| $CH_3$ | $SO_2N(CH_3)CH_2CH_3$ | $CH_2CH_2CH_3$ | $OCH_3$ | $OCH_3$ | N | |
| $CH_3$ | $SO_2N(OCH_3)CH_3$ | $CH_2CH_2CH_3$ | $CH_3$ | $CH_3$ | CH | |
| $CH_3$ | $SO_2N(CH_2CH_3)_2$ | $CH_2CH_2CH_3$ | $CH_3$ | $OCH_3$ | CH | |
| $CH_3$ | $SO_2N(CH_2CH_3)_2$ | $CH_2CH_2CH_3$ | $OCH_3$ | $OCH_3$ | CH | |
| $CH_3$ | $SO_2CH_3$ | $CH_2CH_2CH_3$ | $OCH_3$ | $OCH_3$ | N | |
| $CH_3$ | $SO_2CH_3$ | $CH_2CH_2CH_3$ | $CH_3$ | $CH_2CH_3$ | CH | |
| $CH_3$ | $SO_2CH_3$ | $CH_2CH_2CH_3$ | $CH_3$ | $OCH_3$ | CH | |
| $CH_3$ | $SO_2CH_3$ | $CH_2CH_2CH_3$ | $OCH_3$ | $OCH_2CH_2F$ | CH | |
| $CH_3$ | $SO_2CH_2CH_3$ | $CH_2CH_2CH_3$ | Cl | $OCH_3$ | CH | |
| $CH_3$ | $SO_2CH_2CH_3$ | $CH_2CH_3$ | $OCH_3$ | $OCH_3$ | N | |
| H | H | $CO_2CH_3$ | $CH_3$ | $CH_2CH_3$ | CH | |
| H | H | $CO_2CH_3$ | $CH_3$ | $OCH_3$ | CH | |
| H | H | $CO_2CH_3$ | $OCH_3$ | $OCH_3$ | CH | |
| H | H | $CO_2CH_3$ | $CH_3$ | $CH_3$ | N | |
| H | H | $CO_2CH_3$ | $CH_3$ | $OCH_3$ | N | |
| H | H | $CO_2CH_3$ | $OCH_3$ | $OCH_3$ | N | |

Table II (continued)

| $R_4$ | $R_5$ | $R_6$ | X | Y | Z | m.p. (°C) |
|---|---|---|---|---|---|---|
| H | H | $CO_2CH_2CH_3$ | $CH_3$ | $CH_3$ | CH | |
| H | H | $CO_2CH_2CH_3$ | $CH_3$ | $SCH_3$ | CH | |
| H | H | $CO_2CH_2CH_3$ | $OCH_3$ | $OCH_3$ | CH | |
| H | H | $CO_2CH_2CH_3$ | $CH_3$ | $CH_2CH_2CH_3$ | N | |
| H | H | $CO_2CH_2CH_3$ | $CH_3$ | $OCH_3$ | N | |
| H | H | $SO_2N(CH_3)_2$ | $CH_3$ | $OCH_3$ | CH | |
| H | H | $SO_2N(CH_3)_2$ | $OCH_3$ | $OCH_3$ | CH | |
| H | H | $SO_2N(CH_3)_2$ | $CH_3$ | $CH_2CH_3$ | N | |
| H | H | $SO_2N(CH_3)_2$ | $CH_3$ | $OCH_3$ | N | |
| H | H | $SO_2N(CH_3)_2$ | $OCH_3$ | $OCH_3$ | N | |
| H | H | $SO_2N(CH_3)CH_2CH_3$ | $CH_3$ | $CH_3$ | CH | |
| H | H | $SO_2N(CH_2CH_3)_2$ | $CH_3$ | $OCH_3$ | CH | |
| H | H | $SO_2CH_3$ | $OCH_3$ | $OCH_3$ | CH | |
| H | H | $SO_2CH_3$ | $CH_3$ | $OCH_2CH_3$ | N | |
| H | H | $SO_2CH_3$ | $CH_3$ | $OCH_3$ | N | |
| H | H | $SO_2CH_3$ | $OCH_3$ | $OCH_3$ | N | |
| H | H | $SO_2CH_3$ | $CH_3$ | $CH_3$ | CH | |
| H | H | $SO_2CH_3$ | $CH_3$ | $OCH_3$ | CH | |
| H | H | $SO_2CH_2CH_3$ | $OCH_3$ | $OCH_3$ | CH | |
| H | H | $SO_2CH_2CH_3$ | Cl | $OCH_2CH_2OCH_3$ | CH | |
| $CH_3$ | H | $CO_2CH_3$ | $CH_3$ | $CH_3$ | CH | |
| $CH_3$ | H | $CO_2CH_3$ | $CH_3$ | $OCH_3$ | CH | |
| $CH_3$ | H | $CO_2CH_3$ | $OCH_3$ | $OCH_3$ | CH | |
| $CH_3$ | H | $CO_2CH_3$ | $CH_3$ | $CH_3$ | N | |
| $CH_3$ | H | $CO_2CH_3$ | $CH_3$ | $OCH_3$ | N | |
| $CH_3$ | H | $CO_2CH_3$ | $OCH_3$ | $OCH_3$ | N | |
| $CH_3$ | H | $CO_2CH_2CH_3$ | $CH_3$ | $CH_3$ | CH | |
| $CH_3$ | H | $CO_2CH_2CH_3$ | $CH_3$ | $OCH_3$ | CH | |

Table II (continued)

| $R_4$ | $R_5$ | $R_6$ | X | Y | Z | m.p. (°C) |
|---|---|---|---|---|---|---|
| $CH_3$ | H | $CO_2CH_2CH_3$ | $OCH_3$ | $OCH_3$ | CH | |
| $CH_3$ | H | $CO_2CH_2CH_3$ | $CH_3$ | $CH_3$ | N | |
| $CH_3$ | H | $CO_2CH_2CH_3$ | $CH_3$ | $OCH_3$ | N | |
| $CH_3$ | H | $SO_2N(CH_3)_2$ | $CH_3$ | $CH_3$ | CH | |
| $CH_3$ | H | $SO_2N(CH_3)_2$ | $CH_3$ | $OCH_3$ | CH | |
| $CH_3$ | H | $SO_2N(CH_3)_2$ | $OCH_3$ | $OCH_3$ | CH | |
| $CH_3$ | H | $SO_2N(CH_3)_2$ | $CH_3$ | $CH_2CH_3$ | N | |
| $CH_3$ | H | $SO_2N(CH_3)_2$ | $CH_3$ | $OCH_3$ | N | |
| $CH_3$ | H | $SO_2N(CH_3)_2$ | $OCH_3$ | $OCH_3$ | N | |
| $CH_3$ | H | $SO_2N(CH_3)CH_2CH_3$ | $CH_3$ | $CH_3$ | CH | |
| $CH_3$ | H | $SO_2N(CH_2CH_3)_2$ | $CH_3$ | $OCH_3$ | CH | |
| $CH_3$ | H | $SO_2CH_3$ | $OCH_3$ | $OCH_3$ | CH | |
| $CH_3$ | H | $SO_2CH_3$ | $CH_3$ | $OCH_2CH_3$ | N | |
| $CH_3$ | H | $SO_2CH_3$ | $CH_3$ | $OCH_3$ | N | |
| $CH_3$ | H | $SO_2CH_3$ | $OCH_3$ | $OCH_3$ | N | |
| $CH_3$ | H | $SO_2CH_3$ | $CH_3$ | $CH_3$ | CH | |
| $CH_3$ | H | $SO_2CH_3$ | $CH_3$ | $OCH_3$ | CH | |
| $CH_3$ | H | $SO_2CH_2CH_3$ | $OCH_3$ | $OCH_3$ | CH | |
| $CH_3$ | H | $SO_2CH_2CH_3$ | $OCH_3$ | $CH_2CH_3$ | CH | |
| $CH_3$ | H | $SO_2CH_2CH_3$ | Cl | $OCH_3$ | CH | |
| $CH_3$ | $NO_2$ | H | $CH_3$ | $OCH_3$ | CH | |
| $CH_3$ | $NO_2$ | $CH_3$ | $CH_3$ | $OCH_3$ | N | |

Table III

General Formula 1

$(J=J_3)$

| $R_7$ | $R_8$ | X | Y | Z | m.p. (°C) |
|---|---|---|---|---|---|
| H | $CO_2CH_3$ | $CH_3$ | $CH_3$ | CH | |
| H | $CO_2CH_3$ | $CH_3$ | $OCH_3$ | CH | |
| H | $CO_2CH_3$ | $OCH_3$ | $OCH_3$ | CH | |
| H | $CO_2CH_3$ | $CH_3$ | $CH_3$ | N | |
| H | $CO_2CH_3$ | $CH_3$ | $OCH_3$ | N | |
| H | $CO_2CH_3$ | $OCH_3$ | $OCH_3$ | N | |
| H | $CO_2CH_2CH_3$ | $CH_3$ | $CH_3$ | CH | |
| H | $SO_2N(CH_3)_2$ | $OCH_3$ | $CH_2CH_3$ | N | |
| H | $SO_2N(CH_3)_2$ | $CH_3$ | $OCH_3$ | N | |
| H | $SO_2N(CH_3)_2$ | $OCH_3$ | $OCH_3$ | N | |
| H | $SO_2N(CH_3)_2$ | $CH_3$ | $CH_3$ | CH | |
| H | $SO_2N(CH_3)_2$ | Br | $OCH_3$ | CH | |
| H | $SO_2N(CH_3)_2$ | $OCH_3$ | $OCH_3$ | CH | |
| H | $SO_2N(CH_3)OCH_3$ | $CH_3$ | $CH_3$ | N | |
| H | $SO_2N(CH_3)CH_2CH_3$ | $CH_3$ | $OCH_2CH_3$ | N | |
| H | $SO_2CH_3$ | $OCH_3$ | $OCH_3$ | N | |
| H | $SO_2CH_3$ | $CH_3$ | $CH_3$ | CH | |
| H | $SO_2CH_3$ | $CH_3$ | $OCH_3$ | CH | |
| H | $SO_2CH_3$ | $OCH_3$ | $OCH_3$ | CH | |
| H | $SO_2CH_3$ | $CH_3$ | $CH_3$ | N | |
| H | $SO_2CH_3$ | $CH_3$ | $OCH_3$ | N | |
| H | $SO_2CH_2CH_3$ | $OCH_3$ | $SCH_3$ | N | |

Table III (continued)

| $R_7$ | $R_8$ | X | Y | Z | m.p. (°C) |
|---|---|---|---|---|---|
| H | $NO_2$ | $CH_3$ | $CH_3$ | CH | |
| H | $NO_2$ | $CH_3$ | $OCH_3$ | CH | |
| H | $NO_2$ | $OCH_3$ | $OCH_3$ | CH | |
| H | $NO_2$ | Cl | $OCH_3$ | CH | |
| $CH_3$ | $CO_2CH_3$ | $CH_3$ | $CH_3$ | CH | |
| $CH_3$ | $CO_2CH_3$ | $CH_3$ | $OCH_3$ | CH | |
| $CH_3$ | $CO_2CH_3$ | $OCH_3$ | $OCH_3$ | CH | |
| $CH_3$ | $CO_2CH_3$ | $CH_3$ | $CH_3$ | N | |
| $CH_3$ | $CO_2CH_3$ | $CH_3$ | $OCH_3$ | N | |
| $CH_3$ | $CO_2CH_3$ | $OCH_3$ | $OCH_2CH_2F$ | N | |
| $CH_3$ | $CO_2CH_2CH_3$ | $CH_3$ | $CH_3$ | CH | |
| $CH_3$ | $CO_2CH_2CH_3$ | $CH_3$ | $OCH_3$ | CH | |
| $CH_3$ | $CO_2CH_2CH_3$ | $OCF_2H$ | $OCH_3$ | CH | |
| $CH_3$ | $SO_2N(CH_3)_2$ | $OCH_3$ | $CH_2CH_3$ | N | |
| $CH_3$ | $SO_2N(CH_3)_2$ | $CH_3$ | $OCH_3$ | N | |
| $CH_3$ | $SO_2N(CH_3)_2$ | $OCH_3$ | $OCH_3$ | N | |
| $CH_3$ | $SO_2N(CH_3)_2$ | $OCH_3$ | $OCH_3$ | CH | |
| $CH_3$ | $SO_2N(CH_3)OCH_3$ | $CH_3$ | $CH_3$ | N | |
| $CH_3$ | $SO_2N(CH_3)CH_2CH_3$ | $CH_3$ | $OCH_2CH_3$ | N | |
| $CH_3$ | $SO_2CH_3$ | $OCH_3$ | $OCH_3$ | N | |
| $CH_3$ | $SO_2CH_3$ | $CH_3$ | $CH_3$ | CH | |
| $CH_3$ | $SO_2CH_3$ | $CH_3$ | $OCH_3$ | CH | |
| $CH_3$ | $SO_2CH_3$ | $OCH_2CF_3$ | $OCH_3$ | CH | |
| $CH_3$ | $SO_2CH_3$ | $CH_3$ | $CH_3$ | N | |
| $CH_3$ | $SO_2CH_3$ | $CH_3$ | $OCH_3$ | N | |
| $CH_3$ | $SO_2CH_2CH_3$ | $OCH_3$ | $OCH_3$ | N | |
| $CH_3$ | $NO_2$ | $CH_3$ | Δ | CH | |

Table III (continued)

| $R_7$ | $R_8$ | X | Y | Z | m.p. (°C) |
|---|---|---|---|---|---|
| $CH_3$ | $NO_2$ | $OCH_3$ | $OCH_3$ | CH | |
| $CH_3$ | $NO_2$ | Cl | $OCH_3$ | CH | |
| $CH_3$ | $NO_2$ | $OCH_3$ | $CH_2OCH_3$ | CH | |
| H | $CO_2CH_3$ | $OCH_3$ | $CH_2F$ | CH | |
| H | $CO_2CH_3$ | $OCH_3$ | $C\equiv CH$ | CH | |
| H | $CO_2CH_3$ | $OCH_3$ | $CF_3$ | CH | |
| H | $CO_2CH_3$ | $OCH_3$ | $NHCH_3$ | CH | |
| H | $CO_2CH_3$ | $OCH_3$ | $N(CH_3)_2$ | CH | |
| H | $CO_2CH_3$ | $OCH_3$ | $OCH_2CH{=}CH_2$ | CH | |

Table IV

General Formula 1

(J is $J_4$)

| $R_9$ | $R_{10}$ | $R_{11}$ | X | Y | Z | m.p.(°C) |
|---|---|---|---|---|---|---|
| H | $CO_2CH_3$ | H | $CH_3$ | $CH_3$ | CH | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_2CH_3$ | H | $OCH_3$ | $OCH_3$ | N | |
| H | $CO_2CH_3$ | $CH_3$ | $CH_3$ | $CF_3$ | CH | |
| $CH_3$ | $CO_2CH_3$ | H | $CH_3$ | $OCH_3$ | CH | |
| $CH_3$ | $CO_2CH_3$ | H | $CH_3$ | $OCH_2CH_3$ | CH | |
| $CH_3$ | $CO_2CH_3$ | H | Cl | $OCH_3$ | CH | |
| $CH_3$ | $CO_2CH_3$ | $CH_3$ | $CH_3$ | $CH_3$ | CH | |
| $CH_3$ | $CO_2CH_3$ | $CH_3$ | $CH_3$ | $OCH_3$ | CH | |
| $CH_3$ | $CO_2CH_3$ | $CH_3$ | $OCH_3$ | $OCH_2CH_2OCH_3$ | CH | |
| $CH_3$ | $CO_2CH_3$ | $CH_3$ | $CH_3$ | $CH_3$ | N | |
| $CH_3$ | $CO_2CH_3$ | $CH_3$ | $CH_3$ | $OCH_3$ | N | |
| $CH_3$ | $CO_2CH_3$ | $CH_3$ | $OCH_3$ | $OCH_3$ | N | |
| $CH_3$ | $CO_2CH_3CH$ | H | $CH_3$ | $OCH_3$ | CH | |
| $CH_3$ | $CO_2CH_3CH$ | H | $OCH_3$ | $OCH_3$ | CH | 198-200 |
| $CH_3$ | $CO_2CH_3CH$ | H | $CH_3$ | $OCH_3$ | N | |
| $CH_3$ | $CO_2CH_3CH$ | H | $OCH_3$ | $OCH_3$ | N | 199-201 |
| $CH_3$ | $CO_2CH_3CH$ | H | Cl | $OCH_3$ | CH | |
| $CH_3$ | $CO_2CH_2CH_3$ | $CH_3$ | $OCF_2H$ | $CH_3$ | CH | |
| $CH_3$ | $CO_2CH_2CH_3$ | $CH_3$ | $CH_3$ | $OCH_3$ | CH | |

Table IV (continued)

| $R_9$ | $R_{10}$ | $R_{11}$ | X | Y | Z | m.p. (°C) |
|---|---|---|---|---|---|---|
| $CH_3$ | $CO_2CH_2CH_3$ | $CH_3$ | $OCH_3$ | $OCH_3$ | CH | 190 |
| $CH_3$ | $CO_2CH_2CH_3$ | $CH_3$ | $CH_3$ | $CH_3$ | N | |
| $CH_3$ | $CO_2CH_2CH_3$ | $CH_3$ | $CH_3$ | $OCH_3$ | N | 183-184 |
| $CH_3$ | $CO_2CH_2CH_3$ | $CH_3$ | $OCH_3$ | $OCH_3$ | N | 165-167 |
| $CH_3$ | $CO_2CH_2CH_3$ | $CH_3$ | Cl | $OCH_3$ | CH | 140.5-144.5 |
| $CH_3$ | $CO_2CH_2CH_3$ | $CH_3$ | $OCH_3$ | $OCH_2CH_3$ | CH | |
| $CH_2CH_2CH_3$ | $CO_2CH_2CH_3$ | $CH_3$ | $OCH_3$ | $OCH_3$ | N | |
| $CH_2CH_2CH_3$ | $CO_2CH_2CH_3$ | $CH_3$ | $OCH_3$ | $OCH_3$ | CH | |
| $CH_2CH_2CH_2$ | $CO_2CH_2CH_3$ | $CH_3$ | $CH_3$ | $OCH_3$ | CH | |
| $CH_2CH_2CH_2$ | $CO_2CH_2CH_3$ | H | $OCH_3$ | $CH_2F$ | CH | |
| $CH_2CH_2CH_3$ | $CO_2CH_2CH_3$ | H | $OCH_3$ | $OCH_3$ | N | |
| $CH_2CH_2CH_2$ | $CO_2CH_3$ | H | $OCH_3$ | $OCH_3$ | CH | |
| $CH_2CH_2CH_3$ | $CO_2CH_3$ | $CH_3$ | $OCH_3$ | $OCH_3$ | N | |
| $C_6H_5$ | $CO_2CH_3$ | H | $OCH_3$ | $OCH_3$ | CH | |
| $C_6H_5$ | $CO_2Et$ | $CH_3$ | $CH_3$ | $OCH_3$ | N | |
| $C_6H_5$ | $CO_2Et$ | $CH_3$ | $OCH_3$ | $OCH_3$ | N | |
| $C_6H_5$ | $CO_2CH_3$ | $CH_3$ | $CH_3$ | $CH_3$ | CH | |
| $C_6H_5$ | $CO_2Et$ | $CH_3$ | $CH_3$ | $SCH_3$ | CH | |
| $C_6H_5$ | $CO_2Et$ | $CH_3$ | $OCH_3$ | $OCH_3$ | CH | |
| H | $SO_2N(CH_3)_2$ | H | Br | $OCH_3$ | CH | |
| H | $SO_2N(CH_2CH_3)_2$ | H | $OCH_3$ | $OCH_2CH_3$ | N | |
| H | $SO_2N(CH_3)OCH_3$ | H | $CH_3$ | $OCH_3$ | CH | |
| H | $SO_2N(CH_3)_2$ | $CH_3$ | $OCH_3$ | $OCH_3$ | CH | |
| H | $SO_2N(CH_2CH_3)_2$ | $CH_3$ | $CH_3$ | $CH_2F$ | N | |
| H | $SO_2N(OCH_3)CH_3$ | $CH_3$ | $CH_3$ | $OCH_3$ | N | |
| H | $NO_2$ | $CH_3$ | $OCH_3$ | $OCH_3$ | N | |
| $CH_3$ | $SO_2N(CH_3)_2$ | H | $CH_3$ | $CH_3$ | CH | |
| $CH_3$ | $SO_2N(CH_3)_2$ | $CH_3$ | $CH_3$ | $OCH_3$ | CH | |
| $CH_3$ | $SO_2N(CH_3)CH_2CH_3$ | $CH_3$ | $CH_3$ | $CH_3$ | N | |

Table IV (continued)

| $R_9$ | $R_{10}$ | $R_{11}$ | X | Y | Z | m.p. (°C) |
|---|---|---|---|---|---|---|
| $CH_3$ | $NO_2$ | H | $CH_3$ | $OCH_2CH_3$ | N | |
| $CH_3$ | $NO_2$ | $CH_3$ | $OCH_3$ | $OCH_3$ | N | |
| $CH_2CH_2CH_3$ | $SO_2N(CH_3)_2$ | H | $CH_3$ | $CH_3$ | CH | |
| $CH_2CH_2CH_3$ | $SO_2N(CH_3)_2$ | $CH_3$ | $CH_3$ | $OCH_3$ | CH | |
| $CH_2CH_2CH_3$ | $SO_2N(CH_3)OCH_3$ | H | $OCH_3$ | $OCH_3$ | CH | |
| $CH_2CH_2CH_3$ | $SO_2N(CH_3)OCH_3$ | $CH_3$ | $CH_3$ | $CH_3$ | N | |
| $CH_2CH_2CH_3$ | $NO_2$ | H | $CH_3$ | $OCH_3$ | N | |
| $CH_2CH_2CH_3$ | $NO_2$ | $CH_3$ | $OCH_3$ | $OCH_3$ | N | |
| $C_6H_5$ | $SO_2N(CH_3)_2$ | H | $CH_3$ | $CH_3$ | CH | |
| $C_6H_5$ | $SO_2N(CH_3)CH_2CH_3$ | $CH_3$ | $CH_3$ | $OCH_3$ | CH | |
| $C_6H_5$ | $SO_2N(CH_3)OCH_3$ | H | $OCH_3$ | $OCH_3$ | CH | |
| $C_6H_5$ | $SO_2N(CH_3)OCH_3$ | $CH_3$ | $CH_3$ | $CH_2CH_3$ | N | |
| $C_6H_5$ | $NO_2$ | H | $CH_3$ | $OCH_3$ | N | |
| $C_6H_5$ | $NO_2$ | $CH_3$ | $OCH_3$ | $OCH_3$ | N | |
| $C_6H_5$ | $NO_2$ | $CH_3$ | $CH_3$ | $CH_3$ | CH | |
| H | $SO_2CH_2CH_3$ | H | $CH_3$ | $N(CH_3)_2$ | N | |
| H | $SO_2CH_2CH_3$ | $CH_3$ | $OCH_3$ | $OCH_3$ | N | |
| $CH_3$ | $SO_2CH_2CH_3$ | H | $OCH_2CF_3$ | $CH_3$ | CH | |
| $CH_2CH_3$ | $SO_2CH_2CH_3$ | $CH_3$ | $CH_3$ | $OCH_3$ | CH | |
| $CH(CH_3)_2$ | $SO_2CH_2CH_3$ | H | $OCH_3$ | $OCH_3$ | CH | |
| $C_6H_5$ | $SO_2CH_2CH_3$ | $CH_3$ | $CH_3$ | $CH_2CH_2CH_3$ | N | |

Table V

General Formula 1

(J is $J_5$)

| $R_{10}$ | $R_{11}$ | X | Y | Z | m.p.(°C) |
|---|---|---|---|---|---|
| $CO_2CH_3$ | H | $CH_3$ | $CH_3$ | CH | |
| $CO_2CH_3$ | H | $CH_3$ | $OCH_3$ | CH | |
| $CO_2CH_3$ | H | $OCH_3$ | $OCH_3$ | CH | |
| $CO_2CH_3$ | H | Cl | $OCH_3$ | CH | |
| $CO_2CH_3$ | H | $CH_3$ | $OCH_3$ | N | |
| $CO_2CH_3$ | H | $OCH_3$ | $OCH_3$ | N | |
| $CO_2CH_3$ | $CH_3$ | $CH_3$ | $CH_3$ | CH | |
| $CO_2CH_3$ | $CH_3$ | $CH_3$ | $OCH_3$ | CH | |
| $CO_2CH_3$ | $CH_3$ | $OCH_3$ | $OCH_3$ | CH | |
| $CO_2CH_3$ | $CH_3$ | $CH_3$ | $OCH_2CH_3$ | CH | |
| $CO_2CH_3$ | $CH_3$ | $CH_3$ | $OCH_3$ | N | |
| $CO_2CH_3$ | $CH_3$ | $OCH_3$ | $OCH_3$ | N | |
| $CO_2Et$ | H | $CH_3$ | $CH_3$ | CH | |
| $CO_2Et$ | $CH_3$ | $CH_3$ | $OCH_3$ | CH | |
| $CO_2Et$ | $CH_3$ | $OCH_3$ | $OCH_3$ | CH | 180-183 |
| $CO_2Et$ | $CH_3$ | Br | $OCH_3$ | CH | |
| $CO_2Et$ | $CH_3$ | $CH_3$ | $OCH_2CH_3$ | CH | |
| $CO_2Et$ | $CH_3$ | $CH_3$ | $CH_3$ | N | |
| $CO_2Et$ | $CH_3$ | $CH_3$ | $OCH_3$ | N | 154-157 |
| $CO_2Et$ | $CH_3$ | $OCH_3$ | $OCH_3$ | N | 188-188.5 |
| $CO_2Et$ | $CH_3$ | $OCH_3$ | $OCH_2CH_3$ | N | |

Table V (continued)

| $R_{10}$ | $R_{11}$ | X | Y | Z | m.p.(°C) |
|---|---|---|---|---|---|
| $SO_2N(CH_3)_2$ | H | $OCF_2H$ | $CH_3$ | CH | |
| $SO_2N(CH_3)_2$ | H | $CH_3$ | $OCH_3$ | CH | |
| $SO_2N(CH_3)_2$ | H | $OCH_3$ | $OCH_3$ | CH | |
| $SO_2N(CH_3)_2$ | H | $CH_3$ | $CH_3$ | N | |
| $SO_2N(CH_3)_2$ | H | $CH_3$ | $OCH_3$ | N | |
| $SO_2NCH_3(OCH_3)_2$ | H | $OCH_3$ | $OCH_3$ | N | |
| $SO_2N(CH_2CH_3)_2$ | $CH_3$ | $CH_3$ | $CH_3$ | CH | |
| $SO_2N(CH_2CH_3)_2$ | H | $CH_3$ | $OCH_3$ | CH | |
| $SO_2N(CH_2CH_3)_2$ | H | $OCH_3$ | $OCH_3$ | CH | |
| $SO_2N(CH_2CH_3)_2$ | H | $CH_3$ | $CH_3$ | N | |
| $SO_2N(CH_2CH_3)_2$ | H | $CH_3$ | $OCH_3$ | N | |
| $SO_2N(CH_3)CH_2CH_3$ | H | $OCH_3$ | $OCH_3$ | N | |
| $SO_2CH_3$ | H | $CH_3$ | $CH_3$ | CH | |
| $SO_2CH_3$ | H | $CH_3$ | $OCH_3$ | CH | |
| $SO_2CH_3$ | H | $OCH_3$ | $OCH_3$ | CH | |
| $SO_2CH_3$ | H | $OCH_3$ | $CH_3$ | N | |
| $SO_2CH_3$ | H | $CH_3$ | $NHCH_3$ | N | |
| $SO_2CH_3$ | $CH_3$ | $OCH_2CF_3$ | $OCH_3$ | N | |
| $SO_2CH_3$ | $CH_3$ | $OCH_3$ | $OCH_3$ | N | |
| $SO_2CH_2CH_3$ | H | $OCH_3$ | $OCH_3$ | CH | |
| $SO_2CH_2CH_3$ | H | $CH_3$ | $OCH_3$ | CH | |
| $SO_2CH_2CH_3$ | $CH_3$ | $OCH_3$ | $OCH_3$ | CH | |
| $SO_2CH_2CH_3$ | $CH_3$ | $CH_3$ | $CH_3$ | N | |
| $SO_2CH_2CH_3$ | $CH_3$ | $CH_3$ | $OCH_3$ | N | |
| $SO_2CH_2CH_3$ | $CH_3$ | $OCH_3$ | $OCH_3$ | N | |
| $SO_2CH_2CH_3$ | $CH_3$ | $OCH_3$ | $CH_2CH_3$ | CH | |
| $SO_2CH_2CH_3$ | $CH_3$ | $CH_3$ | $OCH_2CH_3$ | CH | |
| $SO_2CH_2CH_3$ | $CH_3$ | Cl | $OCH_3$ | CH | |
| $NO_2$ | H | $OCH_3$ | $CH_3$ | CH | |
| $NO_2$ | H | $OCH_3$ | $CH_3$ | N | |
| $NO_2$ | H | $OCH_3$ | $OCH_2C{\equiv}CH$ | CH | |

Table VI

General Formula 1

(J is $J_6$)

| $R_{12}$ | $R_{13}$ | $R_{14}$ | X | Y | Z | m.p. (°C) |
|---|---|---|---|---|---|---|
| $CO_2CH_3$ | H | H | $CH_3$ | $OCH_3$ | CH | |
| $CO_2CH_3$ | H | H | $OCH_3$ | $OCH_3$ | CH | |
| $CO_2CH_3$ | H | H | $CH_3$ | $CH_3$ | N | |
| $CO_2CH_3$ | H | H | $CH_3$ | $OCH_3$ | N | |
| $CO_2CH_3$ | H | H | $OCH_3$ | $OCH_3$ | N | |
| $CO_2CH_3$ | H | H | $CH_3$ | $CH_3$ | CH | |
| $CO_2CH_2CH_3$ | H | H | $CH_3$ | $OCH_3$ | CH | |
| $CO_2CH_2CH_3$ | H | H | $OCH_2CF_3$ | $OCH_3$ | CH | |
| $CO_2CH_2CH_3$ | H | H | $CH_3$ | $CH_3$ | N | |
| $CO_2CH_2CH_3$ | H | H | $CH_3$ | $OCH_3$ | N | |
| $CO_2CH_2CH_3$ | H | H | $OCH_3$ | $OCH_3$ | N | |
| $SO_2N(CH_3)_2$ | H | H | $CH_3$ | $OCH_3$ | CH | |
| $SO_2N(CH_3)_2$ | H | H | $OCH_3$ | $OCH_3$ | CH | |
| $SO_2N(CH_3)_2$ | H | H | $CH_3$ | $CH_3$ | N | |
| $SO_2N(CH_3)_2$ | H | H | $CH_3$ | $OCH_3$ | N | |
| $SO_2N(CH_3)_2$ | H | H | $OCH_3$ | $OCH_3$ | N | |
| $SO_2N(CH_3)_2$ | H | H | $OCH_3$ | $CH_2CH_3$ | N | |
| $SO_2N(CH_3)OCH_3$ | H | H | $OCH_3$ | $OCH_2CH_3$ | CH | |
| $SO_2N(CH_3)OCH_3$ | H | H | Cl | $OCH_3$ | CH | |
| $CO_2CH_3$ | H | $CH_3$ | $CH_3$ | $OCH_3$ | CH | |
| $CO_2CH_3$ | H | $CH_3$ | $OCH_3$ | $OCH_3$ | CH | |
| $CO_2CH_3$ | H | $CH_3$ | $CH_3$ | $CH_3$ | N | |

Table VI (continued)

| $R_{12}$ | $R_{13}$ | $R_{14}$ | X | Y | Z | m.p. (°C) |
|---|---|---|---|---|---|---|
| $CO_2CH_3$ | H | $CH_3$ | $CH_3$ | $OCH_3$ | N | |
| $CO_2CH_3$ | H | $CH_3$ | $OCH_3$ | $OCH_3$ | N | |
| $CO_2CH_3$ | H | $CH_3$ | $CH_3$ | $CH_3$ | CH | |
| $CO_2CH_2CH_3$ | H | $CH_3$ | $CH_3$ | $CH_3$ | CH | |
| $CO_2CH_2CH_3$ | H | $CH_3$ | $CH_3$ | $OCH_3$ | CH | |
| $CO_2CH_2CH_3$ | H | $CH_3$ | $OCH_3$ | $OCH_3$ | CH | |
| $CO_2CH_2CH_3$ | H | $CH_3$ | $CH_3$ | $CH_3$ | N | |
| $CO_2CH_2CH_3$ | H | $CH_3$ | $CH_3$ | $OCH_3$ | N | |
| $CO_2CH_2CH_3$ | H | $CH_3$ | $OCH_3$ | $OCH_3$ | N | |
| $SO_2N(CH_3)_2$ | H | $CH_3$ | $CH_3$ | $OCH_3$ | CH | |
| $SO_2N(CH_3)_2$ | H | $CH_3$ | $OCH_3$ | $OCH_3$ | CH | |
| $SO_2N(CH_3)_2$ | H | $CH_3$ | $CH_3$ | $CH_3$ | N | |
| $SO_2N(CH_3)_2$ | H | $CH_3$ | $CH_3$ | $OCH_3$ | N | |
| $SO_2N(CH_3)_2$ | H | $CH_3$ | $OCH_3$ | $OCH_3$ | N | |
| $SO_2N(CH_3)_2$ | H | $CH_3$ | $OCH_3$ | $CH_2CH_3$ | N | |
| $SO_2N(CH_3)OCH_3$ | H | $CH_3$ | $OCH_3$ | $OCH_2CH_2F$ | CH | |
| $SO_2N(CH_3)OCH_3$ | H | $CH_3$ | Cl | $OCH_3$ | CH | |
| $SO_2N(CH_2CH_3)_2$ | H | $CH_3$ | $OCH_3$ | $OCH_3$ | CH | |
| $SO_2CH_3$ | H | $CH_3$ | $OCH_3$ | $CH_3$ | CH | |
| $SO_2CH_2CH_3$ | H | $CH_3$ | $OCH_3$ | $OCH_3$ | N | |
| $NO_2$ | H | $CH_3$ | $OCH_3$ | $OCH_2CH_3$ | CH | |
| $SO_2CH_3$ | H | H | $OCH_3$ | $OCH_3$ | N | |
| $NO_2$ | H | H | $OCH_3$ | $OCH_3$ | N | |
| $NO_2$ | H | H | $OCH_3$ | Δ | N | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2CH_3$ | H | $CH_3$ | $CH_3$ | N | |
| H | $CO_2CH_3$ | H | $CH_3$ | $OCH_3$ | N | |
| H | $CO_2CH_3$ | H | $OCH_3$ | $OCH_3$ | N | |
| H | $CO_2CH_3$ | H | $CH_3$ | $CH_2F$ | CH | |

Table VI (continued)

| $R_{12}$ | $R_{13}$ | $R_{14}$ | X | Y | Z | m.p. (°C) |
|---|---|---|---|---|---|---|
| H | $CO_2CH_2CH_3$ | H | $CH_3$ | $OCH_3$ | CH | |
| H | $CO_2CH_2CH_3$ | H | $OCH_3$ | $OCH_3$ | CH | |
| H | $CO_2CH_2CH_3$ | H | $CH_3$ | $CH_3$ | N | |
| H | $CO_2CH_2CH_3$ | H | $CH_3$ | $OCH_3$ | N | |
| H | $CO_2CH_2CH_3$ | H | $OCH_3$ | $OCH_3$ | N | |
| H | $CO_2CH_2CH_3$ | H | $CH_3$ | $CH_3$ | CH | |
| H | $SO_2N(CH_3)_2$ | H | $CH_3$ | $OCH_3CH_2OCH_3$ | CH | |
| H | $SO_2N(CH_3)_2$ | H | $OCH_3$ | $OCH_3$ | CH | |
| H | $SO_2N(CH_3)_2$ | H | $CH_3$ | $CH_3$ | N | |
| H | $SO_2N(CH_3)_2$ | H | $CH_3$ | $N(CH_3)_2$ | N | |
| H | $SO_2N(CH_3)_2$ | H | $OCH_3$ | $OCH_3$ | N | |
| H | $SO_2N(CH_3)_2$ | H | $OCH_3$ | $CH_2CH_3$ | N | |
| H | $SO_2N(CH_3)OCH_3$ | H | $OCH_3$ | $OCH_2CH_3$ | CH | |
| H | $SO_2N(CH_3)OCH_3$ | H | Cl | $OCH_3$ | CH | |
| H | $CO_2CH_3$ | $CH_3$ | $CH_3$ | $OCH_2C{\equiv}CH$ | CH | |
| H | $CO_2CH_3$ | $CH_3$ | $OCH_3$ | $OCH_3$ | CH | |
| H | $CO_2CH_3$ | $CH_3$ | $CH_3$ | $CH_3$ | N | |
| H | $CO_2CH_3$ | $CH_3$ | $CH_3$ | $OCH_3$ | N | |
| H | $CO_2CH_3$ | $CH_3$ | $OCH_3$ | $OCH_3$ | N | |
| H | $CO_2CH_3$ | $CH_3$ | $CH_3$ | $\Delta$ | CH | |
| H | $CO_2CH_2CH_3$ | $CH_3$ | $CH_3$ | $CF_3$ | CH | |
| H | $CO_2CH_2CH_3$ | $CH_3$ | $CH_3$ | $OCH_3$ | CH | |
| H | $CO_2CH_2CH_3$ | $CH_3$ | $OCH_3$ | $OCH_3$ | CH | |
| H | $CO_2CH_2CH_3$ | $CH_3$ | $CH_3$ | $CH_3$ | N | |
| H | $SO_2N(CH_3)_2$ | $CH_3$ | $CH_3$ | $OCH_3$ | CH | |
| H | $SO_2N(CH_3)_2$ | $CH_3$ | $OCH_3$ | $OCH_3$ | CH | |
| H | $SO_2N(CH_3)_2$ | $CH_3$ | $CH_3$ | $CH_3$ | N | |
| H | $SO_2N(CH_3)_2$ | $CH_3$ | $CH_3$ | $OCH_3$ | N | |
| H | $SO_2N(CH_3)_2$ | $CH_3$ | $OCH_3$ | $OCH_3$ | N | |

Table VI (continued)

| $R_{12}$ | $R_{13}$ | $R_{14}$ | X | Y | Z | m.p. (°C) |
|---|---|---|---|---|---|---|
| H | $SO_2N(CH_3)_2$ | $CH_3$ | $OCH_3$ | $CH_2CH_3$ | N | |
| H | $SO_2N(CH_3)OCH_3$ | $CH_3$ | $OCH_3$ | $OCH_2CH_3$ | CH | |
| H | $SO_2N(CH_3)OCH_3$ | $CH_3$ | Cl | $OCH_3$ | CH | |
| H | $SO_2N(CH_2CH_3)_2$ | $CH_3$ | $OCH_3$ | $OCH_3$ | CH | |
| H | $SO_2CH_3$ | $CH_3$ | $OCH_3$ | $CH_3$ | CH | |
| H | $SO_2CH_2CH_3$ | $CH_3$ | $OCH_3$ | $OCH_3$ | N | |
| H | $NO_2$ | $CH_3$ | $OCH_3$ | $OCH_2CH_3$ | CH | |
| H | $SO_2CH_3$ | H | $OCH_3$ | $OCH_3$ | N | |
| H | $NO_2$ | H | $OCH_3$ | $SCH_3$ | N | |

Table VII

General Formula 1

| J | $R_5$ | $R_{19}$ | R | X | Y | Z | m.p.(°C) |
|---|---|---|---|---|---|---|---|
| J-7 | H | $CH_3$ | H | $OCH_3$ | $CH_3$ | CH | |
| J-7 | H | Cl | H | $OCH_3$ | $CH_3$ | CH | |
| J-7 | H | $NO_2$ | H | $OCH_3$ | $CH_3$ | CH | |
| J-7 | H | $SCH_2CH_3$ | H | $OCH_3$ | $N(CH_3)_2$ | CH | |
| J-7 | H | $SO_2CH_3$ | H | $OCH_2CF_3$ | $CH_3$ | N | |
| J-7 | $CH_3$ | $SO_2CH_2CH_3$ | H | $CH_3$ | C≡CH | N | |
| J-7 | $CO_2CH_3$ | H | H | $OCH_3$ | $OCH_3$ | CH | 192-194 |
| J-7 | $CO_2CH_3$ | H | H | $CH_3$ | $OCH_3$ | CH | 155-162 |
| J-7 | $CO_2CH_3$ | H | H | $CH_3$ | $CH_3$ | CH | 153-156 |
| J-7 | $CO_2CH_3$ | H | H | $OCH_3$ | $OCH_3$ | N | 167-169 |
| J-7 | $CO_2CH_3$ | H | H | $CH_3$ | $OCH_3$ | N | 166-167 |
| J-7 | $CO_2CH_3$ | H | H | Cl | $OCH_3$ | CH | 185-186 |
| J-7 | $CO_2CH_3$ | H | $CH_3$ | $OCH_3$ | $OCH_3$ | N | 165-166 |
| J-7 | Cl | H | H | $OCH_3$ | $CH_2CH_2CH_3$ | CH | |
| J-7 | Cl | $CH_3$ | H | $OCH_3$ | $OCH_2CH_3$ | CH | |
| J-7 | $SO_2N(CH_3)_2$ | H | H | $OCH_3$ | $CF_3$ | CH | |
| J-7 | $SO_2N(CH_3)_2$ | H | H | $OCH_3$ | $SCH_3$ | CH | |
| J-7 | $SO_2CH_3$ | H | H | $OCH_3$ | $OCH_2CH{=}CH_2$ | CH | |
| J-7 | $SO_2CH_2CH_3$ | H | H | $OCHF_2$ | $CH_3$ | CH | |
| J-8 | H | - | H | $OCH_3$ | $CH_3$ | CH | |
| J-8 | $CH_3$ | - | H | $OCH_3$ | $CH_3$ | N | |
| J-8 | $CO_2CH_3$ | - | H | $OCH_3$ | $CH_3$ | CH | |
| J-8 | $CO_2CH_3$ | - | H | $OCH_3$ | $CH_3$ | N | |
| J-8 | Cl | - | H | $OCH_3$ | $OCH_2CH_2F$ | CH | |
| J-8 | $SO_2N(CH_3)_2$ | - | H | $OCH_3$ | $OCH_2CH_3$ | N | |

Table VII (Continued)

| J | $R_5$ | $R_{19}$ | R | X | Y | Z |
|---|---|---|---|---|---|---|
| J-8 | $SO_2CH_3$ | - | H | $OCH_3$ | $CH_3$ | CH |
| J-8 | $NO_2$ | - | H | $OCH_3$ | $NHCH_3$ | CH |
| J-9 | H | Br | H | $OCF_2H$ | $CH_3$ | CH |
| J-9 | H | $CH_3$ | H | $CH_3$ | $OCH_3$ | N |
| J-9 | $CH_3$ | $NO_2$ | H | $OCH_3$ | $OCF_2H$ | CH |
| J-9 | Cl | $SCH_3$ | H | $CH_3$ | $OCH_3$ | N |
| J-9 | $CO_2CH_3$ | Cl | H | Cl | $OCH_3$ | CH |
| J-9 | $CO_2CH_3$ | $CH_3$ | H | $OCH_3$ | $OCH_3$ | N |
| J-9 | $CO_2CH_3$ | $SO_2CH_3$ | H | Br | $OCH_3$ | CH |
| J-9 | $SO_2N(CH_3)_2$ | H | H | $CH_3$ | $CH_3$ | N |
| J-9 | $SO_2CH_3$ | H | H | $OCH_3$ | $CH_2CH_3$ | CH |
| J-9 | $SO_2CH_2CH_3$ | H | H | $OCH_3$ | 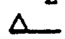 | N |
| J-10 | H | $SO_2CH_2CH_3$ | H | $OCH_3$ | $CH_3$ | CH |
| J-10 | H | $NO_2$ | H | $OCH_3$ | $CH_3$ | N |
| J-10 | Cl | Cl | H | $OCH_3$ | $OCH_3$ | N |
| J-10 | $CO_2CH_3$ | $SCH_3$ | H | $OCH_3$ | $OCH_3$ | CH |
| J-10 | $CO_2CH_3$ | H | H | $OCH_3$ | $OCH_3$ | N |
| J-10 | $SO_2N(CH_3)Et$ | H | H | $OCH_3$ | $OCH_3$ | N |
| J-10 | $SO_2CH_3$ | H | $CH_3$ | $OCH_3$ | $OCH_3$ | CH |
| J-10 | $SO_2CH_3$ | $CH_3$ | H | $OCH_3$ | $OCH_3$ | N |
| J-11 | Cl | - | H | $OCH_3$ | $OCH_3$ | CH |
| J-11 | Br | - | H | $OCH_3$ | $OCH_3$ | N |
| J-11 | $CH_3$ | - | H | $OCH_3$ | $OCH_2C{\equiv}CH$ | CH |
| J-11 | $CO_2CH_3$ | - | H | $OCH_3$ | $CH_3$ | N |
| J-11 | $CO_2CH_3$ | - | H | $OCH_3$ | $CF_3$ | CH |
| J-11 | $CO_2CH_2CH_3$ | - | H | $OCH_3$ | $OCH_3$ | N |
| J-11 | $SO_2N(CH_3)_2$ | - | H | $CH_3$ | $C{\equiv}CH$ | CH |
| J-11 | $SO_2CH_3$ | - | H | $CH_3$ | $N(CH_3)_2$ | N |
| J-11 | $SO_2CH_2CH_3$ | - | H | $CH_3$ | $CH_2F$ | CH |

Table VIII

General Formula 4

| J | $R_4$ | $R_5$ | $R_6$ | $R_{19}$ | $X_1$ | $Y_1$ |
|---|---|---|---|---|---|---|
| $J_2$ | H | $CH_3$ | $CH_3$ | - | $CH_3$ | O |
| $J_2$ | $CH_3$ | Cl | $CH_3$ | - | $OCH_3$ | $CH_2$ |
| $J_2$ | H | Br | $CH_2CH_2CH_3$ | - | $OCH_3$ | O |
| $J_2$ | $CH_3$ | $CO_2CH_3$ | $CH_2CH_2CH_3$ | - | $OCH_3$ | O |
| $J_2$ | $CH_3$ | $SO_2N(CH_3)_2$ | $CH_3$ | - | $CH_3$ | O |
| $J_2$ | $CH_3$ | H | $SO_2CH_3$ | - | $OCH_3$ | $CH_2$ |
| $J_2$ | $CH_3$ | $SO_2CH_2CH_3$ | $CH_3$ | - | $CH_3$ | O |
| $J_7$ | - | $CH_3$ | - | $SCH_3$ | $OCH_3$ | O |
| $J_7$ | - | Cl | - | $CH_3$ | $OCH_3$ | $CH_2$ |
| $J_7$ | - | $CO_2CH_3$ | - | $CH_3$ | $CH_3$ | O |
| $J_7$ | - | H | - | $SO_2CH_3$ | $OCH_3$ | O |
| $J_7$ | - | $SO_2CH_3$ | - | Cl | $OCH_3$ | O |
| $J_7$ | - | $SO_2N(CH_3)_2$ | - | H | $CH_3$ | O |
| $J_8$ | - | $CH_3$ | - | - | $OCH_3$ | $CH_2$ |
| $J_8$ | - | Cl | - | - | $CH_3$ | O |
| $J_8$ | - | $CO_2CH_3$ | - | - | $OCH_3$ | O |
| $J_8$ | - | $CO_2CH_2CH_3$ | - | - | $OCH_2CH_3$ | O |
| $J_8$ | - | $NO_2$ | - | - | $CH_3$ | O |
| $J_8$ | - | $SO_2CH_3$ | - | - | $OCF_2H$ | $CH_2$ |
| $J_8$ | - | $SO_2CH_3$ | - | - | $OCH_3$ | $CH_2$ |
| $J_9$ | - | Cl | - | $CH_3$ | $OCH_3$ | O |
| $J_9$ | - | $CO_2CH_3$ | - | $CH_3$ | $OCH_3$ | O |
| $J_9$ | - | $SO_2CH_2CH_3$ | - | $CH_3$ | $CH_3$ | O |
| $J_{10}$ | - | $SO_2CH_3$ | - | $CH_3$ | $CH_3$ | O |
| $J_{10}$ | - | Cl | - | $CH_3$ | $OCH_3$ | $CH_2$ |
| $J_{10}$ | - | $CO_2CH_3$ | - | $SCH_3$ | $OCH_3$ | O |
| $J_{10}$ | - | $SO_2CH_3$ | - | Cl | $CH_3$ | $CH_2$ |
| $J_{11}$ | - | Cl | - | - | $OCH_3$ | O |
| $J_{11}$ | - | $CO_2CH_3$ | - | - | $OCH_3$ | O |
| $J_{11}$ | - | $SO_2N(CH_3)_2$ | - | - | $OCH_3$ | $CH_2$ |
| $J_{11}$ | - | $SO_2CH_3$ | - | - | $CH_3$ | O |

Table IX

General Formula 4

| J | $R_1$ | $R_2$ | $R_3$ | $R_7$ | $R_8$ | $X_1$ | $Y_1$ |
|---|---|---|---|---|---|---|---|
| $J_1$ | $CH_3$ | H | H | - | - | $CH_3$ | O |
| $J_1$ | $CH_3$ | $CH_3$ | $CH_3$ | - | - | $OCH_3$ | $CH_2$ |
| $J_1$ | $CO_2CH_3$ | $CH_3$ | $CH_3$ | - | - | $CH_3$ | $CH_2$ |
| $J_1$ | $CO_2CH_3$ | H | H | - | - | $OCH_3$ | O |
| $J_1$ | $CO_2CH_3$ | H | H | - | - | $OCF_2H$ | $CH_2$ |
| $J_1$ | $CO_2CH_3$ | $CH_3$ | H | - | - | $OCH_3$ | O |
| $J_1$ | $CO_2CH_2CH_3$ | H | H | - | - | $CH_3$ | O |
| $J_1$ | $CO_2CH_2CH_3$ | $CH_3$ | H | - | - | $CH_3$ | O |
| $J_1$ | $SO_2N(CH_3)_2$ | H | H | - | - | $CH_3$ | O |
| $J_1$ | $SO_2N(CH_3)_2$ | $CH_3$ | $CH_3$ | - | - | $OCH_2CH_3$ | $CH_2$ |
| $J_1$ | $SO_2N(CH_2CH_3)_2$ | H | H | - | - | $OCH_3$ | O |
| $J_1$ | $SO_2CH_3$ | H | H | - | - | $OCH_3$ | $CH_2$ |
| $J_1$ | $SO_2CH_3$ | H | $CH_3$ | - | - | $OCH_3$ | O |
| $J_1$ | $SO_2CH_3$ | $CH_3$ | $CH_3$ | - | - | $CH_3$ | O |
| $J_1$ | $SO_2CH_2CH_3$ | $CH_3$ | $CH_3$ | - | - | $CH_3$ | $CH_2$ |
| $J_3$ | - | - | - | H | $CO_2CH_3$ | $CH_3$ | O |
| $J_3$ | - | - | - | H | $CO_2CH_2CH_3$ | $CH_3$ | O |
| $J_3$ | - | - | - | $CH_3$ | $SO_2N(CH_3)_2$ | $OCH_3$ | $CH_2$ |
| $J_3$ | - | - | - | $CH_3$ | $SO_2N(OCH_3)CH_3$ | $OCH_3$ | $CH_2$ |
| $J_3$ | - | - | - | H | $SO_2CH_3$ | $OCH_3$ | O |
| $J_3$ | - | - | - | H | $SO_2CH_3$ | $OCH_3$ | $CH_2$ |
| $J_3$ | - | - | - | $CH_3$ | $SO_2CH_2CH_3$ | $CH_3$ | O |
| $J_3$ | - | - | - | $CH_3$ | $NO_2$ | $CH_3$ | O |

Table X

General Formula 4

| J | $R_9$ | $R_{10}$ | $R_{11}$ | $R_{12}$ | $R_{13}$ | $R_{14}$ | $X_1$ | $Y_1$ |
|---|---|---|---|---|---|---|---|---|
| $J_4$ | H | $CO_2CH_3$ | H | - | - | - | $OCH_3$ | $CH_2$ |
| $J_4$ | $CH_3$ | $CO_2CH_3$ | $CH_3$ | - | - | - | $CH_3$ | O |
| $J_4$ | $CH_3$ | $CO_2CH_3$ | $CH_3$ | - | - | - | $CH_3$ | O |
| $J_4$ | $CH_3$ | $CO_2CH_2CH_3$ | H | - | - | - | $OCH_3$ | O |
| $J_4$ | $CH_3$ | $SO_2N(CH_3)_2$ | $CH_3$ | - | - | - | $OCH_3$ | O |
| $J_4$ | $CH_3$ | $SO_2N(OCH_3)CH_3$ | $CH_3$ | - | - | - | $OCH_2CH_3$ | $CH_2$ |
| $J_4$ | $CH_3$ | $SO_2CH_3$ | $CH_3$ | - | - | - | $CH_3$ | O |
| $J_4$ | $CH_3$ | $SO_2CH_3$ | $CH_3$ | - | - | - | $OCF_2H$ | O |
| $J_4$ | $CH_2CH_2CH_3$ | $CO_2CH_3$ | $CH_3$ | - | - | - | $OCH_3$ | $CH_2$ |
| $J_4$ | $CH_3$ | $NO_2$ | $CH_3$ | - | - | - | $OCH_3$ | O |
| $J_4$ | $CH_3$ | $SO_2CH_2CH_3$ | H | - | - | - | $CH_3$ | O |
| $J_4$ | Ph | $CO_2CH_3$ | H | - | - | - | $OCH_3$ | $CH_2$ |
| $J_5$ | - | $CO_2CH_3$ | H | - | - | - | $OCH_3$ | O |
| $J_5$ | - | $CO_2CH_3$ | $CH_3$ | - | - | - | $OCH_3$ | O |
| $J_5$ | - | $CO_2H_3$ | H | - | - | - | $CH_3$ | O |
| $J_5$ | - | $SO_2N(CH_3)_2$ | H | - | - | - | $OCH_3$ | $CH_2$ |
| $J_5$ | - | $SO_2N(CH_3)Et$ | H | - | - | - | $OCH_3$ | O |
| $J_5$ | - | $SO_2CH_3$ | $CH_3$ | - | - | - | $CH_3$ | O |
| $J_5$ | - | $SO_2CH_3$ | $CH_3$ | - | - | - | $OCH_3$ | O |
| $J_5$ | - | $SO_2CH_2CH_3$ | $CH_3$ | - | - | - | $OCH_3$ | $CH_2$ |
| $J_5$ | - | $NO_2$ | H | - | - | - | $OCH_3$ | O |
| $J_6$ | - | - | - | H | $CO_2CH_3$ | H | $CH_3$ | O |
| $J_6$ | - | - | - | H | $CO_2CH_3$ | $CH_3$ | $OCH_3$ | $CH_2$ |
| $J_6$ | - | - | - | $CH_3$ | H | $CH_3$ | $CH_3$ | O |
| $J_6$ | - | - | - | H | $SO_2N(CH_3)_2$ | $CH_3$ | $OCH_3$ | O |
| $J_6$ | - | - | - | $NO_2$ | H | $CH_3$ | $OCH_3$ | O |
| $J_6$ | - | - | - | H | $SO_2CH_3$ | $CH_3$ | $OCH_3$ | $CH_2$ |
| $J_6$ | - | - | - | $CO_2CH_3$ | H | H | $CH_3$ | O |
| $J_6$ | - | - | - | $CO_2CH_3$ | H | $CH_3$ | $CH_3$ | O |
| $J_6$ | - | - | - | $SO_2CH_3$ | H | $CH_3$ | $OCH_3$ | $CH_2$ |

Table XI

General Formula 5

| J | $R_1$ | $R_2$ | $R_3$ | $R_7$ | $R_8$ | $X_1$ |
|---|---|---|---|---|---|---|
| $J_1$ | $CH_3$ | H | H | - | - | $CH_3$ |
| $J_1$ | $CH_3$ | $CH_3$ | $CH_3$ | - | - | $OCH_3$ |
| $J_1$ | $CO_2CH_3$ | $CH_3$ | $CH_3$ | - | - | $CH_3$ |
| $J_1$ | $CO_2CH_3$ | H | H | - | - | $OCH_3$ |
| $J_1$ | $CO_2CH_3$ | H | H | - | - | $OCF_2H$ |
| $J_1$ | $CO_2CH_3$ | $CH_3$ | H | - | - | $OCH_3$ |
| $J_1$ | $CO_2CH_2CH_3$ | H | H | - | - | $CH_3$ |
| $J_1$ | $CO_2CH_2CH_3$ | $CH_3$ | H | - | - | $CH_3$ |
| $J_1$ | $SO_2N(CH_3)_2$ | H | H | - | - | $CH_3$ |
| $J_1$ | $SO_2N(CH_3)_2$ | H | $CH_3$ | - | - | $OCH_3$ |
| $J_1$ | $SO_2N(CH_3)_2$ | $CH_3$ | $CH_3$ | - | - | $OCH_2CH_3$ |
| $J_1$ | $SO_2N(CH_2CH_3)_2$ | H | H | - | - | $OCH_3$ |
| $J_1$ | $SO_2CH_3$ | H | H | - | - | $OCH_3$ |
| $J_1$ | $SO_2CH_3$ | H | $CH_3$ | - | - | $OCH_3$ |
| $J_1$ | $SO_2CH_3$ | $CH_3$ | $CH_3$ | - | - | $CH_3$ |
| $J_1$ | $SO_2CH_2CH_3$ | $CH_3$ | $CH_3$ | - | - | $CH_3$ |
| $J_3$ | - | - | - | H | $CO_2CH_3$ | $CH_3$ |
| $J_3$ | - | - | - | H | $CO_2CH_2CH_3$ | $CH_3$ |
| $J_3$ | - | - | - | H | $SO_2N(CH_3)_2$ | $OCH_3$ |
| $J_3$ | - | - | - | $CH_3$ | $SO_2N(OCH_3)CH_3$ | $OCH_3$ |
| $J_3$ | - | - | - | H | $SO_2CH_3$ | $OCH_3$ |
| $J_3$ | - | - | - | H | $SO_2CH_3$ | $OCH_2CH_3$ |
| $J_3$ | - | - | - | $CH_3$ | $SO_2CH_3$ | $CH_3$ |
| $J_3$ | - | - | - | $CH_3$ | $NO_2$ | $CH_3$ |

Table XII

General Formula 5

| J | $R_4$ | $R_5$ | $R_6$ | $R_{19}$ | $X_1$ |
|---|---|---|---|---|---|
| $J_2$ | H | $CH_3$ | $CH_3$ | - | $CH_3$ |
| $J_2$ | $CH_3$ | Cl | $CH_3$ | - | $OCH_3$ |
| $J_2$ | H | Br | $CH_2CH_2CH_3$ | - | $OCF_2H$ |
| $J_2$ | $CH_3$ | $CO_2CH_3$ | $CH_3$ | - | $OCH_3$ |
| $J_2$ | $CH_3$ | $SO_2N(CH_3)_2$ | $CH_3$ | - | $CH_3$ |
| $J_2$ | $CH_3$ | $SO_2CH_3$ | $SO_2CH_3$ | - | $OCH_3$ |
| $J_2$ | $CH_3$ | $SO_2CH_2CH_3$ | $CH_3$ | - | $CH_3$ |
| $J_7$ | - | $CH_3$ | - | $SCH_3$ | $OCH_3$ |
| $J_7$ | - | Cl | - | $CH_3$ | $OCH_3$ |
| $J_7$ | - | $CO_2CH_3$ | - | $CH_3$ | $CH_3$ |
| $J_7$ | - | H | - | $SO_2CH_3$ | $OCH_3$ |
| $J_7$ | - | $SO_2CH_3$ | - | Cl | $OCH_3$ |
| $J_7$ | - | $SO_2N(CH_3)_2$ | - | $SCH_3$ | $CH_3$ |
| $J_8$ | - | $CH_3$ | - | - | $OCH_3$ |
| $J_8$ | - | Cl | - | - | $CH_3$ |
| $J_8$ | - | $CO_2CH_3$ | - | - | $OCH_3$ |
| $J_8$ | - | $CO_2CH_2CH_3$ | - | - | $OCH_2CH_3$ |
| $J_8$ | - | $NO_2$ | - | - | $CH_3$ |
| $J_8$ | - | $SO_2CH_3$ | - | - | $OCH_3$ |
| $J_9$ | - | Cl | - | $CH_3$ | $OCH_3$ |
| $J_9$ | - | $CO_2CH_3$ | - | H | $OCH_3$ |
| $J_9$ | - | $SO_2CH_3$ | - | $CH_3$ | $CH_3$ |
| $J_{10}$ | - | Cl | - | $CH_3$ | $OCH_3$ |
| $J_{10}$ | - | $CO_2CH_3$ | - | $SCH_3$ | $OCH_3$ |
| $J_{10}$ | - | $SO_2CH_3$ | - | Cl | $CH_3$ |
| $J_{11}$ | - | Cl | - | - | $OCH_3$ |
| $J_{11}$ | - | $CO_2CH_3$ | - | - | $OCH_3$ |
| $J_{11}$ | - | $SO_2N(CH_3)_2$ | - | - | $OCH_3$ |
| $J_{11}$ | - | $SO_2CH_3$ | - | - | $CH_3$ |

Table XIII

General Formula 5

| J | $R_9$ | $R_{10}$ | $R_{11}$ | $R_{12}$ | $R_{13}$ | $R_{14}$ | $X_1$ |
|---|---|---|---|---|---|---|---|
| $J_4$ | H | $CO_2CH_3$ | H | - | | | $OCH_3$ |
| $J_4$ | $CH_3$ | $CO_2CH_3$ | $CH_3$ | - | - | - | $CH_3$ |
| $J_4$ | $CH_3$ | $CO_2CH_2CH_3$ | H | - | - | - | $OCH_3$ |
| $J_4$ | $CH_3$ | $SO_2N(CH_3)_2$ | $CH_3$ | - | - | - | $OCH_2CH_3$ |
| $J_4$ | $CH_3$ | $SO_2CH_3$ | $CH_3$ | - | - | - | $CH_3$ |
| $J_4$ | $CH_3$ | $SO_2CH_3$ | $CH_3$ | - | - | - | $OCF_2H$ |
| $J_4$ | $CH_2CH_2CH_2$ | $CO_2CH_3$ | $CH_3$ | - | - | - | $OCH_3$ |
| $J_4$ | $CH_3$ | $NO_2$ | $CH_3$ | - | - | - | $OCH_3$ |
| $J_4$ | $CH_3$ | $SO_2CH_2CH_3$ | H | - | - | - | $CH_3$ |
| $J_4$ | Ph | $CO_2CH_3$ | H | - | - | - | $OCH_3$ |
| $J_5$ | - | $CO_2CH_3$ | $CH_3$ | - | - | - | $OCH_3$ |
| $J_5$ | - | $CO_2CH_3$ | H | - | - | - | $OCH_3$ |
| $J_5$ | - | $CO_2H_3$ | H | - | - | - | $CH_3$ |
| $J_5$ | - | $SO_2N(CH_3)_2$ | H | - | - | - | $OCH_3$ |
| $J_5$ | - | $SO_2N(CH_3)Et$ | H | - | - | - | $OCH_3$ |
| $J_5$ | - | $SO_2CH_3$ | $CH_3$ | - | - | - | $CH_3$ |
| $J_5$ | - | $SO_2CH_3$ | $CH_3$ | - | - | - | $OCH_3$ |
| $J_5$ | - | $SO_2CH_2CH_3$ | $CH_3$ | - | - | - | $OCH_3$ |
| $J_5$ | - | $NO_2$ | H | - | - | - | $OCH_3$ |
| $J_6$ | - | - | - | H | $CO_2CH_3$ | H | $CH_3$ |
| $J_6$ | - | - | - | H | $CO_2CH_3$ | $CH_3$ | $OCH_3$ |
| $J_6$ | - | - | - | $CH_3$ | H | $CH_3$ | $CH_3$ |
| $J_6$ | - | - | - | H | $SO_2N(CH_3)_2$ | $CH_3$ | $OCH_3$ |
| $J_6$ | - | - | - | $NO_2$ | H | $CH_3$ | $OCH_3$ |
| $J_6$ | - | - | - | H | $SO_2CH_3$ | $CH_3$ | $OCH_3$ |
| $J_6$ | - | - | - | $CO_2CH_3$ | H | $CH_3$ | $CH_3$ |
| $J_6$ | - | - | - | $CO_2CH_3$ | H | $CH_3$ | $CH_3$ |
| $J_6$ | - | - | - | $SO_2CH_3$ | H | $CH_3$ | $OCH_3$ |

Table XIV

General Formula 6

| J | $R_1$ | $R_2$ | $R_3$ | $R_7$ | $R_8$ | $X_1$ | $Y_3$ |
|---|---|---|---|---|---|---|---|
| $J_1$ | $CH_3$ | H | H | - | - | $CH_3$ | H |
| $J_1$ | $CH_3$ | $CH_3$ | $CH_3$ | - | - | $OCH_3$ | H |
| $J_1$ | $CO_2CH_3$ | $CH_3$ | $CH_3$ | - | - | $CH_3$ | H |
| $J_1$ | $CO_2CH_3$ | H | H | - | - | $OCH_3$ | H |
| $J_1$ | $CO_2CH_3$ | H | H | - | - | $OCF_2H$ | $CH_3$ |
| $J_1$ | $CO_2CH_3$ | $CH_3$ | H | - | - | $OCH_3$ | $CH_3$ |
| $J_1$ | $CO_2CH_2CH_3$ | H | H | - | - | $CH_3$ | $CH_3$ |
| $J_1$ | $CO_2CH_2CH_3$ | $CH_3$ | H | - | - | $CH_3$ | H |
| $J_1$ | $SO_2N(CH_3)_2$ | H | H | - | - | $CH_3$ | H |
| $J_1$ | $SO_2N(CH_3)_2$ | H | $CH_3$ | - | - | $OCH_3$ | H |
| $J_1$ | $SO_2N(CH_3)_2$ | $CH_3$ | $CH_3$ | - | - | $OCH_2CH_3$ | $CH_3$ |
| $J_1$ | $SO_2N(CH_2CH_3)_2$ | H | H | - | - | $OCH_3$ | $CH_3$ |
| $J_1$ | $SO_2CH_3$ | H | H | - | - | $OCH_3$ | $CH_3$ |
| $J_1$ | $SO_2CH_3$ | H | $CH_3$ | - | - | $OCH_3$ | H |
| $J_1$ | $SO_2CH_3$ | $CH_3$ | $CH_3$ | - | - | $CH_3$ | H |
| $J_1$ | $SO_2CH_2CH_3$ | $CH_3$ | $CH_3$ | - | - | $CH_3$ | H |
| $J_3$ | - | - | - | H | $CO_2CH_3$ | $CH_3$ | $CH_3$ |
| $J_3$ | - | - | - | H | $CO_2CH_2CH_3$ | $CH_3$ | $CH_3$ |
| $J_3$ | - | - | - | $CH_3$ | $SO_2N(CH_3)_2$ | $OCH_3$ | $CH_3$ |
| $J_3$ | - | - | - | $CH_3$ | $SO_2N(OCH_3)CH_3$ | $OCH_3$ | H |
| $J_3$ | - | - | - | H | $SO_2CH_3$ | $OCH_3$ | H |
| $J_3$ | - | - | - | $CH_3$ | $SO_2CH_3$ | $OCH_3$ | H |
| $J_3$ | - | - | - | $CH_3$ | $SO_2CH_3$ | $CH_3$ | $CH_3$ |
| $J_3$ | - | - | - | $CH_3$ | $NO_2$ | $CH_3$ | $CH_3$ |

Table XV

General Formula 6

| J | $R_4$ | $R_5$ | $R_6$ | $R_{19}$ | $X_1$ | $Y_3$ |
|---|---|---|---|---|---|---|
| $J_2$ | H | $CH_3$ | $CH_3$ | - | $CH_3$ | H |
| $J_2$ | $CH_3$ | Cl | $CH_3$ | - | $OCH_3$ | H |
| $J_2$ | H | Br | $CH_2CH_2CH_3$ | - | $OCH_3$ | H |
| $J_2$ | $CH_3$ | $CO_2CH_3$ | $CH_3$ | - | $OCH_3$ | $CH_3$ |
| $J_2$ | $CH_3$ | $SO_2N(CH_3)_2$ | $CH_3$ | - | $CH_3$ | $CH_3$ |
| $J_2$ | $CH_3$ | H | $SO_2CH_3$ | - | $OCH_3$ | $CH_3$ |
| $J_2$ | $CH_3$ | $SO_2CH_2CH_3$ | $CH_3$ | - | $CH_3$ | H |
| $J_7$ | - | $CH_3$ | - | $SCH_3$ | $OCH_3$ | $CH_3$ |
| $J_7$ | - | Cl | - | $CH_3$ | $OCH_3$ | $CH_3$ |
| $J_7$ | - | $CO_2CH_3$ | - | $CH_3$ | $CH_3$ | $CH_3$ |
| $J_7$ | - | H | - | $SO_2CH_3$ | $OCH_3$ | $CH_3$ |
| $J_7$ | - | $SO_2CH_3$ | - | Cl | $OCH_3$ | $CH_3$ |
| $J_7$ | - | $SO_2N(CH_3)_2$ | - | $SCH_3$ | $CH_3$ | H |
| $J_8$ | - | $CH_3$ | - | - | $OCH_3$ | H |
| $J_8$ | - | Cl | - | - | $CH_3$ | H |
| $J_8$ | - | $CO_2CH_3$ | - | - | $OCH_3$ | H |
| $J_8$ | - | $CO_2CH_2CH_3$ | - | - | $OCH_2CH_3$ | $CH_3$ |
| $J_8$ | - | $NO_2$ | - | - | $CH_3$ | $CH_3$ |
| $J_8$ | - | $SO_2CH_3$ | - | - | $OCH_3$ | $CH_3$ |
| $J_9$ | - | Cl | - | $CH_3$ | $OCH_3$ | H |
| $J_9$ | - | $CO_2CH_3$ | - | $CH_3$ | $OCH_3$ | H |
| $J_9$ | - | $SO_2CH_3$ | - | $CH_3$ | $CH_3$ | $CH_3$ |
| $J_{10}$ | - | Cl | - | $CH_3$ | $OCH_3$ | H |
| $J_{10}$ | - | $CO_2CH_3$ | - | $SCH_3$ | $OCH_3$ | $CH_3$ |
| $J_{10}$ | - | $SO_2CH_3$ | - | Cl | $CH_3$ | $CH_3$ |
| $J_{11}$ | - | Cl | - | - | $OCF_2H$ | $CH_3$ |
| $J_{11}$ | - | $CO_2CH_3$ | - | - | $OCH_3$ | H |
| $J_{11}$ | - | $SO_2N(CH_3)_2$ | - | - | $OCH_3$ | H |
| $J_{11}$ | - | $SO_2CH_3$ | - | - | $CH_3$ | H |

Table XVI

General Formula 6

| J | $R_9$ | $R_{10}$ | $R_{11}$ | $R_{12}$ | $R_{13}$ | $R_{14}$ | $X_1$ | $Y_3$ |
|---|---|---|---|---|---|---|---|---|
| $J_4$ | H | $CO_2CH_3$ | H | - | - | - | $OCH_3$ | H |
| $J_4$ | $CH_3$ | $CO_2CH_3$ | $CH_3$ | - | - | - | $CH_3$ | H |
| $J_4$ | $CH_3$ | $CO_2CH_2CH_3$ | H | - | - | - | $OCH_3$ | H |
| $J_4$ | $CH_3$ | $SO_2N(CH_3)_2$ | $CH_3$ | - | - | - | $OCH_3$ | H |
| $J_4$ | $CH_3$ | $SO_2N(OCH_3)CH_3$ | $CH_3$ | - | - | - | $OCH_2CH_3$ | $CH_3$ |
| $J_4$ | $CH_3$ | $SO_2CH_3$ | $CH_3$ | - | - | - | $OCF_2H$ | $CH_3$ |
| $J_4$ | $CH_2CH_2CH_3$ | $CO_2CH_3$ | $CH_3$ | - | - | - | $OCH_3$ | $CH_3$ |
| $J_4$ | $CH_3$ | $NO_2$ | $CH_3$ | - | - | - | $OCH_3$ | $CH_3$ |
| $J_4$ | $CH_3$ | $SO_2CH_2CH_3$ | H | - | - | - | $CH_3$ | $CH_3$ |
| $J_4$ | Ph | $CO_2CH_3$ | H | - | - | - | $OCH_3$ | $CH_3$ |
| $J_5$ | - | $CO_2CH_3$ | H | - | - | - | $OCH_2CH_3$ | H |
| $J_5$ | - | $CO_2CH_3$ | H | - | - | - | $OCH_3$ | H |
| $J_5$ | - | $CO_2CH_3$ | H | - | - | - | $CH_3$ | H |
| $J_5$ | - | $SO_2N(CH_3)_2$ | H | - | - | - | $OCH_3$ | H |
| $J_5$ | - | $SO_2N(CH_3)Et$ | H | - | - | - | $OCH_3$ | H |
| $J_5$ | - | $SO_2CH_3$ | $CH_3$ | - | - | - | $CH_3$ | H |
| $J_5$ | - | $SO_2CH_3$ | $CH_3$ | - | - | - | $OCH_3$ | H |
| $J_5$ | - | $SO_2CH_2CH_3$ | $CH_3$ | - | - | - | $OCH_3$ | $CH_3$ |
| $J_5$ | - | $NO_2$ | H | - | - | - | $OCH_3$ | $CH_3$ |
| $J_6$ | - | - | - | H | $CO_2CH_3$ | H | $CH_3$ | $CH_3$ |
| $J_6$ | - | - | - | H | $CO_2CH_3$ | $CH_3$ | $OCH_3$ | $CH_3$ |
| $J_6$ | - | - | - | $CH_3$ | H | $CH_3$ | $CH_3$ | $CH_3$ |
| $J_6$ | - | - | - | H | $SO_2N(CH_3)_2$ | $CH_3$ | $OCH_3$ | $CH_3$ |
| $J_6$ | - | - | - | $NO_2$ | H | $CH_3$ | $OCH_3$ | H |
| $J_6$ | - | - | - | H | $SO_2CH_3$ | $CH_3$ | $OCH_3$ | H |
| $J_6$ | - | - | - | $CO_2CH_3$ | H | H | $CH_3$ | H |
| $J_6$ | - | - | - | $CO_2CH_3$ | H | $CH_3$ | $CH_3$ | H |
| $J_6$ | - | - | - | $SO_2CH_3$ | $CH_3$ | $CH_3$ | $OCH_3$ | H |

Table XVII

General Formula 7

| J | $R_4$ | $R_5$ | $R_6$ | $R_{19}$ | $X_2$ | $Y_2$ |
|---|---|---|---|---|---|---|
| $J_2$ | H | $CH_3$ | $CH_3$ | - | $CH_3$ | $OCH_3$ |
| $J_2$ | $CH_3$ | Cl | $CH_3$ | - | $CH_3$ | $OCH_3$ |
| $J_2$ | H | Br | $CH_2CH_2CH_3$ | - | $CH_3$ | $OCH_3$ |
| $J_2$ | $CH_3$ | $CO_2CH_3$ | $CH_3$ | - | $CH_3$ | $OCH_3$ |
| $J_2$ | $CH_3$ | $SO_2N(CH_3)_2$ | $CH_3$ | - | $CH_3$ | $OCH_3$ |
| $J_2$ | $CH_3$ | H | $SO_2CH_3$ | - | $CH_3$ | $CH_3$ |
| $J_2$ | $CH_3$ | $SO_2CH_2CH_3$ | $CH_3$ | - | $C_2H_5$ | $OCH_2CH_3$ |
| $J_7$ | - | $CH_3$ | - | $SCH_3$ | $CH_2CF_3$ | $OCH_3$ |
| $J_7$ | - | Cl | - | $CH_3$ | $CH_3$ | $OCH_3$ |
| $J_7$ | - | $CO_2CH_3$ | - | $CH_3$ | $CH_3$ | $OCH_3$ |
| $J_7$ | - | H | - | $SO_2CH_3$ | $CH_3$ | $OCH_3$ |
| $J_7$ | - | $SO_2CH_3$ | - | Cl | $CH_3$ | $SCH_3$ |
| $J_7$ | - | $SO_2N(CH_3)_2$ | - | $SCH_3$ | $C_2H_5$ | $OCH_3$ |
| $J_8$ | - | $CH_3$ | - | - | $CH_2CF_3$ | $OCH_3$ |
| $J_8$ | - | Cl | - | - | $CH_3$ | $OCH_3$ |
| $J_8$ | - | $CO_2CH_3$ | - | - | $CH_3$ | $SCF_2H$ |
| $J_8$ | - | $CO_2CH_2CH_3$ | - | - | $CH_3$ | $OCH_3$ |
| $J_8$ | - | $NO_2$ | - | - | $CH_3$ | $OCH_3$ |
| $J_8$ | - | $SO_2CH_3$ | - | - | $CH_2CH_3$ | $OCH_3$ |
| $J_9$ | - | Cl | - | $CH_3$ | $CH_3$ | $OCH_3$ |
| $J_9$ | - | $CO_2CH_3$ | - | $SO_2CH_3$ | $CH_3$ | $CH_3$ |
| $J_9$ | - | $SO_2CH_3$ | - | $CH_3$ | $CH_3$ | $OCH_3$ |
| $J_{10}$ | - | Cl | - | $CH_3$ | $CH_3$ | $SCH_2CH_3$ |
| $J_{10}$ | - | $CO_2CH_3$ | - | $SCH_3$ | $C_2H_5$ | $OCH_3$ |
| $J_{10}$ | - | $SO_2CH_3$ | - | Cl | $CH_2CF_3$ | $OCH_3$ |
| $J_{11}$ | - | Cl | - | - | $CH_3$ | $OCH_3$ |
| $J_{11}$ | - | $CO_2CH_3$ | - | - | $CH_3$ | $SC_2H_5$ |
| $J_{11}$ | - | $SO_2N(CH_3)_2$ | - | - | $CH_3$ | $OCH_3$ |
| $J_{11}$ | - | $SO_2CH_3$ | - | - | $CH_3$ | $OCH_3$ |

Table XVIII

General Formula 7

| J | $R_1$ | $R_2$ | $R_3$ | $R_7$ | $R_8$ | $X_2$ | $Y_2$ |
|---|---|---|---|---|---|---|---|
| $J_1$ | $CH_3$ | H | H | - | - | $CH_3$ | $OCH_3$ |
| $J_1$ | $CH_3$ | $CH_3$ | $CH_3$ | - | - | $CH_3$ | $OCH_3$ |
| $J_1$ | $CO_2CH_3$ | $CH_3$ | $CH_3$ | - | - | $CH_3$ | $OCH_3$ |
| $J_1$ | $CO_2CH_3$ | H | H | - | - | $CH_3$ | $OCH_3$ |
| $J_1$ | $CO_2CH_3$ | H | H | - | - | $CH_3$ | $OCH_3$ |
| $J_1$ | $CO_2CH_3$ | $CH_3$ | H | - | - | $C_2H_5$ | $OCH_3$ |
| $J_1$ | $CO_2CH_2CH_3$ | H | H | - | - | $CH_2CF_3$ | $OCH_3$ |
| $J_1$ | $CO_2CH_2CH_3$ | $CH_3$ | H | - | - | $CH_3$ | $OCH_3$ |
| $J_1$ | $SO_2N(CH_3)_2$ | H | H | - | - | $CH_3$ | $CH_3$ |
| $J_1$ | $SO_2N(CH_3)_2$ | H | $CH_3$ | - | - | $CH_3$ | OEt |
| $J_1$ | $SO_2N(CH_3)_2$ | $CH_3$ | $CH_3$ | - | - | $CH_3$ | $OCH_3$ |
| $J_1$ | $SO_2N(CH_2CH_3)_2$ | H | H | - | - | $CH_3$ | $OCH_3$ |
| $J_1$ | $SO_2CH_3$ | H | H | - | - | $CH_3$ | $OCH_3$ |
| $J_1$ | $SO_2CH_3$ | H | $CH_3$ | - | - | $CH_3$ | $SCH_3$ |
| $J_1$ | $SO_2CH_3$ | $CH_3$ | $CH_3$ | - | - | $CH_3$ | $CH_2CH_3$ |
| $J_1$ | $SO_2CH_2CH_3$ | $CH_3$ | $CH_3$ | - | - | $C_2H_5$ | $OCH_3$ |
| $J_3$ | - | - | - | H | $CO_2CH_3$ | $CH_2CF_3$ | $OCH_3$ |
| $J_3$ | - | - | - | H | $CO_2CH_2CH_3$ | $CH_3$ | $OCH_3$ |
| $J_3$ | - | - | - | $CH_3$ | $SO_2N(CH_3)_2$ | $CH_3$ | $SCF_2H$ |
| $J_3$ | - | - | - | $CH_3$ | $SO_2N(OCH_3)CH_3$ | $CH_3$ | $OCH_3$ |
| $J_3$ | - | - | - | H | $SO_2CH_3$ | $CH_3$ | $OCH_3$ |
| $J_3$ | - | - | - | H | $SO_2CH_3$ | $CH_3$ | $OCH_3$ |
| $J_3$ | - | - | - | $CH_3$ | $SO_2CH_2CH_3$ | $CH_3$ | $SCH_2CH_3$ |
| $J_3$ | - | - | - | $CH_3$ | $NO_2$ | $CH_3$ | $OCH_3$ |

Table XIX

General Formula 7

| J | $R_9$ | $R_{10}$ | $R_{11}$ | $R_{12}$ | $R_{13}$ | $R_{14}$ | $X_2$ | $Y_2$ |
|---|---|---|---|---|---|---|---|---|
| $J_4$ | H | $CO_2CH_3$ | H | - | - | - | $CH_3$ | $OCH_3$ |
| $J_4$ | $CH_3$ | $CO_2CH_3$ | $CH_3$ | - | - | - | $CH_3$ | $OCH_3$ |
| $J_4$ | $CH_3$ | $CO_2CH_2CH_3$ | H | - | - | - | $CH_3$ | $OCH_3$ |
| $J_4$ | $CH_3$ | $SO_2N(CH_3)_2$ | $CH_3$ | - | - | - | $CH_3$ | $OCH_3$ |
| $J_4$ | $CH_3$ | $SO_2N(OCH_3)CH_3$ | $CH_3$ | - | - | - | $CH_3$ | $OCH_3$ |
| $J_4$ | $CH_3$ | $SO_2CH_3$ | $CH_3$ | - | - | - | $C_2H_5$ | $OCH_3$ |
| $J_4$ | $CH_3$ | $SO_2CH_3$ | $CH_3$ | - | - | - | $CH_3$ | $OCH_3$ |
| $J_4$ | $CH_2CH_2CH_3$ | $CO_2CH_3$ | $CH_3$ | - | - | - | $CH_3$ | $CH_3$ |
| $J_4$ | $CH_3$ | $NO_2$ | $CH_3$ | - | - | - | $OCH_3$ | $OCH_2CH_3$ |
| $J_4$ | $CH_3$ | $SO_2CH_2CH_3$ | H | - | - | - | $CH_3$ | $OCH_3$ |
| $J_4$ | Ph | $CO_2CH_3$ | H | - | - | - | $CH_2CF_3$ | $CH_3$ |
| $J_5$ | - | $CO_2CH_3$ | H | - | - | - | $CH_3$ | $OCH_3$ |
| $J_5$ | - | $CO_2CH_3$ | H | - | - | - | $CH_3$ | $CH_2CH_3$ |
| $J_5$ | - | $CO_2CH_3$ | H | - | - | - | $CH_3$ | $OCH_3$ |
| $J_5$ | - | $SO_2N(CH_3)_2$ | H | - | - | - | $CH_3$ | $OCH_3$ |
| $J_5$ | - | $SO_2N(CH_3)Et$ | H | - | - | - | $CH_3$ | $SCHF_2$ |
| $J_5$ | - | $SO_2CH_3$ | $CH_3$ | - | - | - | $CH_3$ | $OCH_3$ |
| $J_5$ | - | $SO_2CH_3$ | $CH_3$ | - | - | - | $CH_2CH_3$ | $OCH_3$ |
| $J_5$ | - | $SO_2CH_2CH_3$ | $CH_3$ | - | - | - | $CH_3$ | $OCH_3$ |
| $J_5$ | - | $NO_2$ | H | - | - | - | $CH_3$ | $OCH_3$ |
| $J_6$ | - | - | - | H | $CO_2CH_3$ | H | $CH_3$ | $SCH_2CH_3$ |
| $J_6$ | - | - | - | H | $CO_2CH_3$ | $CH_3$ | $CH_3$ | $OCH_3$ |
| $J_6$ | - | - | - | $CH_3$ | $CO_2CH_3$ | $CH_3$ | $CH_3$ | $OCH_3$ |
| $J_6$ | - | - | - | H | $SO_2N(CH_3)_2$ | $CH_3$ | $CH_3$ | $OCH_3$ |
| $J_6$ | - | - | - | $CH_3$ | $SO_2N(CH_3)_2$ | $CH_3$ | $CH_3$ | $OCH_3$ |
| $J_6$ | - | - | - | H | $SO_2CH_3$ | $CH_3$ | $CH_2CF_3$ | $OCH_3$ |
| $J_6$ | - | - | - | $CO_2CH_3$ | H | H | $CH_3$ | $OCH_3$ |
| $J_6$ | - | - | - | $CO_2CH_3$ | H | $CH_3$ | $CH_3$ | $OCH_3$ |
| $J_6$ | - | - | - | $SO_2CH_3$ | $CH_3$ | $CH_3$ | $CH_3$ | $OCH_3$ |

Table XX

General Formula 8

| J | $R_9$ | $R_{10}$ | $R_{11}$ | $R_{12}$ | $R_{13}$ | $R_{14}$ | $X_3$ |
|---|---|---|---|---|---|---|---|
| $J_4$ | H | $CO_2CH_3$ | H | - | - | - | $CH_3$ |
| $J_4$ | $CH_3$ | $CO_2CH_3$ | $CH_3$ | - | - | - | $OCH_3$ |
| $J_4$ | $CH_3$ | $CO_2CH_2CH_3$ | H | - | - | - | $CH_3$ |
| $J_4$ | $CH_3$ | $SO_2N(CH_3)_2$ | $CH_3$ | - | - | - | $OCH_3$ |
| $J_4$ | $CH_3$ | $SO_2N(OCH_3)CH_3$ | $CH_3$ | - | - | - | $CH_3$ |
| $J_4$ | $CH_3$ | $SO_2CH_3$ | $CH_3$ | - | - | - | $OCH_3$ |
| $J_4$ | $CH_3$ | $SO_2CH_3$ | $CH_3$ | - | - | - | $CH_3$ |
| $J_4$ | $CH_2CH_2CH_3$ | $CO_2CH_3$ | $CH_3$ | - | - | - | $OCH_3$ |
| $J_4$ | $CH_3$ | $NO_2$ | $CH_3$ | - | - | - | $CH_3$ |
| $J_4$ | $CH_3$ | $SO_2CH_2CH_3$ | H | - | - | - | $OCH_3$ |
| $J_4$ | Ph | $CO_2CH_3$ | H | - | - | - | $CH_3$ |
| $J_5$ | - | $CO_2CH_3$ | H | - | - | - | $OCH_3$ |
| $J_5$ | - | $CO_2CH_3$ | H | - | - | - | $CH_3$ |
| $J_5$ | - | $CO_2CH_3$ | $CH_3$ | - | - | - | $OCH_3$ |
| $J_5$ | - | $SO_2N(CH_3)_2$ | H | - | - | - | $CH_3$ |
| $J_5$ | - | $SO_2N(CH_3)Et$ | H | - | - | - | $OCH_3$ |
| $J_5$ | - | $SO_2CH_3$ | $CH_3$ | - | - | - | $CH_3$ |
| $J_5$ | - | $SO_2CH_3$ | $CH_3$ | - | - | - | $OCH_3$ |
| $J_5$ | - | $SO_2CH_2CH_3$ | $CH_3$ | - | - | - | $CH_3$ |
| $J_5$ | - | $NO_2$ | H | - | - | - | $OCH_3$ |
| $J_6$ | - | - | - | H | $CO_2CH_3$ | H | $CH_3$ |
| $J_6$ | - | - | - | H | $CO_2CH_3$ | $CH_3$ | $OCH_3$ |
| $J_6$ | - | - | - | $CH_3$ | H | $CH_3$ | $CH_3$ |
| $J_6$ | - | - | - | H | $SO_2N(CH_3)_2$ | $CH_3$ | $OCH_3$ |
| $J_6$ | - | - | - | $NO_2$ | H | $CH_3$ | $CH_3$ |
| $J_6$ | - | - | - | H | $SO_2CH_3$ | $CH_3$ | $OCH_3$ |
| $J_6$ | - | - | - | $CO_2CH_3$ | H | H | $CH_3$ |
| $J_6$ | - | - | - | $CO_2CH_3$ | H | $CH_3$ | $OCH_3$ |
| $J_6$ | - | - | - | $SO_2CH_3$ | $CH_3$ | $CH_3$ | $CH_3$ |

Table XXI

General Formula 8

| J | $R_1$ | $R_2$ | $R_3$ | $R_7$ | $R_8$ | $X_3$ |
|---|---|---|---|---|---|---|
| $J_1$ | $CH_3$ | H | H | - | - | $CH_3$ |
| $J_1$ | $CH_3$ | $CH_3$ | $CH_3$ | - | - | $OCH_3$ |
| $J_1$ | $CO_2CH_3$ | $CH_3$ | $CH_3$ | - | - | $CH_3$ |
| $J_1$ | $CO_2CH_3$ | H | H | - | - | $OCH_3$ |
| $J_1$ | $CO_2CH_3$ | H | H | - | - | $CH_3$ |
| $J_1$ | $CO_2CH_3$ | $CH_3$ | H | - | - | $OCH_3$ |
| $J_1$ | $CO_2CH_2CH_3$ | H | H | - | - | $CH_3$ |
| $J_1$ | $CO_2CH_2CH_3$ | $CH_3$ | H | - | - | $CH_3$ |
| $J_1$ | $SO_2N(CH_3)_2$ | H | H | - | - | $OCH_3$ |
| $J_1$ | $SO_2N(CH_3)_2$ | H | $CH_3$ | - | - | $CH_3$ |
| $J_1$ | $SO_2N(CH_3)_2$ | $CH_3$ | $CH_3$ | - | - | $OCH_3$ |
| $J_1$ | $SO_2N(CH_2CH_3)_2$ | H | H | - | - | $CH_3$ |
| $J_1$ | $SO_2CH_3$ | H | H | - | - | $OCH_3$ |
| $J_1$ | $SO_2CH_3$ | H | $CH_3$ | - | - | $CH_3$ |
| $J_1$ | $SO_2CH_3$ | $CH_3$ | $CH_3$ | - | - | $CH_3$ |
| $J_1$ | $SO_2CH_2CH_3$ | $CH_3$ | $CH_3$ | - | - | $OCH_3$ |
| $J_3$ | - | - | - | H | $CO_2CH_3$ | $CH_3$ |
| $J_3$ | - | - | - | H | $CO_2CH_2CH_3$ | $OCH_3$ |
| $J_3$ | - | - | - | $CH_3$ | $SO_2N(CH_3)_2$ | $CH_3$ |
| $J_3$ | - | - | - | $CH_3$ | $SO_2N(OCH_3)CH_3$ | $OCH_3$ |
| $J_3$ | - | - | - | H | $SO_2CH_3$ | $CH_3$ |
| $J_3$ | - | - | - | H | $SO_2CH_3$ | $OCH_3$ |
| $J_3$ | - | - | - | $CH_3$ | $SO_2CH_3$ | $CH_3$ |
| $J_3$ | - | - | - | $CH_3$ | $NO_2$ | $OCH_3$ |

Table XXII

General Formula 8

| J | $R_4$ | $R_5$ | $R_6$ | $R_{19}$ | $X_3$ |
|---|---|---|---|---|---|
| $J_2$ | H | $CH_3$ | $CH_3$ | - | $CH_3$ |
| $J_2$ | $CH_3$ | Cl | $CH_3$ | - | $OCH_3$ |
| $J_2$ | H | Br | $CH_2CH_2CH_3$ | - | $CH_3$ |
| $J_2$ | $CH_3$ | $CO_2CH_3$ | $CH_3$ | - | $OCH_3$ |
| $J_2$ | $CH_3$ | $SO_2N(CH_3)_2$ | $CH_3$ | - | $CH_3$ |
| $J_2$ | $CH_3$ | H | $SO_2CH_3$ | - | $OCH_3$ |
| $J_2$ | $CH_3$ | $SO_2CH_2CH_3$ | $CH_3$ | - | $CH_3$ |
| $J_7$ | - | $CH_3$ | - | $SCH_3$ | $OCH_3$ |
| $J_7$ | - | Cl | - | $CH_3$ | $CH_3$ |
| $J_7$ | - | $CO_2CH_3$ | - | $CH_3$ | $OCH_3$ |
| $J_7$ | - | H | - | $SO_2CH_3$ | $CH_3$ |
| $J_7$ | - | $SO_2CH_3$ | - | Cl | $OCH_3$ |
| $J_7$ | - | $SO_2N(CH_3)_2$ | - | $SCH_3$ | $CH_3$ |
| $J_8$ | - | $CH_3$ | - | - | $OCH_3$ |
| $J_8$ | - | Cl | - | - | $CH_3$ |
| $J_8$ | - | $CO_2CH_3$ | - | - | $OCH_3$ |
| $J_8$ | - | $CO_2CH_2CH_3$ | - | - | $CH_3$ |
| $J_8$ | - | $NO_2$ | - | - | $OCH_3$ |
| $J_8$ | - | $SO_2CH_3$ | - | - | $CH_3$ |
| $J_9$ | - | Cl | - | $CH_3$ | $OCH_3$ |
| $J_9$ | - | $CO_2CH_3$ | - | $CH_3$ | $CH_3$ |
| $J_9$ | - | $SO_2CH_3$ | - | $CH_3$ | $OCH_3$ |
| $J_{10}$ | - | Cl | - | $CH_3$ | $CH_3$ |
| $J_{10}$ | - | $CO_2CH_3$ | - | $SCH_3$ | $OCH_3$ |
| $J_{10}$ | - | $SO_2CH_3$ | - | Cl | $CH_3$ |
| $J_{11}$ | - | Cl | - | - | $OCH_3$ |
| $J_{11}$ | - | $CO_2CH_3$ | - | - | $CH_3$ |
| $J_{11}$ | - | $SO_2N(CH_3)_2$ | - | - | $OCH_3$ |
| $J_{11}$ | - | $SO_2CH_3$ | - | - | $CH_3$ |

Table XXIII

General Formula 2a

| J | L | $R_{28}$ | X | Y | m.p. (°C) |
|---|---|---|---|---|---|
| $J_{32}$ | $CH_2$ | F | $OCH_3$ | $OCH_3$ | |
| $J_{32}$ | $CH_2$ | Cl | Cl | $OCH_3$ | |
| $J_{32}$ | $CH_2$ | Br | $OCH_3$ | $CH_3$ | |
| $J_{32}$ | $CH_2$ | $OCH_3$ | $OCH_3$ | $OCH_3$ | |
| $J_{32}$ | $CH_2$ | $SCH_3$ | $OCH_3$ | $OCH_3$ | |
| $J_{32}$ | $CH_2$ | $SO_2CH_3$ | $OCH_3$ | $OCH_3$ | |
| $J_{32}$ | $CH_2$ | $CO_2CH_3$ | $OCH_3$ | $OCH_3$ | |
| $J_{32}$ | $CH_2$ | $SO_2N(CH_3)_2$ | $OCH_3$ | $OCH_3$ | |
| $J_{32}$ | $CH_2$ | $OSO_2CH_3$ | $OCH_3$ | $OCH_3$ | |
| $J_{32}$ | $CH_2$ | Cl | $OCH_3$ | $OCH_3$ | |
| $J_{32}$ | $CH_2$ | Cl | Br | $OCH_3$ | |
| $J_{32}$ | $CH_2$ | Cl | $OCH_3$ | $CH_2F$ | |
| $J_{32}$ | $CH_2$ | Cl | $CH_3$ | $OC_2H_5$ | |
| $J_{32}$ | $CH_2$ | Cl | $OCH_3$ | $C_2H_5$ | |
| $J_{32}$ | O | F | $OCH_3$ | $OCH_3$ | |
| $J_{32}$ | O | Cl | $OCH_3$ | $OCH_3$ | |
| $J_{32}$ | O | Br | Cl | $OCH_3$ | |
| $J_{32}$ | O | $OCH_3$ | $OCH_3$ | $OCH_3$ | |
| $J_{32}$ | O | $SCH_3$ | $OCH_3$ | $OCH_3$ | |
| $J_{32}$ | O | $SO_2CH_3$ | $OCH_3$ | $OCH_3$ | |
| $J_{32}$ | O | $CO_2CH_3$ | $OCH_3$ | $OCH_3$ | |
| $J_{32}$ | O | $SO_2N(CH_3)_2$ | $OCH_3$ | $OCH_3$ | |
| $J_{32}$ | O | $OSO_2CH_3$ | $OCH_3$ | $CH_3$ | |
| $J_{32}$ | $CH_2$ | $CO_2CH_3$ | $CH_3$ | $OCH_3$ | |
| $J_{32}$ | $CH_2$ | $CO_2CH_3$ | $CH_3$ | $CH_3$ | |
| $J_{32}$ | $CH_2$ | $CO_2CH_3$ | Cl | $OCH_3$ | |
| $J_{32}$ | $CH_2$ | $CO_2CH_3$ | Br | $OCH_3$ | |
| $J_{32}$ | $CH_2$ | $CO_2CH_3$ | $OCH_3$ | $C_2H_5$ | |
| $J_{32}$ | $CH_2$ | $CO_2CH_3$ | $CH_3$ | $OC_2H_5$ | |
| $J_{32}$ | $CH_2$ | $CO_2CH_3$ | $OCH_3$ | $CH_2F$ | |
| $J_{32}$ | $CH_2$ | $CO_2CH_3$ | $OCH_3$ | $CF_3$ | |

Table XXIII (continued)

| J | L | $R_{28}$ | X | Y | m.p. (°C) |
|---|---|---|---|---|---|
| $J_{32}$ | $CH_2$ | Cl | $OCH_3$ | $NHCH_3$ | |
| $J_{32}$ | $CH_2$ | Br | $OCH_3$ | $N(CH_3)_2$ | |
| $J_{32}$ | $CH_2$ | F | $OCH_3$ | $NHCH_3$ | |
| $J_{32}$ | $CH_2$ | $OCH_3$ | $OCH_3$ | $NHCH_3$ | |
| $J_{32}$ | $CH_2$ | $SO_2N(CH_3)_2$ | $OCH_3$ | $C\equiv CH$ | |
| $J_{32}$ | $CH_2$ | Cl | $OCH_3$ | $OCH_2CH_2F$ | |
| $J_{32}$ | O | Cl | Cl | $NHCH_3$ | |
| $J_{32}$ | O | Br | $OCH_3$ | $NHCH_3$ | |
| $J_{32}$ | O | $OCH_3$ | $OCH_3$ | $NHCH_3$ | |
| $J_{32}$ | O | $SO_2N(CH_3)_2$ | $OCH_3$ | $NHCH_3$ | |
| $J_{32}$ | O | $SO_2CH_3$ | $OCH_3$ | $NHCH_3$ | |
| $J_{32}$ | $CH_2$ | $CO_2CH_3$ | $OCH_3$ | $OCH_3$ | 148-151.5 |
| $J_{32}$ | $CH_2$ | $CO_2CH_3$ | $CH_3$ | $CH_3$ | 191-193.5 |
| $J_{32}$ | $CH_2$ | $CO_2CH_3$ | $OCH_3$ | $CH_3$ | |

Table XXIV

General Formula 3a

| J | L | $R_{28}$ | X | Y | m.p. (°C) |
|---|---|---|---|---|---|
| $J_{32}$ | $CH_2$ | F | $OCH_3$ | $CH_3$ | |
| $J_{32}$ | $CH_2$ | Cl | $OCH_3$ | $CH_3$ | |
| $J_{32}$ | $CH_2$ | Br | $OCH_3$ | $CH_3$ | |
| $J_{32}$ | $CH_2$ | $OCH_3$ | $OCH_3$ | $OCH_3$ | |
| $J_{32}$ | $CH_2$ | $SCH_3$ | $OCH_3$ | $CH_3$ | |
| $J_{32}$ | $CH_2$ | $SO_2CH_3$ | $OCH_3$ | $CH_3$ | |
| $J_{32}$ | $CH_2$ | $CO_2CH_3$ | $OCH_3$ | $OCH_3$ | 122-122.5 |
| $J_{32}$ | $CH_2$ | $SO_2N(CH_3)_2$ | $OCH_3$ | $CH_3$ | |
| $J_{32}$ | $CH_2$ | $OSO_2CH_3$ | $OCH_3$ | $CH_3$ | |
| $J_{32}$ | $CH_2$ | Cl | $OCH_3$ | $CF_3$ | |
| $J_{32}$ | $CH_2$ | Cl | $CH_3$ | $OC_2H_5$ | |
| $J_{32}$ | $CH_2$ | Cl | $OCH_3$ | $C_2H_5$ | |
| $J_{32}$ | O | F | $OCH_3$ | $CH_3$ | |
| $J_{32}$ | O | Cl | $OCH_3$ | $CH_3$ | |
| $J_{32}$ | O | Br | $OCH_3$ | $CH_3$ | |
| $J_{32}$ | O | $OCH_3$ | $OCH_3$ | $CH_3$ | |
| $J_{32}$ | O | $SCH_3$ | $OCH_3$ | $CH_3$ | |
| $J_{32}$ | O | $SO_2CH_3$ | $OCH_3$ | $CH_3$ | |

Table XXIV (continued)

| J | L | $R_{28}$ | X | Y | m.p. (°C) |
|---|---|---|---|---|---|
| $J_{32}$ | O | $CO_2CH_3$ | $OCH_3$ | $CH_3$ | |
| $J_{32}$ | O | $SO_2N(CH_3)_2$ | $OCH_3$ | $CH_3$ | |
| $J_{32}$ | O | $OSO_2CH_3$ | $OCH_3$ | $CH_3$ | |
| $J_{32}$ | $CH_2$ | $CO_2CH_3$ | $OCH_3$ | $CH_3$ | 187-190 |
| $J_{32}$ | $CH_2$ | $CO_2CH_3$ | $CH_3$ | $OC_2H_5$ | |
| $J_{32}$ | $CH_2$ | $CO_2CH_3$ | $OCH_3$ | $CF_3$ | |
| $J_{32}$ | $CH_2$ | $CO_2CH_3$ | $OCH_3$ | $C_2H_5$ | |
| $J_{32}$ | $CH_2$ | Cl | $OCH_3$ | $NHCH_3$ | |
| $J_{32}$ | $CH_2$ | Br | $OCH_3$ | $N(CH_3)_2$ | |
| $J_{32}$ | $CH_2$ | F | $OCH_3$ | $NHCH_3$ | |
| $J_{32}$ | $CH_2$ | $OCH_3$ | $OCH_3$ | $NHCH_3$ | |
| $J_{32}$ | $CH_2$ | $SO_2N(CH_3)_2$ | $OCH_3$ | $C\equiv CH$ | |
| $J_{32}$ | $CH_2$ | Cl | $OCH_3$ | $OCH_2CH_2F$ | |
| $J_{32}$ | O | Cl | $OCH_3$ | $NHCH_3$ | |
| $J_{32}$ | O | Br | $OCH_3$ | $NHCH_3$ | |
| $J_{32}$ | O | $OCH_3$ | $OCH_3$ | $NHCH_3$ | |
| $J_{32}$ | O | $SO_2N(CH_3)_2$ | $OCH_3$ | $NHCH_3$ | |
| $J_{32}$ | O | $SO_2CH_3$ | $OCH_3$ | $NHCH_3$ | |

Table XXV

General Formula 4

| J | L | $R_{28}$ | $R_{22}$ | $X_1$ | $Y_1$ | m.p. (°C) |
|---|---|---|---|---|---|---|
| $J_{32}$ | $CH_2$ | Cl | - | $OCH_3$ | O | |
| $J_{32}$ | $CH_2$ | F | - | $OCH_3$ | O | |
| $J_{32}$ | $CH_2$ | Br | - | $OCH_3$ | O | |
| $J_{32}$ | $CH_2$ | $SO_2CH_3$ | - | $OCH_3$ | O | |
| $J_{32}$ | $CH_2$ | $OSO_2CH_3$ | - | $OCH_3$ | $CH_2$ | |
| $J_{32}$ | $CH_2$ | $SO_2N(CH_3)_2$ | - | $OCH_3$ | $CH_2$ | |
| $J_{32}$ | $CH_2$ | $CO_2CH_3$ | - | $OCH_3$ | $CH_2$ | |
| $J_{32}$ | O | Cl | - | $OCH_3$ | O | |
| $J_{32}$ | O | F | - | $OCH_3$ | O | |
| $J_{32}$ | O | Br | - | $OCH_3$ | O | |
| $J_{32}$ | O | $SO_2CH_3$ | - | $OCH_3$ | O | |
| $J_{32}$ | O | $OSO_2CH_3$ | - | $OCH_3$ | O | |
| $J_{32}$ | O | $SO_2N(CH_3)_2$ | - | $OCH_3$ | O | |
| $J_{32}$ | O | $CO_2CH_3$ | - | $OCH_3$ | O | |

Table XXVI

General Formula 5

| J | L | $R_{28}$ | $R_{22}$ | $X_1$ | m.p. (°C) |
|---|---|---|---|---|---|
| $J_{32}$ | $CH_2$ | Cl | - | $OCH_3$ | |
| $J_{32}$ | $CH_2$ | F | - | $OCH_3$ | |
| $J_{32}$ | $CH_2$ | Br | - | $OCH_3$ | |
| $J_{32}$ | $CH_2$ | $SO_2CH_3$ | - | $OCH_3$ | |
| $J_{32}$ | $CH_2$ | $OSO_2CH_3$ | - | $OCH_3$ | |
| $J_{32}$ | $CH_2$ | $SO_2N(CH_3)_2$ | - | $OCH_3$ | |
| $J_{32}$ | $CH_2$ | $CO_2CH_3$ | - | $OCH_3$ | |
| $J_{32}$ | O | Cl | - | $OCH_3$ | |
| $J_{32}$ | O | F | - | $OCH_3$ | |
| $J_{32}$ | O | Br | - | $OCH_3$ | |
| $J_{32}$ | O | $SO_2CH_3$ | - | $OCH_3$ | |
| $J_{32}$ | O | $OSO_2CH_3$ | - | $OCH_3$ | |
| $J_{32}$ | O | $SO_2N(CH_3)_2$ | - | $OCH_3$ | |
| $J_{32}$ | O | $CO_2CH_3$ | - | $OCH_3$ | |

Table XXVII

General Formula 6

| J | L | $R_{28}$ | $R_{22}$ | $X_1$ | $Y_3$ | m.p. (°C) |
|---|---|---|---|---|---|---|
| $J_{32}$ | $CH_2$ | Cl | - | $CH_3$ | H | |
| $J_{32}$ | $CH_2$ | F | - | $OCH_3$ | H | |
| $J_{32}$ | $CH_2$ | Br | - | $OCH_3$ | H | |
| $J_{32}$ | $CH_2$ | $SO_2CH_3$ | - | $OCH_3$ | H | |
| $J_{32}$ | $CH_2$ | $OSO_2CH_3$ | - | $OCH_3$ | H | |
| $J_{32}$ | $CH_2$ | $SO_2N(CH_3)_2$ | - | $OCH_3$ | H | |
| $J_{32}$ | $CH_2$ | $CO_2CH_3$ | - | $OCH_3$ | H | |
| $J_{32}$ | O | Cl | - | $OCH_3$ | H | |
| $J_{32}$ | O | F | - | $OCH_3$ | H | |
| $J_{32}$ | O | Br | - | $OCH_3$ | H | |
| $J_{32}$ | O | $SO_2CH_3$ | - | $OCH_3$ | H | |
| $J_{32}$ | O | $OSO_2CH_3$ | - | $OCH_3$ | H | |
| $J_{32}$ | O | $SO_2N(CH_3)_2$ | - | $OCH_3$ | H | |
| $J_{32}$ | O | $CO_2CH_3$ | - | $OCH_3$ | H | |

Table XXVIII

General Formula 7

| J | L | $R_{28}$ | $R_{22}$ | $X_2$ | $Y_2$ | m.p. (°C) |
|---|---|---|---|---|---|---|
| $J_{32}$ | $CH_2$ | Cl | - | $CH_3$ | $SCH_3$ | |
| $J_{32}$ | $CH_2$ | F | - | $CH_3$ | $SCH_3$ | |
| $J_{32}$ | $CH_2$ | Br | - | $CH_3$ | $SCH_3$ | |
| $J_{32}$ | $CH_2$ | $SO_2CH_3$ | - | $CH_3$ | $SCH_3$ | |
| $J_{32}$ | $CH_2$ | $OSO_2CH_3$ | - | $CH_3$ | $SCH_3$ | |
| $J_{32}$ | $CH_2$ | $SO_2N(CH_3)_2$ | - | $CH_3$ | $SCH_3$ | |
| $J_{32}$ | $CH_2$ | $CO_2CH_3$ | - | $CH_3$ | $SCH_3$ | |
| $J_{32}$ | O | Cl | - | $CH_3$ | $SCH_3$ | |
| $J_{32}$ | O | F | - | $CH_3$ | $SCH_3$ | |
| $J_{32}$ | O | Br | - | $CH_3$ | $SCH_3$ | |
| $J_{32}$ | O | $SO_2CH_3$ | - | $CH_3$ | $SCH_3$ | |
| $J_{32}$ | O | $OSO_2CH_3$ | - | $CH_3$ | $SCH_3$ | |
| $J_{32}$ | O | $SO_2N(CH_3)_2$ | - | $CH_3$ | $SCH_3$ | |
| $J_{32}$ | O | $CO_2CH_3$ | - | $CH_3$ | $SCH_3$ | |

Table XXIX

General Formula 8

| J | L | $R_{28}$ | $X_3$ | m.p. (°C) |
|---|---|---|---|---|
| $J_{32}$ | $CH_2$ | Cl | $OCH_3$ | |
| $J_{32}$ | $CH_2$ | F | $OCH_3$ | |
| $J_{32}$ | $CH_2$ | Br | $OCH_3$ | |
| $J_{32}$ | $CH_2$ | $SO_2CH_3$ | $OCH_3$ | |
| $J_{32}$ | $CH_2$ | $OSO_2CH_3$ | $OCH_3$ | |
| $J_{32}$ | $CH_2$ | $SO_2N(CH_3)_2$ | $OCH_3$ | |
| $J_{32}$ | $CH_2$ | $CO_2CH_3$ | $OCH_3$ | |
| $J_{32}$ | O | Cl | $OCH_3$ | |
| $J_{32}$ | O | F | $OCH_3$ | |
| $J_{32}$ | O | Br | $OCH_3$ | |
| $J_{32}$ | O | $SO_2CH_3$ | $OCH_3$ | |
| $J_{32}$ | O | $OSO_2CH_3$ | $OCH_3$ | |
| $J_{32}$ | O | $SO_2N(CH_3)_2$ | $OCH_3$ | |
| $J_{32}$ | O | $CO_2CH_3$ | $OCH_3$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table XXX

| | Percent by Weight | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspension | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredients can, of course, be present depending on the intended use and the physical properties of the compound. Higher ratios of surfactant to active ingredients are sometimes desirable, and are achieved by incorporation into the formulation or by tank mixing.

Typical solid diluents are described in Watkins, et al., "Handbook of Insecticide Dust Diluents and Carriers", 2nd. Edn., Dorland Books, Caldwell, N. J. Typical liquid diluents and solvents are described in Marsden, "Solvents Guide", 2nd. Ed., Interscience, New York, 1950. Solubility under 0.1% is preferred for suspension concentrates; solution concentrates are preferably stable against phase separation at 0°C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, Dec. 4, 1967, pp. 147ff. and "Perry's Chemical Engineer's Handbook", 4th. Edn., McGraw-Hill, N.Y., 1963, pp. 8—59ff.

For further information regarding the art of formulation, see for example:

H. M. Loux, U.S. Patent 3,235,361. Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7 Line 62 and Ex. 8, 12, 15, 39, 41, 52, 53, 58, 132, 138—140, 162—164, 166, 167, 169—182.

H. Gysin and E. Knusli, U.S. Patent 2,891,855, June 23, 1959, Col. 3, Line 66 through Col. 5, Line 17 and Examples 1—4.

G. C. Klingman, "Weed Control as a Science", John Wiley & Sons, Inc., New York, 1961 pp. 81—96.

J. D. Fryer and S. A. Evans, "Weed Control Handbook", 5th Edn., Blackwell Scientific Publications, Oxford, 1968, pp. 101—103.

J. B. Buchanan, U.S. Patent 3,576,834, April 27, 1971, Col. 5, Line 36 through Col. 7, Line 70 and Ex. 1—4, 17, 106, 123—140.

R. R. Shaffer, U.S. Patent 3,560,616, Feb. 2, 1971, Col. 3, Line 48 through Col. 7, Line 26 and Examples 3—9, 11—18.

E. Somers, "Formulation", Chapter 6 in Torgeson, "Fungicides", Vol. I, Academic Press, New York, 1967.

In the following examples, all parts are by weight unless otherwise indicated.

### Example 5

Wettable Powder

| | |
|---|---|
| 5-[[(4,6-dimethoxypyrimidin-2-yl)aminocarbonyl]aminosulfonylmethyl]-1,3-dimethyl-1H-pyrazole-4-carboxylic acid, ethyl ester | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended, passed through a mill, such as an air mill or hammer mill, to produce an average particle size under 25 microns, reblended and sifted through a U.S.S. No. 50 sieve (0.3 mm opening) before packaging.

### Example 6

Granule

| | |
|---|---|
| Wettable Powder of Example 10 | 5% |
| attapulgite granules (U.S.S. 20—40 mesh: 0.84—0.42 mm) | 95% |

A slurry of wettable powder containing 25% solids is sprayed on the surface of attapulgite granules in a double-cone blender. The granules are dried and packaged.

### Example 7

High Strength Concentrate

| | |
|---|---|
| 3-[[(4,6-dimethoxypyrimidin-2-yl)aminocarbonyl]aminosulfonylmethyl]-1,5-dimethyl-1H-pyrazole-4-carboxylic acid, ethyl ester | 98.5% |
| silica aerogel | 0.5% |
| synthetic amorphous fine silica | 1.0% |

The ingredients are blended and ground in a hammer mill to produce a high strength concentrate essentially all passing a U.S.S. No. 50 sieve (0.3 mm openings). This material may then be formulated in a variety of ways.

Example 8

Dusts

| | |
|---|---|
| wettable powder of Example 7 | 5% |
| pyrophyllite (powder) | 95% |

The wettable powder and the pyrophyllite diluent are thoroughly blended and then packaged. The product is suitable for use as a dust.

Example 9

Aqueous Suspension

| | |
|---|---|
| 5-[[(4,6-dimethoxypyrimidin-2-yl)aminocarbonyl]aminosulfonylmethyl]-1,3-dimethyl-1H-pyrazole-4-carboxylic acid, ethyl ester | 25% |
| hydrated attapulgite | 3% |
| crude calcium ligninsulfonate | 10% |
| water | 62% |

The ingredients are ground together in a ball, sand or roller mill until the solid particles have been reduced to diameters under 10 microns.

Example 10

Extruded Pellet

| | |
|---|---|
| 3-[[(4,6-dimethoxypyrimidin-2-yl)aminocarbonyl]aminosulfonylmethyl]-1,5-dimethyl-1H-pyrazole-4-carboxylic acid, ethyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded as cylinders about 1—3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 11

Solution

| | |
|---|---|
| 5-[[(4,6-dimethoxypyrimidin-2-yl)aminocarbonyl]aminosulfonylmethyl]-1,3-dimethyl-1H-pyrazole-4-carboxylic acid, ethyl ester | 5% |
| dimethylformamide | 95% |

Utility

The compounds of the present invention are active herbicides, having utility for broad-spectrum pre- and/or post-emergence weed control in areas where complete control of all vegetation is desired, such as around fuel storage tanks, ammunition depots, industrial storage areas, parking lots, drive-in theaters, billboards, and highway and railroad structures.

The rates of application for the compounds of the invention are determined by a number of factors, including the types of weeds to be controlled, weather and climate, formulations selected, mode of application, amount of foliage present, etc. In general terms, the subject compounds should be applied at levels of around 0.05 to 5 kg/ha, the lower rates being suggested for use on lighter soils and/or those having a low organic matter content, or for situations where only short-term persistence is required.

The compounds of the invention may be used in combination with any other commercial herbicide; examples of which are those of the triazine, triazole, uracil, urea, amide, diphenylether, carbamate and bipyridylium types. The compounds may also be used in combination with mefluidide.

The herbicidal properties of the subject compounds were discovered in a number of greenhouse tests. The test procedure and results follow.

*Test A*

Seeds of crabgrass *(Digitaria* sp.), barnyard grass *(Echinochloa crusqalli)*, wild oats *(Avena fatua)*, sicklepod *(Cassia obtusifolia)*, morningglory *(Ipomoea* sp.), cocklebur *(Xanthium pensylvanicum)*, sorghum, corn, soybean, sugar beet, rice, wheat, cotton and purple nutsedge *(Cyperus rotundus)* tubers were planted and treated pre-emergence with the test chemicals dissolved in a non-phytotoxic solvent. At the same time, these crop and weed species were treated post-emergence with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis or necrosis;
E = emergence inhibition;
G = growth retardation; and
H = formative effects.

The test compounds are referenced in the table of results as follows.

Compounds

Compound 1

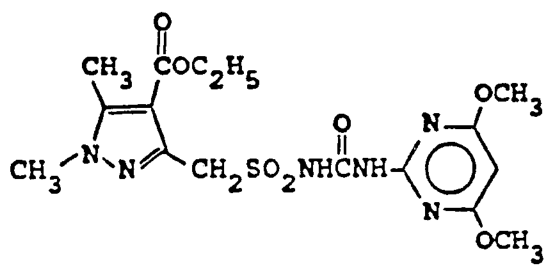

Compound 2

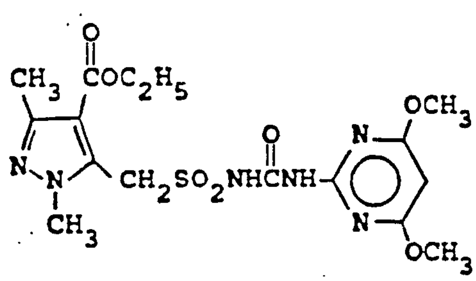

Compound 3

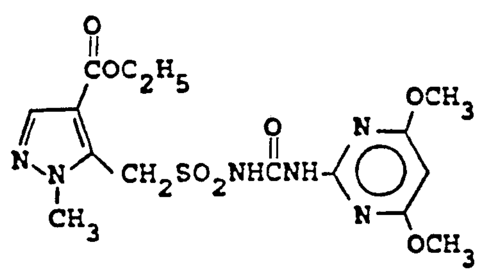

Compound 4

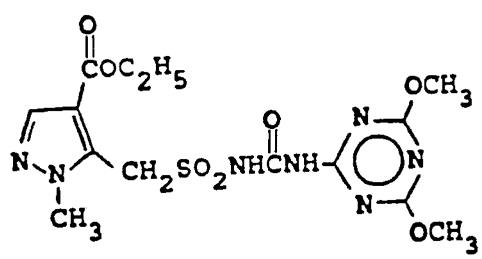

Compounds (Continued)

Compound 5

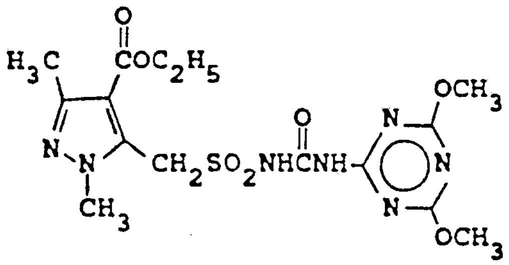

Compound 6

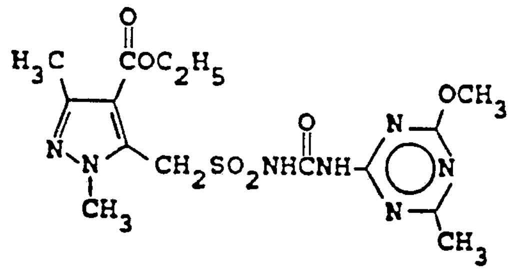

Compound 7

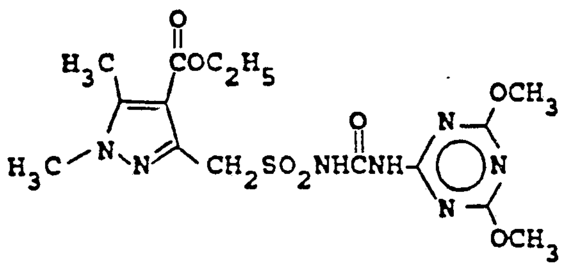

Compound 8

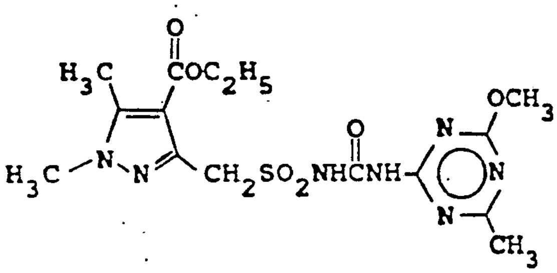

Compound 9

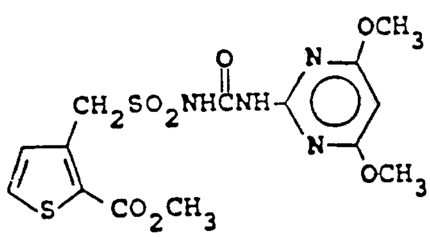

Compound 10

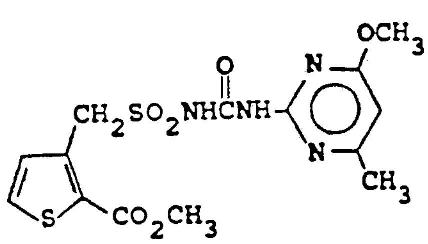

Compounds (Continued)

Compound 11

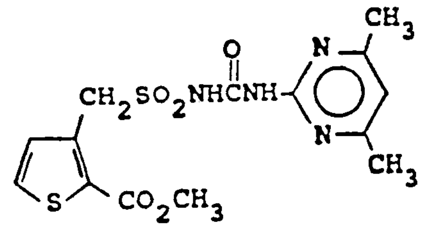

Compound 12

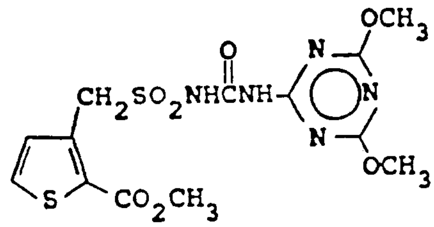

Compound 13

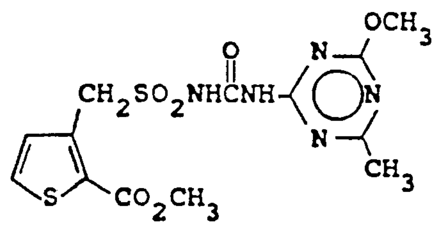

Compound 14

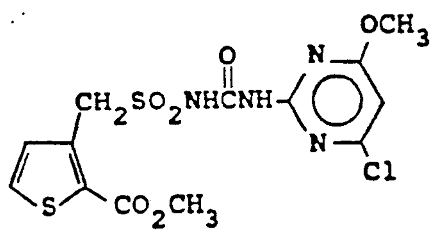

Compound 15

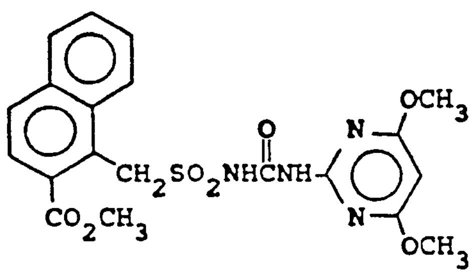

Compound 16

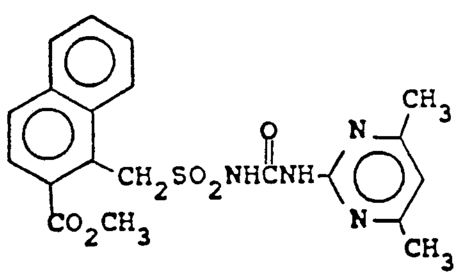

Compounds (continued)

Compound 17

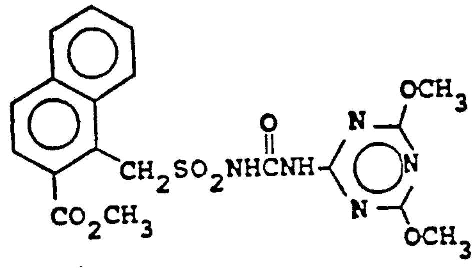

Compound 18

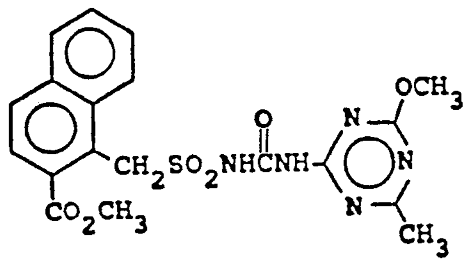

Table A

| | Compound 1 | | |
|---|---|---|---|
| Rate kg/ha | 2.0 | 0.4 | 0.05 |
| POST-EMERGENCE | | | |
| Morningglory | 5C,9G | 2C,8G | 4G |
| Cocklebur | 2C,8G | 1C | 0 |
| Sicklepod | 2C,9H | 3C,7H | 3C,5G |
| Nutsedge | 8G | 5G | 7G |
| Crabgrass | 6G | 2G | 0 |
| Barnyardgrass | 3C,8H | 3C,8H | 2C,5H |
| Wild Oats | 2C,5G | 0 | 0 |
| Wheat | 4G | 0 | 0 |
| Corn | 2C,9G | 3C,8G | 3C,8H |
| Soybean | 3C,9H | 4C,8H | 3C,9G |
| Rice | 2G | 2G | 0 |
| Sorghum | 3C,8H | 3C,8H | 3C,8H |
| Sugar beet | 3C,7G | 3C,5G | 3G |
| Cotton | 3C,9G | 4C,7G | 3C,5H |
| PRE-EMERGENCE | | | |
| Morningglory | 5C,9G | 4C,7G | 3H |
| Cocklebur | - | 0 | 5H |
| Sicklepod | 4C,8G | 4C,6G | 3G |
| Nutsedge | 10E | 0 | 0 |
| Crabgrass | 6G | 2C,5G | 2C,3G |
| Barnyardgrass | 4C,9H | 2C,5G | 0 |
| Wild Oats | 4C,8G | 2C,3G | 0 |
| Wheat | 8G | 3G | 0 |
| Corn | 5C,9G | 4C,5G | 2C,2G |
| Soybean | 3C,5H | 2C,2H | 1C |
| Rice | 3C,8H | 2C,5G | 0 |
| Sorghum | 3C,9H | 3C,8G | 2C,3G |
| Sugar beet | 4C,8G | 3C,8G | 2C,4G |
| Cotton | 2C | 0 | 0 |

Table A (continued)

| | Compound 2 | | |
|---|---|---|---|
| Rate kg/ha | 2.0 | 0.4 | 0.05 |
| POST-EMERGENCE | | | |
| Morningglory | 9C | 9C | 9C |
| Cocklebur | 3H | 5G | 1H |
| Sicklepod | 5C,6G | 3C,4H | 4C,4H |
| Nutsedge | 8G | 8G | 8G |
| Crabgrass | 4G | 2G | 0 |
| Barnyardgrass | 5C,9H | 3C,9H | 2C,3H |
| Wild Oats | 2G | 0 | 0 |
| Wheat | 0 | 0 | 0 |
| Corn | 2C,8H | 3C,8H | 2C,5H |
| Soybean | 4C,9G | 5C,9G | 3C,7G |
| Rice | 2C,7G | 2C,7G | 4G |
| Sorghum | 3C,8H | 3C,7G | 5G |
| Sugar beet | 3C,8H | 1C,4G | 4G |
| Cotton | 4C,8G | 3C,8G | 2C,7G |
| PRE-EMERGENCE | | | |
| Morningglory | 9C | 9G | 8G |
| Cocklebur | 9H | 8H | 0 |
| Sicklepod | 9G | - | 8G |
| Nutsedge | 10E | 10E | 0 |
| Crabgrass | 4G | 3G | 0 |
| Barnyardgrass | 4C,9H | 3C,8H | 3G |
| Wild Oats | 2C,6G | 1C,3G | 2G |
| Wheat | 2C,8H | 4G | 0 |
| Corn | 2C,9H | 4C,9H | 2C |
| Soybean | 4C,7G | 3C,3H | 0 |
| Rice | 10E | 4C,9H | 2C |
| Sorghum | 6G,9H | 4C,9G | 2C,3H |
| Sugar beet | 5C,9G | 9G | 7G |
| Cotton | 9G | 2C,8G | 0 |

Table A (continued)

| | Compound 3 | | Compound 4 |
|---|---|---|---|
| Rate kg/ha | 0.4 | 0.05 | 0.4 |
| POST-EMERGENCE | | | |
| Morningglory | 4C,9G | 3C,6H | 2C,2H |
| Cocklebur | 2C,5H | 0 | 0 |
| Nutsedge | 3G | 4G | 0 |
| Crabgrass | 1C | 0 | 0 |
| Barnyardgrass | 0 | 0 | 0 |
| Wild Oats | 0 | 0 | 0 |
| Wheat | 0 | 0 | 0 |
| Corn | 0 | 0 | 0 |
| Soybean | 2C,8G | 2C,2H | 0 |
| Rice | 0 | 0 | 0 |
| Sorghum | 3C,5G | 2G | 0 |
| Sugar beets | 3C,6G | 2C,3G | 1C,1H |
| Cotton | 3C,8H | 0 | 0 |
| Sicklepod | 3C,8H | 3C,3H | 0 |
| PRE-EMERGENCE | | | |
| Morningglory | 2C,5H | 0 | 1C |
| Cocklebur | 0 | 0 | 0 |
| Nutsedge | 10E | 0 | 0 |
| Crabgrass | 0 | 0 | 0 |
| Barnyardgrass | 0 | 0 | 0 |
| Wild Oats | 0 | 0 | 0 |
| Wheat | 0 | 0 | 0 |
| Corn | 2C | 0 | 3G |
| Soybean | 2C,3H | 0 | 0 |
| Rice | 0 | 0 | 0 |
| Sorghum | 4C,5G | 0 | 0 |
| Sugar beet | 3C,8G | 2C | 2C,3G |
| Cotton | 4G | 0 | 0 |
| Sicklepod | 2C | 0 | 0 |

Table A (continued)

| | Cmpd. 5 | Cmpd. 6 | Cmpd. 7 |
|---|---|---|---|
| Rate kg/ha | 0.4 | 0.4 | 0.4 |
| POST-EMERGENCE | | | |
| Morningglory | 5C,8H | 2C,3H | 3C,5G |
| Cocklebur | 0 | 3G | 0 |
| Nutsedge | 0 | 0 | 0 |
| Crabgrass | 0 | 0 | 0 |
| Barnyardgrass | 2C,4H | 2C,5H | 0 |
| Wild Oats | 0 | 0 | 0 |
| Wheat | 3G | 0 | 0 |
| Corn | 3C,8H | 2C,5H | 0 |
| Soybean | 2C,3H | 2C,3H | 3C,6G |
| Rice | 4C,8G | 2C,6G | 4G |
| Sorghum | 5C,9H | 3C,5H | 3G |
| Sugar beets | 4C,8H | 3C,6H | 3C,6G |
| Cotton | 4G | 2H | 0 |
| Sicklepod | 0 | 0 | 1C |
| PRE-EMERGENCE | | | |
| Morningglory | 2C | 0 | 0 |
| Cocklebur | 0 | 0 | 0 |
| Nutsedge | 0 | 0 | 0 |
| Crabgrass | 0 | 0 | 0 |
| Barnyardgrass | 0 | 0 | 0 |
| Wild Oats | 0 | 0 | 0 |
| Wheat | 2G | 0 | 0 |
| Corn | 3G | 0 | 0 |
| Soybean | 1C | 0 | 0 |
| Rice | 2C | 0 | 0 |
| Sorghum | 2C,5G | 2C,6G | 0 |
| Sugar beet | 0 | 0 | 0 |
| Cotton | 0 | 0 | 0 |
| Sicklepod | 0 | 0 | 0 |

TABLE A (Continued)

| | Compound 8 | Compound 9 | |
|---|---|---|---|
| Rate (kg/ha) | 0.4 | 0.4 | 0.05 |
| POSTEMERGENCE | | | |
| Morningglory | 2C,3H | 4C,5G | 8G,2C |
| Cocklebur | 0 | 1H | 0 |
| Nutsedge | 0 | 2C,8G | 2C,5G |
| Crabgrass | 0 | 4H | 0 |
| Barnyardgrass | 0 | 1H | 0 |
| Wild Oats | 0 | 0 | 0 |
| Wheat | 0 | 0 | 0 |
| Corn | 0 | 0 | 0 |
| Soybean | 3C,7G | 3C,8G | 2C,9G |
| Rice | 3G | 2C,3G | 2G |
| Sorghum | 3C,5H | 2C,2H | 0 |
| Sugar Beets | 2C | 5C,8H | 1C,4G |
| Cotton | 0 | 3C,7G | 2C,5G |
| Cassia | 0 | 2C,5G | 0 |
| PREEMERGENCE | | | |
| Morningglory | 0 | 9G | 7G |
| Cocklebur | 0 | 2G | 0 |
| Nutsedge | 0 | 8G | 5G |
| Crabgrass | 0 | 2G | 0 |
| Barnyardgrass | 0 | 4G | 1C |
| Wild Oats | 0 | 2G | 0 |
| Wheat | 0 | 0 | 0 |
| Corn | 0 | 2C,8H | 2C,5G |
| Soybean | 0 | 2C | 0 |
| Rice | 0 | 4G | 2G |
| Sorghum | 0 | 2C,7G | 3G |
| Sugar Beets | 0 | 9G | 4G |
| Cotton | 0 | 5G | 0 |
| Cassia | 0 | 2C,7G | 0 |

Table A (continued)

| | Cmpd. 10 | Cmpd. 11 | | Cmpd. 12 | Cmpd. 13 |
|---|---|---|---|---|---|
| Rate kg/ha | 0.4 | 0.4 | 2.0 | 0.4 | 0.4 |
| POST-EMERGENCE | | | | | |
| Morningglory | 3C,7H | 1C | 0 | 2C,9G | 5C,9G |
| Cocklebur | 4C,9H | 2C,5G | 6H | 0 | 5C,9G |
| Nutsedge | 0 | 0 | 0 | 3G | 5G |
| Crabgrass | 0 | 0 | 0 | 2G | 4G |
| Barnyardgrass | 0 | 0 | 0 | 2H | 2G |
| Wild Oats | 0 | 0 | 0 | 0 | 0 |
| Wheat | 0 | 0 | 0 | 0 | 2G |
| Corn | 0 | 0 | 0 | 2H | 2C,8H |
| Soybean | 3C,8G | 0 | 2C,5G | 3C,9G | 4C,9G |
| Rice | 0 | 0 | 0 | 3C,9G | 2C,8H |
| Sorghum | 0 | 0 | 0 | 2C,9H | 3C,9G |
| Sugar beets | 2C,2G | 0 | 2H | 9C | 9C |
| Cotton | 3G | 0 | 0 | 4C,9G | 3C,8G |
| Sicklepod | 2C | 0 | - | 2C,3G | 4C,6G |
| PRE-EMERGENCE | | | | | |
| Morningglory | 8G | 0 | 4H | 2C,8H | 9G |
| Cocklebur | 3C,6G | 0 | 3H | 0 | 9H |
| Nutsedge | 0 | 0 | 0 | 2G | 2G |
| Crabgrass | 0 | 0 | 0 | 0 | 0 |
| Barnyardgrass | 0 | 0 | 0 | 6H | 5H |
| Wild Oats | 0 | 0 | 0 | 2C,4G | 2G |
| Wheat | 0 | 0 | 0 | 8G | 4G |
| Corn | 2C,4G | 0 | 0 | 2C,8G | 9G |
| Soybean | 0 | 0 | 0 | 2C,3H | 2C,4H |
| Rice | 0 | 0 | 0 | 3C,5G | 2C,2G |
| Sorghum | 2C,3G | 0 | 0 | 2C,9H | 9H |
| Sugar beet | 8G | 0 | 4H | 5C,9G | 5C,9G |
| Cotton | 0 | 0 | 0 | 2C,4G | 5G |
| Sicklepod | 0 | 0 | - | 1C | 3C,5G |

TABLE A (Continued)

| | Compound 14 | |
|---|---|---|
| Rate (kg/ha) | 0.4 | 2.0 |
| POSTEMERGENCE | | |
| Morningglory | 1C | 0 |
| Cocklebur | 3C,8H | 2C,7H |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 0 | 2H |
| Soybean | 0 | 0 |
| Rice | 0 | 0 |
| Sorghum | 0 | 0 |
| Sugar Beets | 0 | 0 |
| Cotton | 0 | 2H |
| Cassia | 0 | 0 |
| PREEMERGENCE | | |
| Morningglory | 1C | 7G |
| Cocklebur | 2C,5H | 5H |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 0 | 0 |
| Soybean | 0 | 0 |
| Rice | 0 | 0 |
| Sorghum | 0 | 0 |
| Sugar Beets | 0 | 0 |
| Cotton | 0 | 0 |
| Cassia | 0 | - |

Table A (continued)

| | Cmpd. 15 | Cmpd. 16 | Cmpd. 17 | Cmpd. 18 |
|---|---|---|---|---|
| Rate kg/ha | 0.05 | 0.05 | 0.05 | 0.05 |
| POST-EMERGENCE | | | | |
| Morningglory | 3C,9G | 3G | 9C | 10C |
| Cocklebur | 2G | 3G | 2C | 5C,9G |
| Nutsedge | 5G | 0 | 2C,7G | 2C,6G |
| Crabgrass | 0 | 0 | 2G | 2C |
| Barnyardgrass | 0 | 0 | 0 | 0 |
| Wild Oats | 0 | 0 | 0 | 0 |
| Wheat | 0 | 0 | 0 | 0 |
| Corn | 0 | 0 | 1H | 3C,8H |
| Soybean | 5C,9G | 3C,8G | 5C,9G | 5C,9G |
| Rice | 0 | 0 | 0 | 0 |
| Sorghum | 0 | 0 | 3G | 3C,8H |
| Sugar beet | 3C,6G | 0 | 5C,9G | 5C,9G |
| Cotton | 9C | 2G | 4C,9G | 4C,9G |
| Sicklepod | 2C,3G | 1C | 3C,8G | 4C,6G |
| PRE-EMERGENCE | | | | |
| Morningglory | 8G | 3G | 9G | 9G |
| Cocklebur | 2H | 3G | - | 8H |
| Nutsedge | 3G | 0 | 5G | 0 |
| Crabgrass | 4G | 3G | 0 | 0 |
| Barnyardgrass | 0 | 0 | 3G | 3G |
| Wild Oats | 0 | 0 | 3G | 5G |
| Wheat | 0 | 0 | 4G | 8G |
| Corn | 0 | 3G | 2C,7G | 9G |
| Soybean | 1H | 0 | 2H | 3C,7G |
| Rice | 0 | 0 | 2G | 2G |
| Sorghum | 0 | 2G | 5G | 9G |
| Sugar beet | 5G | 2G | 5C,9G | 5C,9G |
| Cotton | 2G | 0 | 8G | 8G |
| Sicklepod | 5G | 4G | 2C,7G | 2C |

## Claims

1. A compound of the formula:

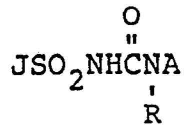

I

wherein

J is

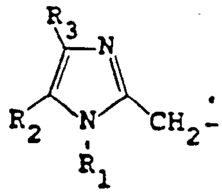

J-1

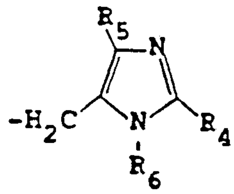

J-2

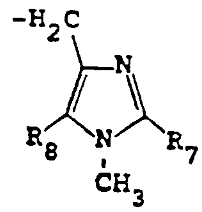

J-3

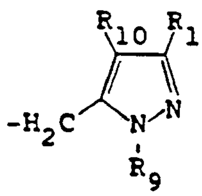

J-4

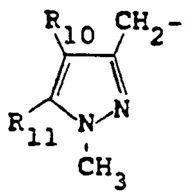

J-5

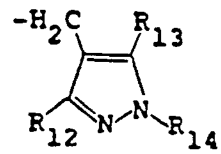

J-6

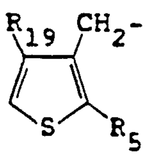

J-7

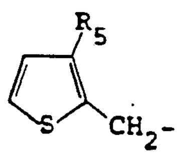

J-8

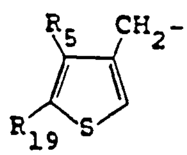

J-9

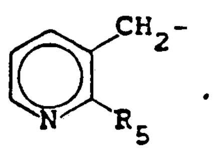

J-10

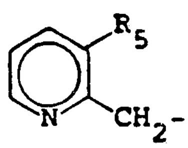

J-11

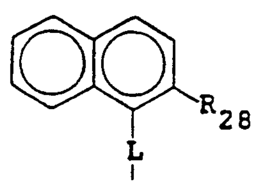

$J_{32}$

L is $CH_2$ or O;
R is H or $CH_3$;
$R_1$ is H, $C_1$—$C_3$ alkyl, $CO_2R_{15}$, $SO_2NR_{16}R_{17}$ or $SO_2R_{18}$;
$R_2$ is H or $CH_3$;
$R_3$ is H or $CH_3$
$R_4$ is H or $CH_3$
$R_5$ is H, $CH_3$, Cl, Br, $CO_2R_{15}$, $SO_2NR_{16}R_{17}$, $SO_2N(OCH_3)CH_3$, $SO_2R_{18}$ or $NO_2$;
$R_6$ is H, $C_1$—$C_3$ alkyl, $CO_2R_{15}$, $SO_2NR_{16}R_{17}$ or $SO_2R_{18}$;
$R_7$ is H or $CH_3$;
$R_8$ is $CO_2R_{15}$, $SO_2NR_{16}R_{17}$, $SO_2N(OCH_3)CH_3$, $SO_2R_{18}$ or $NO_2$;
$R_9$ is H, $C_1$—$C_3$ alkyl or phenyl;
$R_{10}$ is $CO_2R_{15}$, $SO_2NR_{16}R_{17}$, $SO_2N(OCH_3)CH_3$, $SO_2R_{18}$ or $NO_2$;

$R_{11}$ is H or $CH_3$;
$R_{12}$ is H, $CO_2R_{15}$, $SO_2NR_{16}R_{17}$, $SO_2N(OCH_3)CH_3$, $SO_2R_{18}$ or $NO_2$;
$R_{13}$ is H, $CO_2R_{15}$, $SO_2NR_{16}R_{17}$, $SO_2N(OCH_3)CH_3$, $SO_2R_{18}$ or $NO_2$;
$R_{14}$ is H or $CH_3$;
$R_{15}$ is $C_1$—$C_2$ alkyl;
$R_{16}$ is $C_1$—$C_2$ alkyl;
$R_{17}$ is $C_1$—$C_2$ alkyl;
$R_{18}$ is $C_1$—$C_2$ alkyl;
$R_{19}$ is $CH_3$, Cl, Br, $NO_2$, $C_1$—$C_2$ alkylthio or $C_1$—$C_2$ alkylsulfonyl;
$R_{28}$ is F, Cl, Br, $OCH_3$, $SCH_3$, $SO_2CH_3$, $CO_2CH_3$, $SO_2N(CH_3)_2$ or $OSO_2CH_3$;

A is 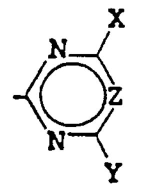 , 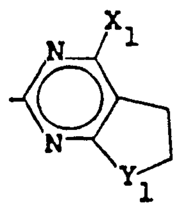 , 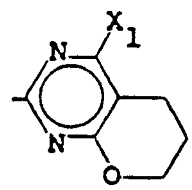 , 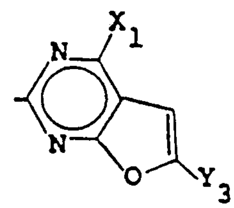 ,

A-1 A-2 A-3 A-4

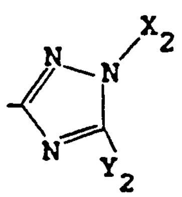 , 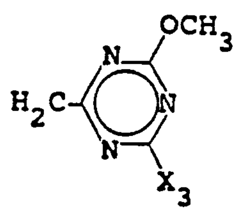 ;

A-5 A-6

X is $CH_3$, $OCH_3$, Cl, Br, $OCH_2CF_3$ or $OCHF_2$;
Y is $C_1$—$C_3$ alkyl, $CH_2F$, cyclopropyl, C≡CH, $OCH_3$, $OC_2H_5$, $NH_2$, $NHCH_3$, $N(CH_3)_2$, $OCH_2CH_2F$, $CF_3$, $SCH_3$, $OCH_2CH{=}CH_2$, $OCH_2C{\equiv}CH$, $OCH_2CH_2OCH_3$ or $OCF_2H$;
Z is CH or N;
$X_1$ is $CH_3$, $OCH_3$, $OC_2H_5$ or $OCF_2H$;
$Y_1$ is O or $CH_2$;
$X_2$ is $CH_3$, $C_2H_5$ or $CH_2CF_3$;
$Y_2$ is $OCH_3$, $OC_2H_5$, $SCH_3$, $SC_2H_5$, $OCF_2H$, $SCF_2H$, $CH_3$ or $CH_2CH_3$;
$X_3$ is $CH_3$ or $OCH_3$;
$Y_3$ is H or $CH_3$; and agriculturally suitable salts thereof; provided that
1) when X is Cl or Br, then Z is CH and Y is $OCH_3$, $OC_2H_5$, $NHCH_3$, $N(CH_3)_2$ or $OCF_2H$;
2) when X or Y is $OCF_2H$, then Z is CH;
3) $R_5$ and $R_6$ are not simultaneously H;
4) when $R_6$ is other than H or $C_1$—$C_3$ alkyl, then $R_5$ must be H;
5) $R_{12}$ and $R_{13}$ are not simultaneously H;
6) when $R_{12}$ is other than H, then $R_{13}$ must be H;
7) $R_5$ and $R_{19}$ are not simultaneously H; and
8) when J is J-8 or J-11 then $R_5$ is other than H.

2. The compound of Claim 1 where R is H and A is A—1.

3. The compound of Claim 2 where Y is $CH_3$, $C_2H_5$, $OCH_3$, $OC_2H_5$, $CH_2F$ or $CF_3$; and L is $CH_2$.

4. The compound of Claim 2 where J is J—1, J—4, J—5 or J—6; X is $CH_3$ or $OCH_3$; and Y is $C_1$—$C_2$ alkoxy or $C_1$—$C_2$ alkyl.

5. The compound of Claim 1 which is 3-[[(4,6-dimethoxypyrimidin-2-yl)aminocarbonyl]aminosulfonyl methyl]-1,5-dimethyl-1H-pyrazole-4-carboxylic acid, ethyl ester, or an agriculturally suitable salt thereof.

6. The compound of Claim 1 which is 5-[[(4,6-dimethoxypyrimidin-2-yl)aminocarbonyl]aminosulfonyl-methyl]-1,3-dimethyl-1H-pyrazole-4-carboxylic acid, ethyl ester, or an agriculturally suitable salt thereof.

7. A composition suitable for controlling the growth of undesired vegetation which comprises an effective amount of a compound of any of Claims 1 to 6 and at least one of the following: surfactant, solid or liquid diluent.

8. A method for controlling the growth of undesired vegetation which comprises applying to the locus to be protected an effective amount of a compound of any of Claims 1 to 6.

9. A method for regulating the growth of plants which comprises applying to the locus of such plants an effective but substantially non-phytotoxic amount of a plant growth regulant selected from compounds of any of claims 1 to 6.

10. A process for the production of a compound of claim 1 which comprises:

(a) reacting a sulfonyl isocyanate of formula

$$JSO_2NCO \qquad \text{(II)}$$

with an appropriate heterocyclic amine of formula

$$\underset{\displaystyle R}{\overset{}{HN}}\!-\!A \qquad \text{(III)}$$

wherein J, R and A are as defined in claim 1; or

(b) reacting a sulfonamide of formula

$$JSO_2NH_2 \qquad \text{(IV)}$$

wherein L is —$CH_2$— and $R_1$, $R_5$, $R_6$, $R_8$, $R_{10}$, $R_{12}$ and $R_{13}$ of J are other than $CO_2R_{15}$; with an appropriate methyl carbamate of formula

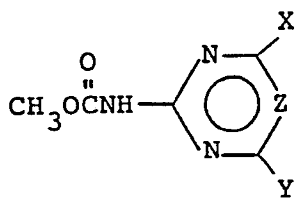

(V)

wherein X, Y and Z are as defined in claim 1; or

(c) reacting a sulfonyl carbamate of formula

$$JSO_2NH\overset{O}{\overset{\|}{C}}OC_6H_5 \qquad \text{(VI)}$$

where J is as defined in claim 1 and L is —$CH_2$—, with an appropriate heterocyclic amine of formula (III) as defined above; or

(d) reacting a sulfonamide of formula (III) wherein L is —$CH_2$—, with an appropriate heterocyclic phenyl carbamate of formula

$$C_6H_5O\overset{O}{\overset{\|}{C}}NHA \qquad \text{(VII)}$$

wherein A is as defined in claim 1; or

(e) reacting a phenol of formula

$$J—H \qquad \text{(IX)}$$

wherein J is $J_{32}$ as defined in claim 1 and L is O, with a chlorosulfonylurea of formula

$$ClSO_2NH\overset{O}{\overset{\|}{C}}\underset{\displaystyle R}{N}\!-\!A \qquad \text{(VII)}$$

wherein A and R are as defined in claim 1.

**Patentänspruche**

1. Verbindung der Formel

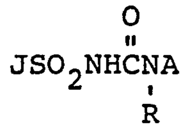

I

worin J

J is 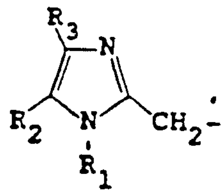 , 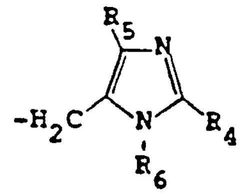 , 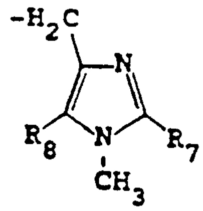 ,

J-1 J-2 J-3

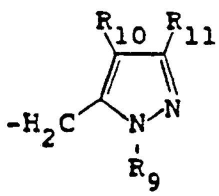 , 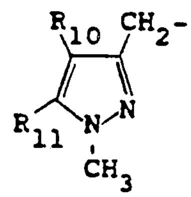 , 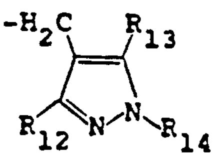 ,

J-4 J-5 J-6

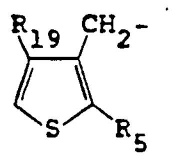 , 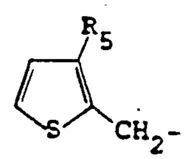 , 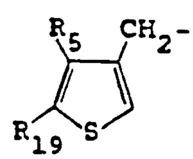 ,

J-7 J-8 J-9

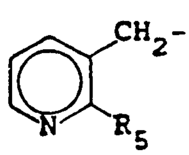 , 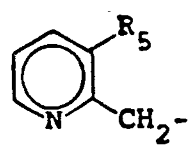 , oder 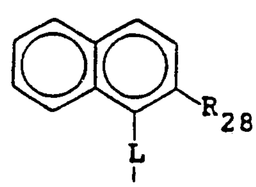

J-10 J-11 oder $J_{32}$ ist;

n 0 oder 1 ist;
L $CH_2$ oder O ist;
R H oder $CH_3$ ist;
$R_1$ H, $C_1$—$C_3$-Alkyl, $CO_2R_{15}$, $SO_2NR_{16}R_{17}$ oder $SO_2R_{18}$ ist;
$R_2$ H oder $CH_3$ ist;
$R_3$ H oder $CH_3$ ist;
$R_4$ H oder $CH_3$ ist;
$R_5$ H, $CH_3$, Cl, Br, $CO_2R_{15}$, $SO_2NR_{16}R_{17}$, $SO_2N(OCH_3)CH_3$, $SO_2R_{18}$ oder $NO_2$ ist;
$R_6$ H, $C_1$—$C_3$-Alkyl, $CO_2R_{15}$, $SO_2NR_{16}R_{17}$ oder $SO_2R_{18}$ ist;
$R_7$ H oder $CH_3$ ist;
$R_8$ $CO_2R_{15}$, $SO_2NR_{16}R_{17}$, $SO_2N(OCH_3)CH_3$, $SO_2R_{18}$ oder $NO_2$ ist;
$R_9$ H, $C_1$—$C_3$ alkyl oder Phenyl ist;

$R_{10}$ $CO_2R_{15}$, $SO_2NR_{16}R_{17}$, $SO_2N(OCH_3)CH_3$, $SO_2R_{18}$ oder $NO_2$ ist;
$R_{11}$ H oder $CH_3$ ist;
$R_{12}$ H, $CO_2R_{15}$, $SO_2NR_{16}R_{17}$, $SO_2N(OCH_3)CH_3$, $SO_2R_{18}$ oder $NO_2$ ist;
$R_{13}$ H, $CO_2R_{15}$, $SO_2NR_{16}R_{17}$, $SO_2N(OCH_3)CH_3$, $SO_2R_{18}$ oder $NO_2$ ist;
$R_{14}$ H oder $CH_3$ ist;
$R_{15}$ $C_1$—$C_2$-Alkyl ist;
$R_{16}$ $C_1$—$C_2$-Alkyl ist;
$R_{17}$ $C_1$—$C_2$-Alkyl ist;
$R_{18}$ $C_1$—$C_2$-Alkyl ist;
$R_{19}$ $CH_3$, Cl, Br, $NO_2$, $C_1$—$C_2$-Alkylthio oder $C_1$—$C_2$-Alkylsulfonyl ist;
$R_{28}$ F, Cl, Br, $OCH_3$, $SCH_3$, $SO_2CH_3$, $CO_2CH_3$, $SO_2N(CH_3)_2$ oder $OSO_2CH_3$ ist;

A is 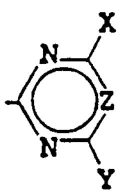 , 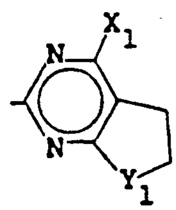 , 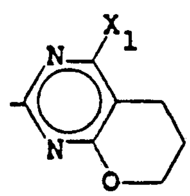 , 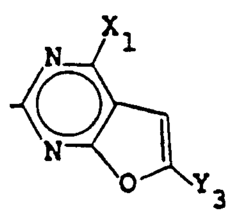 ,

A-1 A-2 A-3 A-4

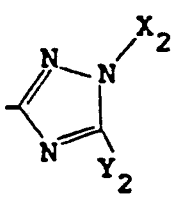 , 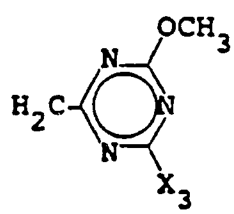 ; ist;

A-5 A-6.

X $CH_3$, $OCH_3$, Cl, Br, $OCH_2CF_3$ oder $OCHF_2$ ist;
Y $C_1$—$C_3$-Alkyl, $CH_2F$, Cyclopropyl, C≡CH, $OCH_3$, $OC_2H_5$, $NH_2$, $NHCH_3$, $N(CH_3)_2$, $OCH_2CH_2F$, $CF_3$, $SCH_3$, $OCH_2CH=CH_2$, $OCH_2C≡CH$, $OCH_2CH_2OCH_3$ oder $OCF_2H$ ist;
Z CH oder N ist;
$X_1$ $CH_3$, $OCH_3$, $OC_2H_5$ oder $OCF_2H$ ist;
$Y_1$ O oder $CH_2$ ist;
$X_2$ $CH_3$, $C_2H_5$ oder $CH_2CF_3$ ist;
$Y_2$ $OCH_3$, $OC_2H_5$, $SCH_3$, $SC_2H_5$, $OCF_2H$, $SCF_2H$, $CH_3$ oder $CH_2CH_3$ ist;
$X_3$ $CH_3$ oder $OCH_3$ ist;
$Y_3$ H oder $CH_3$ ist;
sowie landwirtschaftlich geeignete Salze davon; mit der Maßgabe, daß
1) wenn X Cl oder Br ist, dann Z CH ist und Y $OCH_3$, $OC_2H_5$, $NHCH_3$, $N(CH_3)_2$ oder $OCF_2H$ ist;
2) wenn X oder Y $OCF_2H$ ist, dann Z CH ist;
3) $R_5$ und $R_6$ nicht gleichzeitig H sind;
4) wenn $R_6$ von H oder $C_1$—$C_3$-Alkyl verschieden ist, dann $R_5$ H sein muß;
5) $R_{12}$ und $R_{13}$ nicht gleichzeitig H sind;
6) wenn $R_{12}$ von H verschieden ist, dann $R_{13}$ H sein muß;
7) $R_5$ und $R_{19}$ nicht gleichzeitig H sind; und
8) wenn J J—8 oder J—11 ist, dann $R_5$ von H verscheiden ist.

2. Verbindung nach Anspruch 1, worin R H ist und A A—1 ist.

3. Verbindung nach Anspruch 2, worin Y $CH_3$, $C_2H_5$, $OCH_3$, $OC_2H_5$, $CH_2F$ oder $CF_3$ ist; und L $CH_2$ ist.

4. Verbindung nach Anspruch 2, worin J J—1, J—4, J—5 oder J—6 ist; X $CH_3$ oder $OCH_3$ ist; und Y $C_1$—$C_2$-Alkoxy oder $C_1$—$C_2$-Alkyl ist.

5. Verbindung nach Anspruch 1, welche 3-[[(4,6-Dimethoxypyrimidin-2-yl)aminocarbonyl]aminosulfonylmethyl]-1,5-dimethyl-1H-pyrazol-4-carbonsäure-ethylester oder ein landwirtschaftlich geeignetes Salz davon ist.

6. Verbindung nach Anspruch 1, welche 5-[[(4,6-Dimethoxypyrimidin-2-yl)aminocarbonyl]aminosulfonylmethyl]-1,3-dimethyl-1H-pyrazol-4-carbonsäure-ethylester oder ein landwirtschaftlich geeignetes Salz davon ist.

7. Zur Bekämpfung des Wachstums unerwünschter Vegetation geeignete Zusammensetzung, welche eine wirksame Menge einer Verbindung nach einem der Ansprüche 1 bis 6 und wenigstens eines der folgenden: oberflächenaktives Mittel, festes oder flüssiges Verdünnungsmittel, umfaßt.

8. Verfahren zur Bekämpfung des Wachstums unerwünschter Vegetation, welches die Anwendung einer wirksamen Menge einer Verbindung nach einem der Ansprüche 1 bis 6 auf den zu schützenden Ort umfaßt.

9. Verfahren zur Steuerung des Wachstums von Pflanzen, welches die Anwendung einer wirksamen, jedoch im wesentlichen nicht phytotoxischen Menge eines Pflanzenwachstumsregulans, ausgewählt aus den Verbindungen nach einem der Ansprüche 1 bis 6, auf den Ort solcher Pflanzen umfaßt.

10. Verfahren zur Herstellung einer Verbindung nach Anspruch 1 durch

(a) Reagieren eines Sulfonylisocyanats der Formel

$$JSO_2NCO \qquad (II)$$

mit einem geeigneten heterocyclischen Amin der Formel

$$\begin{matrix} HN{-}A \\ | \\ R \end{matrix} \qquad (III)$$

worin J, R und A wie in Anspruch 1 definiert sind; oder

(b) Reagieren eines Sulfonamids der Formel

$$JSO_2NH_2 \qquad (IV)$$

worin L —$CH_2$— ist und $R_1$, $R_5$, $R_6$, $R_8$, $R_{10}$, $R_{12}$ und $R_{13}$ von J von $CO_2R_{15}$ verschieden sind, mit einem geeigneten Methylcarbamat der Formel

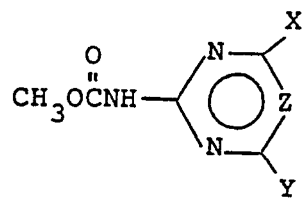

(V)

worin X, Y und Z wie in Anspruch 1 definiert sind; oder

(c) Reagieren eines Sulfonylcarbamates der Formel

$$JSO_2NH\overset{\overset{\displaystyle O}{\|}}{C}OC_6H_5 \qquad (VI)$$

worin J wie in Anspruch 1 definiert ist und L —$CH_2$— ist, mit einem geeigneten heterocyclischen Amin der Formel (III), wie oben definiert; oder

(d) Reagieren eines Sulfonamids der Formel (III), worin L —$CH_2$— ist, mit einem geeigneten heterocyclischen Phenylcarbamat der Formel

$$C_6H_5O\overset{\overset{\displaystyle O}{\|}}{C}NHA \qquad (VII)$$

worin A wie in Anspruch 1 definiert ist; oder

(e) Reagieren eines Phenols der Formel

$$J{-}H \qquad (IX)$$

worin J $J_{32}$ ist, wie in Anspruch 1 definiert, und L O is, mit einem Chlorsulfonylharnstoff der Formel

$$ClSO_2NH\overset{\overset{\displaystyle O}{\|}}{C}\underset{\underset{\displaystyle R}{|}}{N}{-}A \qquad (VIII)$$

worin A und R wie in Anspruch 1 definiert sind.

**Revendications**

1. Un composé de la formule

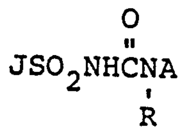

I

J is 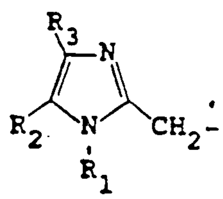, 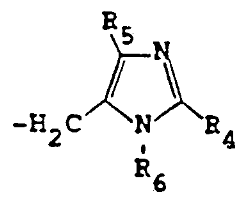, 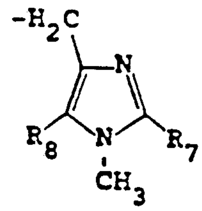,

J-1 J-2 J-3

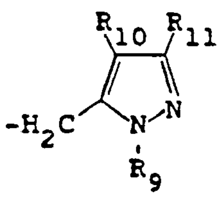, 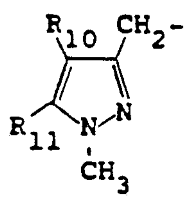, 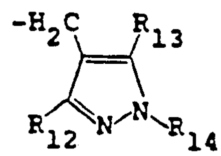,

J-4 J-5 J-6

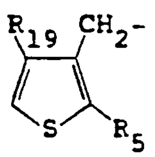, 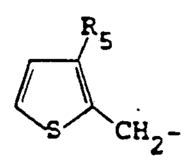, 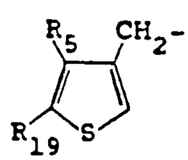,

J-7 J-8 J-9

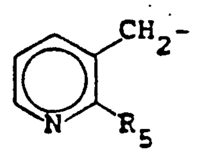, 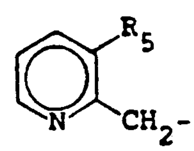, 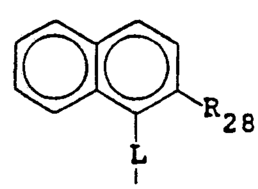

J-10 J-11 J32

n est 0 ou 1;
L est $CH_2$ ou O;
R est H ou $CH_3$;
$R_1$ est H, un groupe alkyle en $C_1$—$C_3$, $CO_2R_{15}$, $SO_2NR_{16}R_{17}$ ou $SO_2R_{18}$;
$R_2$ est H ou $CH_3$;
$R_3$ est H ou $CH_3$;
$R_4$ est H ou $CH_3$;
$R_5$ est H, $CH_3$, Cl, Br, $CO_2R_{15}$, $SO_2NR_{16}R_{17}$, $SO_2N(OCH_3)CH_3$, $SO_2R_{18}$ ou $NO_2$;
$R_6$ est H, un groupe alkyle en $C_1$—$C_3$, $CO_2R_{15}$, $SO_2NR_{16}R_{17}$ ou $SO_2R_{18}$;
$R_7$ est H ou $CH_3$;
$R_8$ est $CO_2R_{15}$, $SO_2NR_{16}R_{17}$, $SO_2N(OCH_3)CH_3$, $SO_2R_{18}$ ou $NO_2$;
$R_9$ est H ou un groupe alkyle en $C_1$—$C_3$ ou phényle;
$R_{10}$ est $CO_2R_{15}$, $SO_2NR_{16}R_{17}$, $SO_2N(OCH_3)CH_3$, $SO_2R_{18}$ ou $NO_2$;
$R_{11}$ est H ou $CH_3$;

$R_{12}$ est H, $CO_2R_{15}$, $SO_2NR_{16}R_{17}$, $SO_2N(OCH_3)CH_3$, $SO_2R_{18}$ ou $NO_2$;
$R_{13}$ est H, $CO_2R_{15}$, $SO_2NR_{16}R_{17}$, $SO_2N(OCH_3)CH_3$, $SO_2R_{18}$ ou $NO_2$;
$R_{14}$ est H ou $CH_3$;
$R_{15}$ est un groupe alkyle en $C_1$—$C_2$;
$R_{16}$ est un groupe alkyle en $C_1$—$C_2$;
$R_{17}$ est un groupe alkyle en $C_1$—$C_2$;
$R_{18}$ est un groupe alkyle en $C_1$—$C_2$;
$R_{19}$ est $CH_3$, Cl, Br, $NO_2$ ou un groupe alkylthio en $C_1$—$C_2$ ou alkylsulfonyle en $C_1$—$C_2$;
$R_{28}$ est F, Cl, Br, $OCH_3$, $SCH_3$, $SO_2CH_3$, $CO_2CH_3$, $SO_2N(CH_3)_2$ ou $OSO_2CH_3$;

A is 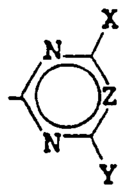 , 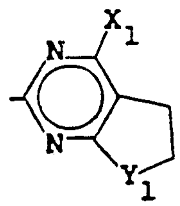 , 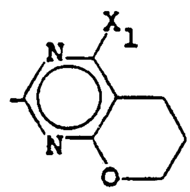 , 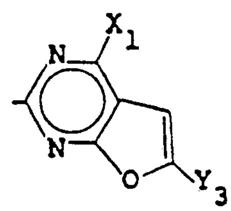 ,

A-1 A-2 A-3 A-4

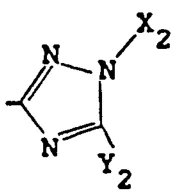 , 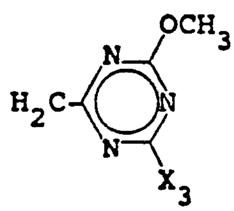 ;

A-5 A-6

Y est un groupe alkyle en $C_1$—$C_3$, $CH_2F$, le groupe cyclopropyle, C≡CH, $OCH_3$, $OC_2H_5$, $NH_2$, $NHCH_3$, $N(CH_3)_2$, $OCH_2CH_2F$, $CF_3$, $SCH_3$, $OCH_2CH=CH_2$, $OCH_2C≡CH$, $OCH_2CH_2OCH_3$ ou $OCF_2H$;
Z est CH ou N;
$X_1$ est $CH_3$, $OCH_3$, $OC_2H_5$ ou $OCF_2H$;
$Y_1$ est O ou $CH_2$;
$X_2$ est $CH_3$, $C_2H_5$ ou $CH_2CF_3$;
$Y_2$ est $OCH_3$, $OC_2H_5$, $SCH_3$, $SC_2H_5$, $OCF_2H$, $SCF_2H$, $CH_3$ ou $CH_2CH_3$;
$X_3$ est $CH_3$ ou $OCH_3$;
$Y_3$ est H ou $CH_3$; et ses sels utilisables en agriculture; avec les conditions suivantes
1) si X est Cl ou Br, alors Z est CH et Y est $OCH_3$, $OC_2H_5$, $NHCH_3$, $N(CH_3)_2$ ou $OCF_2H$;
2) si X ou Y est $OCF_2H$, alors Z est CH;
3) $R_5$ et $R_6$ ne sont pas simultanément H;
4) si $R_6$ est autre chose que H ou un groupe alkyle en $C_1$—$C_3$, alors $R_5$ doit être H;
5) $R_{12}$ et $R_{13}$ ne sont pas simultanément H;
6) si $R_{12}$ est autre chose que H, alors $R_{13}$ doit être H;
7) $R_5$ et $R_{19}$ ne sont pas simultanément H; et
8) si J est J—8 ou J—11, alors $R_5$ est autre chose que H.

2. Le composé de la revendication 1, où R est H et A est A—1.

3. Le composé de la revendication 1, où Y est $CH_3$, $C_2H_5$, $OCH_3$, $OC_2H_5$, $CH_2F$ ou $CF_3$; et L est $CH_2$.

4. Le composé de la revendication 2, où J est J—1, J—4, J—5 ou J—6; X est $CH_3$ ou $OCH_3$; et Y est un groupe alcoxy en $C_1$—$C_2$ ou alkyle en $C_1$—$C_2$.

5. Le composé de la revendication 1, qui est l'ester éthylique de l'acide 3-[[(4,6-diméthoxypyrimidine-2-yl)aminocarbonyl]aminosulfonylméthyl]-1,5-diméthyl-1H-pyrazole-4-carboxylique, ou un sel utilisable en agriculture de celui-ci.

6. Le composé de la revendication 1, qui est l'ester éthylique de l'acide 5-[[(4,6-diméthoxypyrimidine-2-yl)aminocarbonyl]aminosulfonylméthyl]-1,3-diméthyl-1H-pyrazole-4-carboxylique, ou un sel utilisable en agriculture de celui-ci.

7. Une composition covenant pour lutter contre la croissance de végétation indésirable, qui comprend une quantité efficace d'un composé de l'une quelconque des revendications 1 à 6 et l'un au moins des ingrédients suivants: agent tensio-actif et diluant solide ou liquide.

8. Une procédé pour lutter contre la croissance de végétation indésirable, qui consiste à appliquer à l'emplacement à protéger une quantité efficace d'un composé de l'une quelconque des revendications 1 à 6.

9. Un procédé pour réguler le croissance de plantes, qui consiste à appliquer à l'emplacement où se trouvent ces plantes une quantité efficace mais sensiblement non phytotoxique d'un régulateur de croissance de plantes choisi parmi les composés de l'une quelconque des revendications 1 à 6.

10. Un procédé pour la production d'un composé de la revendication 1, qui consiste:

(a) à faire réagir un isocyanate de sulfonyle de formule

$$JSO_2NCO \qquad (II)$$

avec une amine hétérocyclique appropriée de formule

$$\begin{array}{c} HN—A \\ | \\ R \end{array} \qquad (III)$$

où J, R et A sont tels que définis dans la revendication 1; ou

(b) à faire réagir un sulfonamide de formule

$$JSO_2NH_2 \qquad (IV)$$

où L est —$CH_2$— et $R_1$, $R_5$, $R_6$, $R_8$, $R_{10}$, $R_{12}$ et $R_{13}$ de J sont autre chose que $CO_2R_{15}$, avec un méthyl carbamate approprié de formule

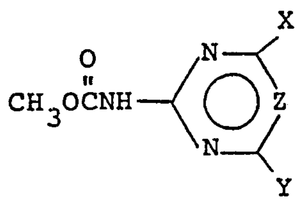

(V)

où X, Y et Z sont tels que définis dans la revendication 1; ou

(c) à faire réagir un sulfonylcarbamate de formule

$$\begin{array}{c} O \\ \| \\ JSO_2NHCOC_6H_5 \end{array} \qquad (VI)$$

où J est tel que défini dans la revendication 1 et L est —$CH_2$—, avec une amine hétérocyclique appropriée de formule (III) telle que définie ci-dessus; ou

(d) à faire réagir un sulfonamide de formule (IV) où L est —$CH_2$—, avec un phényl carbamate hétérocyclique approprié de formule

$$\begin{array}{c} O \\ \| \\ C_6H_5OCNHA \end{array} \qquad (VII)$$

où A est tel que défini dans la revendication 1; ou

(e) à faire réagir un phénol de formule

$$J—H \qquad (IX)$$

où J est $J_{32}$ tel que définis dans la revendication 1 et L est O, avec une chlorosulfonylurée de formule

$$\begin{array}{c} O \\ \| \\ ClSO_2NHCN—A \\ | \\ R \end{array} \qquad (VIII)$$

où A et R sont tels que définis dans la revendication 1.